# EUROPEAN PATENT APPLICATION

(11) **EP 2 361 910 A1**
(43) Date of publication of application: **31.08.2011**
(21) Application number: 09823635.9
(22) Date of filing: 28.10.2009
(51) Int. Cl.: C07D 471/04, A61K 31/4745, A61K 31/5383, A61K 31/55, A61P 3/04, A61P 7/02, A61P 9/10, A61P 11/00, A61P 13/12, A61P 27/02, A61P 29/00, A61P 35/00, A61P 43/00, C07D 471/14, C07D 487/04, C07D 498/04

(54) **COMPOUND HAVING TAFIA INHIBITORY ACTIVITY**

(30) Priority: 29.10.2008 JP 2008278302
(71) Applicant: Taisho Pharmaceutical Co., Ltd., Tokyo 170-8633 (JP)
(72) Inventor: AMADA, Hideaki, Tokyo 170-8633 (JP); MATSUDA, Daisuke, Tokyo 170-8633 (JP); BOHNO, Masahiro, Tokyo 170-8633 (JP); SAITO, Shiuji, Tokyo 170-8633 (JP); BOHNO, Ayako, Tokyo 170-8633 (JP)
(74) Representative: HOFFMANN EITLE
(86) International application number: PCT/JP2009/068526
(87) International publication number: WO 2010/050525

(57) **Abstract**

Provided are compounds having superior TAFIa inhibitory activity. Specifically, there are provided compounds represented by the following formula (I) or pharmaceutically acceptable salts thereof: 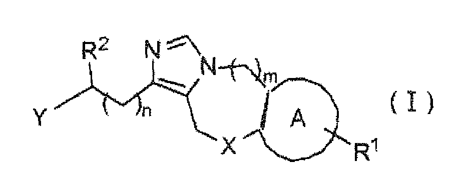 wherein A is a benzene ring or a pyridine ring; X is the formula -(CH₂)-, the formula -(CH₂)₂-, an oxygen atom, a nitrogen atom or a single bond; Y is the formula -(CH₂)₃-NH-R³, the formula -(CH₂)₄-NH-R³ or a 2-aminopyridyl group; R³ is a hydrogen atom, a C₁₋₆ alkyl group, or the formula -CO₂R⁴; R⁴ is a C₁₋₆ alkyl group, the formula -CHR⁵OC(O)R⁶, or a substituent having the structure represented by the following formula Ia; 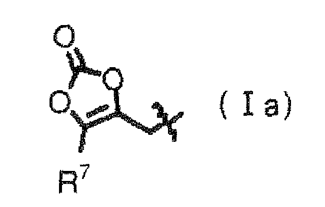 R⁵ is a C₁₋₆ alkyl group; R⁶ is a C₁₋₆ alkyl group, a C₃₋₈ cycloalkyl group, or a phenyl group; R⁷ is a C₁₋₆ alkyl group or a phenyl group; a R¹ is a hydrogen atom, a halogen atom, a C₁₋₄ alkyl group substituted by 1-3 halogen atoms, a C₁₋₁₀ alkyl group, a C₁₋₈ alkoxy group, a C₃₋₈ cycloalkyl group, a C₃₋₈ cycloalkoxy group, a C₄₋₁₄ cycloalkylalkyl group, or a phenyl group; R² is CO₂R⁸, or a tetrazolyl group; R⁸ is a hydrogen atom, a C₁₋₁₀ alkyl group, or a substituent having the structure represented by the following formula Ib or Ic; 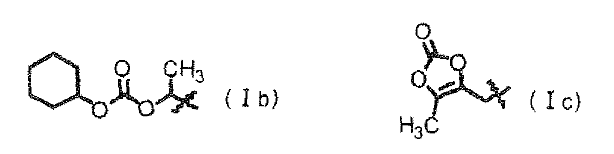 m and n are each an integer of zero or one.

## Description

### TECHNICAL FIELD

The present invention relates to compounds having TAFIa (thrombin-activated thrombin-activatable fibrinolysis inhibitor) inhibitory activity.

### Background Art

Thrombin-activatable fibrinolysis inhibitor (TAFI) is a carboxypeptidase that is activated by thrombin and thrombomodulin to cleave the lysine residues at the C terminus of the α-chain of fibrin. On the fibrin clot, tissue plasminogen activator (t-PA) and plasminogen bind to the lysine residues at the C terminus of the α-chain of fibrin, whereby plasmin is generated efficiently and fibrinolysis is eventually promoted. On the other hand, TAFIa decreases the affinity of t-PA and plasminogen for the fibrin clot and fibrinolysis activity through the cleavage of lysine residues at the C terminus of the fibrin clot. Hence, TAFIa inhibitors, which efficiently enhance the dissolution of fibrin clots but do not directly inhibit coagulation factors, are expected to contribute to the discovery of antithrombotics or fibrinolysis promoters that have higher clot specificity than the conventional anticoagulants and thrombolytics. Thereby, TAFIa inhibitors are expected to be anti-thrombosis agents that present a lower risk for bleeding and feature higher safety.

Several compounds have heretofore been reported as TAFIa inhibitors and they include thiol derivatives, phosphoric acid derivatives, imidazole derivatives and urea derivatives, all chelating with zinc at the active center of the enzyme (see PTL 1-13 and NPL 1-7). However, nothing has been known about tricyclic compounds typified by dihydroimidazoquinoline derivatives which are related to the compounds of the present invention. In addition, those known TAFIa inhibitors are not considered to have adequate activity and it is desired to develop compounds that have therapeutic effects based on the TAFIa inhibitory action and which hence are satisfactory as pharmaceuticals.

### CITATION LIST

### PATENT LITERATURE

PTL 1: Pamphlet of International Publication W02000/066557
PTL 2: Pamphlet of International Publication WO2000/066550
PTL 3: Pamphlet of International Publication WO2001/019836
PTL 4: Pamphlet of International Publication W02002/014285
PTL 5: Pamphlet of International Publication W02003/106420
PTL 6: Pamphlet of International Publication W02003/027128
PTL 7: Pamphlet of International Publication WO2003/013526
PTL 8: Pamphlet of International Publication WO2003/061652
PTL 9: Pamphlet of International Publication WO2003/061653
PTL 10: Pamphlet of International Publication WO2003/080631
PTL 11: Pamphlet of International Publication WO2005/105781
PTL 12: Pamphlet of International Publication WO2007/045339
PTL 13: Pamphlet of International Publication WO2008/067909

### NON PATENT LITERATURE

NPL 1: J. Med. Chem., Vol. 46, No. 25, pp. 5294-5297, 2003
NPL 2: Bioorganic & Medicinal Chemistry, Vol. 12, No. 5, pp. 1151-1175, 2004
NPL 3: Bioorganic & Medicinal Chemistry Letters, Vol. 14, No. 9, pp. 2141-2145, 2004
NPL 4: J. Pharmacol., Exp., Ther., Vol. 309, No. 2, pp. 607-615, 2004
NPL 5: J. Med. Chem., Vol. 50, No. 24, pp. 6095-6103, 2007
NPL 6: Bioorganic & Medicinal Chemistry Letters, Vol. 17, No. 5, pp. 1349-1354, 2007
NPL 7: Current Opinion in Drug & Development, Vol. 11, No. 4, pp. 480-486, 2008

### SUMMARY OF INVENTION

### TECHNICAL PROBLEM

An object of the present invention is to provide compounds having superior TAFIa inhibitory activity.

### SOLUTION TO PROBLEM

The present inventors conducted intensive studies with a view to attaining the stated object and found that compounds represented by the following formula (I) have superior TAFIa inhibitory activity. Some of the compounds represented by formula (I) are prodrugs for other compounds of formula (I). In the section of EXAMPLES, 5-amino-2-(4,5-dihydroimidazo[1,5-a]quinolin-3-ylmehyl)pentanoic acid derivatives were chosen as exemplary prodrugs and subjected to an animal experiment, whereupon these types of prodrug were found to increase the in vivo exposure level of the parent compound. The present invention has been accomplished on the basis of this finding.

Briefly, the present invention provides a compound represented by the following formula (I) or a pharmaceutically acceptable salt thereof:

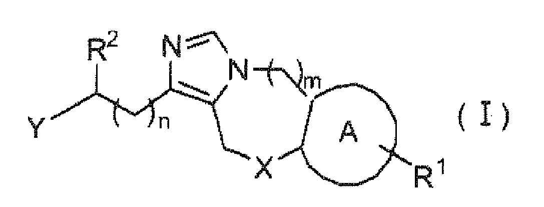

wherein A is a benzene ring or a pyridine ring;
X is the formula -(CH₂)-, the formula -(CH₂)₂-, an oxygen atom, a nitrogen atom or a single bond;
Y is the formula -(CH₂)₃-NH-R³ [where R³ is a hydrogen atom, a C₁₋₆ alkyl group, or the formula -CO₂R⁴ {where R⁴ is a C₁₋₆ alkyl group, the formula -CHR⁵OC(O)R⁶, or a substituent having the structure represented by the following formula Ia (where R⁵ is a C₁₋₆ alkyl group; R⁶ is a C₁₋₆ alkyl group, a C₃₋₈ cycloalkyl group, or a phenyl group; R⁷ is a C₁₋₆ alkyl group or a phenyl group)}], the formula - (CH₂)₄-NH-R³ or a 2-aminopyridyl group;

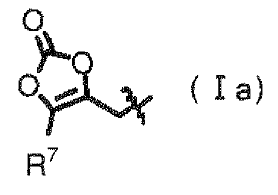

R¹ is a hydrogen atom, a halogen atom, a C₁₋₄ alkyl group substituted by 1-3 halogen atoms, a C₁₋₁₀ alkyl group, a C₁₋₈ alkoxy group, a C₃₋₈ cycloalkyl group, a C₃₋₈ cycloalkoxy group, a C₄₋₁₄ cycloalkylalkyl group, or a phenyl group;
R² is CO₂R⁸ (where R⁸ is a hydrogen atom, a C₁₋₁₀ alkyl group, or a substituent having the structure represented by the following formula Ib or Ic) or a tetrazolyl group;

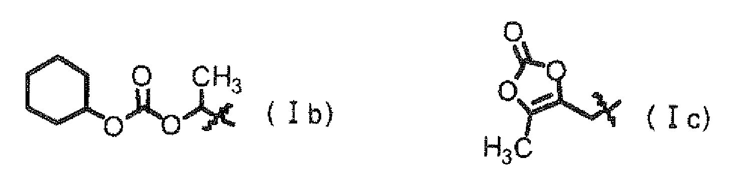

m and n are each an integer of zero or one.

In another embodiment of the present invention, the compound of formula (I) or a salt thereof is a compound represented by the following formula (II) or a pharmaceutically acceptable salt thereof: 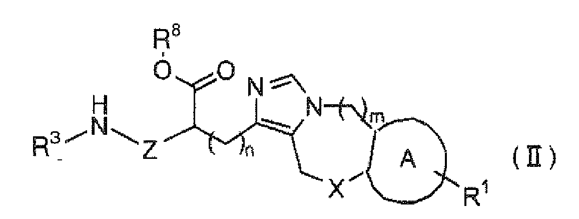 wherein Z is the formula -(CH₂)₃- or the formula -(CH₂)₄-; A, X, R¹, R³-R⁸, m and n are as defined above in connection with formula (I).

In another embodiment of the present invention, the compound of formula (II) or a salt thereof is a compound represented by the following formula (III) or a pharmaceutically acceptable salt thereof: 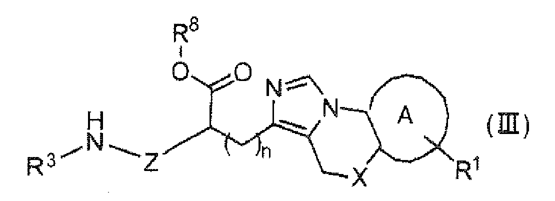
wherein X is the formula -(CH₂)-, the formula -(CH₂)₂-, an oxygen atom, or a nitrogen atom;
A, Z, R¹, R³-R⁸, and n are as defined above in connection with formula (II).

In another embodiment of the present invention, the compound of formula (III) or a salt thereof is a dihydroimidazoquinoline compound represented by the following formula (IV) or a pharmaceutically acceptable salt thereof: 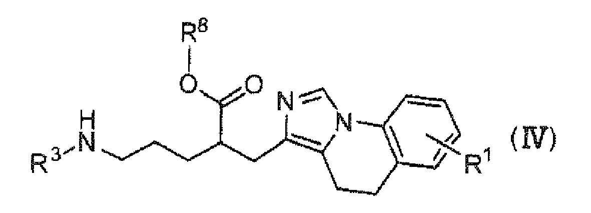 wherein R¹ and R³-R^{B} are as defined above in connection with formula (III).

In another embodiment of the present invention, the compound of formula (IV) or a salt thereof is a dihydroimidazoquinoline compound represented by the following formula (V) or a pharmaceutically acceptable salt thereof: 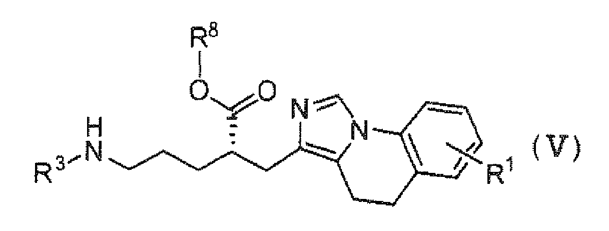 wherein R¹ and R³-R⁸ are as defined above in connection with formula (IV). The steric configuration of the asymmetric carbon atom in formula (V) is the (S)-configuration.

In another embodiment of the present invention, the compound of formula (V) or a salt thereof is a dihydroimidazoquinoline compound represented by the following formula (VI) or a pharmaceutically acceptable salt thereof: 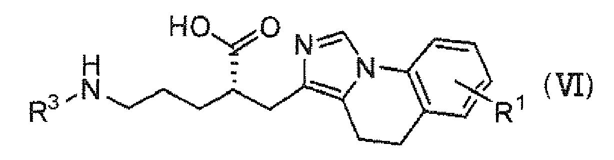 wherein R¹ and R³-R⁷ are as defined above in connection with formula (V). The steric configuration of the asymmetric carbon atom in formula (VI) is the (S)-configuration.

In another embodiment of the present invention, the compound of formula (V) or a salt thereof is a dihydroimidazoquinoline compound represented by the following formula (VII) or a pharmaceutically acceptable salt thereof: 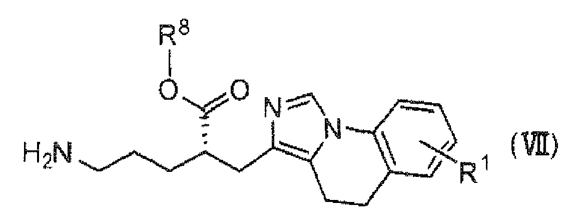 wherein R¹ and R⁸ are as defined above in connection with formula (V). The steric configuration of the asymmetric carbon atom in formula (VII) is the (S)-configuration.

In another embodiment of the present invention, the compound of formula (III) or a salt thereof is a dihydroimidazoquinoline compound represented by the following formula (VIII) or a pharmaceutically acceptable salt thereof: 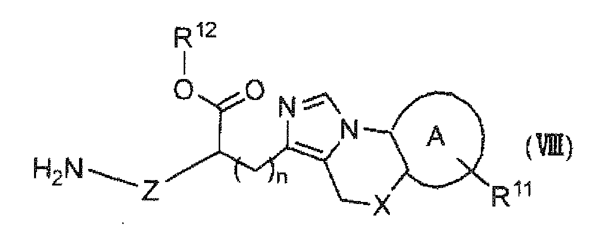
wherein A, X, Z and n are as defined above in connection with formula (III); and
R¹¹ is a hydrogen atom, a halogen atom, a C₁₋₁₀ alkyl group, a C₁₋₈ alkoxy group, a C₃₋₈ cycloalkoxy group, or a phenyl group; R¹² is a hydrogen atom or a C₁₋₆ alkyl group.

In another embodiment of the present invention, the compound of formula (VIII) or a salt thereof is a dihydroimidazoquinoline compound represented by the following formula (IX) or a pharmaceutically acceptable salt thereof: 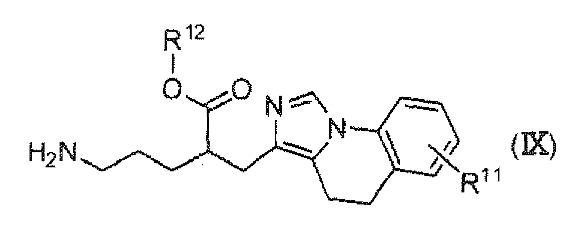 wherein R¹¹ and R¹² are as defined above in connection with formula (VIII).

In another embodiment of the present invention, the compound of formula (IX) or a salt thereof is a dihydroimidazoquinoline compound represented by the following formula (X) or a pharmaceutically acceptable salt thereof: 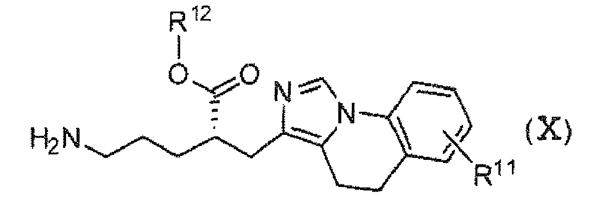 wherein R¹¹ and R¹² are as defined above in connection with formula (IX). The steric configuration of the asymmetric carbon atom in formula (X) is the (S)-configuration.

### ADVANTAGEOUS EFFECTS OF INVENTION

According to the present invention, compounds having superior TAFIa inhibitory activity can be provided.

### DESCRIPTION OF EMBODIMENTS

The present invention provides compounds of formulas (I) to (X), and pharmaceutically acceptable salts thereof, having superior TAFIa inhibitory activity.
In compounds of formulas (I)-(III) and (VIII), when A is a pyridine ring, the position of nitrogen is not limited but the pyridine ring portion is preferably represented by the following structural formula:

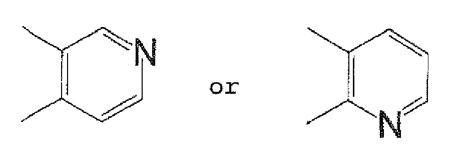

When A is a benzene ring, the position of substitution of R¹ is not limited but R¹ is preferably located at the following position:

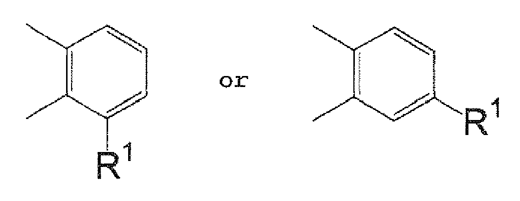

In the compound of formula (I), the position of substitution of the tetrazolyl group as R² is not limited and it may be either a 1H-tetrazol-4-yl group or a 1H-tetrazol-5-yl group, with the 1H-tetrazol-5-yl group being preferred.
In the compound of formula (I), when Y is a 2-aminopyridyl group, Y may be any one of a 2-aminopyridin-3-yl group, a 2-aminopyridin-4-yl group, a 2-aminopyridin-5-yl group and a 2-aminopyridin-6-yl group, with the 2-aminopyridin-4-yl group being preferred.

As for formula (VI), compounds where R¹ is a hydrogen atom, a halogen atom or a C₁₋₁₀ alkyl group are preferred, and compounds where R¹ is a hydrogen atom, a fluorine atom, a methyl group, a n-propyl group or an isopropyl group are more preferred. In addition, R¹ is preferably located at 6-position or 7-position, more preferably at 7-position, of the dihydroimidazoquinoline ring. As for R³, preferred compounds are such that it is a hydrogen atom or the formula

-CO₂R⁴

(where R⁴ is the formula -CHR⁵OC(O)R⁶ or a substituent having the structure represented by the following formula Ia, R⁵ is a C₁₋₆ alkyl group, R⁶ is preferably a C₁₋₆ alkyl group, a C₃₋₈ cycloalkyl group or a phenyl group, R⁷ is preferably a C₁₋₆ alkyl group), more preferably a hydrogen atom or the formula -CO₂R⁴ (where R⁴ is the formula -CHR⁵OC(O)R⁶ or a substituent having the structure represented by the following formula Ia, R⁵ is more preferably a methyl group or an isopropyl group, R⁶ is more preferably a methyl group, an ethyl group, a n-propyl group, an isopropyl group, an isobutyl group, a cyclohexyl group or a phenyl group, R⁷ is more preferably a methyl group, a n-propyl group, an isopropyl group, or a t-butyl group).

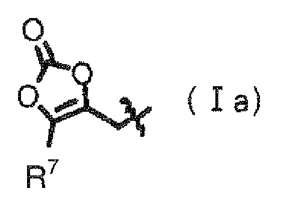

On the following pages, the compounds of the present invention are described in greater detail.

The "halogen atom" may be exemplified by a fluorine atom, a chlorine atom, a bromine atom, or an iodine atom.

The "C₁₋₄ alkyl group substituted by 1-3 halogen atoms" refers to linear or branched alkyl groups that have 1 to 4 carbon atoms and which are substituted by 1-3 halogen atoms. Examples include a trifluoromethyl group, a difluoromethyl group, a 2,2,2-trifluoroethyl group, and a 1,1-difluoroethyl group.

The "C₁₋₆ alkyl group" refers to linear or branched alkyl groups having 1 to 6 carbon atoms. Examples include a methyl group, an ethyl group, a n-propyl group, an isopropyl group, a n-butyl group, an isobutyl group, a sec-butyl group, a tert-butyl group, a n-pentyl group, an isopentyl group, a neopentyl group, a n-hexyl group, and an isohexyl group.

The "C₁₋₁₀ alkyl group" refers to linear or branched alkyl groups having 1 to 10 carbon atoms. Examples include a methyl group, an ethyl group, a n-propyl group, an isopropyl group, a n-butyl group, an isobutyl group, a sec-butyl group, a tert-butyl group, a n-pentyl group, an isopentyl group, a neopentyl group, a n-hexyl group, an isohexyl group, a n-heptyl group, a n-octyl group, a n-nonyl group, and a n-decyl group.

The "C₁₋₈ alkoxy group" refers to linear or branched alkoxy groups having 1 to 8 carbon atoms. Examples include a methoxy group, an ethoxy group, a n-propoxy group, an isopropoxy group, a n-butoxy group, an isobutoxy group, a sec-butoxy group, a tert-butoxy group, a n-pentyloxy group, an isopentyloxy group, a neopentyloxy group, a n-hexyloxy group, an isohexyloxy group, a n-heptyloxy group, and a n-octyloxy group.

The "C₃₋₈ cycloalkyl group" refers to cyclic alkyl groups having 3 to 8 carbon atoms. Examples include a cyclopropyl group, a cyclobutyl group, a cyclopentyl group, a cyclohexyl group, a cycloheptyl group, and a cyclooctyl group.

The "C₃₋₈ cycloalkoxy group" refers to cyclic alkoxy groups having 3 to 8 carbon atoms. Examples include a cyclopropoxy group, a cyclobutoxy group, a cyclopentyloxy group, a cyclohexyloxy group, a cycloheptyloxy group, and a cyclooctyloxy group.

The "C₄₋₁₄ cycloalkylalkyl group" refers to cyclic alkyl groups that have 3 to 8 carbon atoms and which are substituted by linear or branched alkyl groups having 1 to 6 carbon atoms. Examples include a cyclopropylmethyl group, a cyclopropylethyl group, a cyclobutylmethyl group, a cyclobutylethyl group, a cyclopentylmethyl group, a cyclopentylethyl group, a cyclohexylmethyl group, a cyclohexylethyl group, a cycloheptylmethyl group, a cycloheptylethyl group, a cyclooctylmethyl group, and a cyclooctylethyl group.

The compounds of the present invention are tricyclic compounds typified by dihydroimidazoquinoline derivatives, or they may be pharmaceutically acceptable salts of such compounds (either type is hereinafter called "the compounds of the present invention" as appropriate).

Examples of the pharmaceutically acceptable salts include acid addition salts such as mineral acid salts (e.g. hydrochloride, hydrobromide, hydroiodide, phosphate, sulfate, and nitrate), sulfonic acid salts (e.g. methanesulfonate, ethanesulfonate, benzenesulfonate, p-toluenesulfonate, and trifluoromethanesulfonate), and organic acid salts (e.g. oxalate, tartarate, citrate, maleate, succinate, acetate, benzoate, mandelate, ascorbate, lactate, gluconate, and malate); amino acid salts such as glycine salt, lysine salt, arginine salt, ornithine salt, glutamic acid salt, and aspartic acid salt; and inorganic salts such as lithium salt, sodium salt, potassium salt, calcium salt and magnesium salt, as well as salts with organic bases, as exemplified by ammonium salt, triethylamine salt, diisopropylamine salt, and cyclohexylamine salt. The salts may be hydrate salts.

Some of the compounds of the present invention are prodrugs. Specifically, those compounds of formula (I) in which Y is the formula -(CH₂)₃-NH-R³ or the formula -(CH₂)₄-NH-R³, with R³ being -CO₂R⁴, or those compounds of formula (I) in which R² is CO₂R⁸, with R⁸ being a substituent other than a hydrogen atom, or those compounds of formula (I) in which Y is the formula -(CH₂)₃-NH-R³ or the formula -(CH₂)₄-NH-R³ (R³ is -CO₂R⁴) and R² is CO₂R⁸ (R⁸ is a substituent other than a hydrogen atom) undergo enzymatic or chemical hydrolysis in *vivo* so that the amino group and the carboxylic acid group are deprotected, yielding compounds in which both R³ and R⁸ are a hydrogen atom and which have a strong inhibitory activity on TAFIa.

For instance, an ester derivative having a protected carboxylic acid group located on the carbon chain extending from the imidazole ring, or a carbamate derivative having a protected amino group also located on the same carbon chain, typically a compound that is represented by formula (IV) or (V) 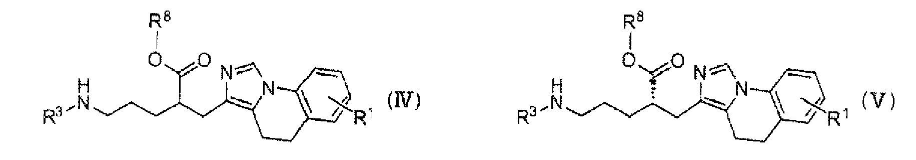 wherein either R³ or R⁸ is other than a hydrogen atom is converted to a 5-amino-2-(4,5-dihydroimidazo[1,5-a]quinolin-3-yl)pentanoic acid derivative that has the structure represented by the following formula (XI) or (XII) (where R¹ is as defined in connection with formula IV or V) and which has a strong inhibitory activity on TAFIa:

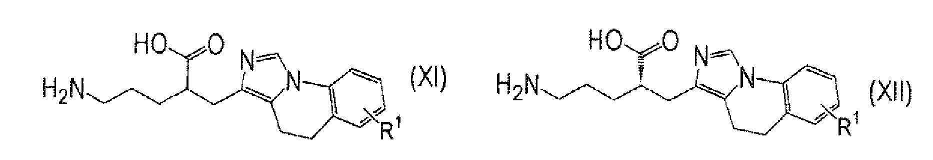

Thus, the above-described ester derivative and carbamate derivative which function as prodrugs are extremely useful compounds.

The compounds of the present invention sometimes have an asymmetric center and in that case they occur as various optical isomers or with various configurations. Hence, the compounds of the present invention may be able to occur as separate optically active substances with the (R) and (S) configurations; alternatively, they may be able to occur as a racemate or an (RS) mixture. In the case of a compound having two or more asymmetric centers, diastereomers can also occur on account of the optical isomerism of each asymmetric center. The compounds of the present invention include ones that contain all of these forms in desired proportions. For example, diastereomers can be separated by methods well known to those in the art, say, fractional crystallization, and optically active substances can be obtained by techniques in organic chemistry that are well known for this purpose. The compounds of the present invention may contain isomers such as a cis form and a trans form. The compounds of the present invention include these isomers, as well as compounds that contain these isomers in desired proportions.

The compounds of the present invention have TAFIa inhibitory activity and can be used as therapeutics or prophylactics for diseases involving TAFIa, such as deep vein thrombosis, disseminated intravascular coagulation syndrome, pulmonary embolism, cardiogenic cerebral infarction, ischemic heart disease, sepsis, pulmonary fibrosis, respiratory distress syndrome, cerebral stroke, obstructive renal disorder, Behcet's disease, mouth cancer, obesity, tissue degeneration, preeclampsia, retinal vein occlusion, inflammatory intestinal disease, arthritis, meningococcemia, and complications of kidney transplantation. The compounds of the present invention can be administered either alone or together with pharmacologically or pharmaceutically acceptable carriers or diluents. If the compounds of the present invention are to be used typically as TAFIa inhibitors, they may be administered as such either orally or parenterally. If desired, the compounds of the present invention may be administered orally or parenterally as formulations that contain them as an active ingredient. An example of the parenteral administration is intravenous administration by injection.

Since the compounds of the present invention have TAFIa inhibitory activity, patients who are suspected of the development of thrombotic diseases such as deep vein thrombosis caused by risk factors including a surgical operation such as artificial joint replacement, as well as pulmonary embolism, cardiogenic cerebral infarction and ischemic heart disease, or patients in whom the manifestation of such diseases has been confirmed may be administered with these compounds as antithrombotics or fibrinolysis promoters to prevent or treat those diseases.

The compounds of the present invention may be administered in amounts of, say, 1 mg to 1000 mg, preferably 10 mg to 200 mg, per dose, and the frequency of administration may be once to three times a day. The dosage of the compounds of the present invention can be adjusted as appropriate for the age, body weight, and symptoms of the patient under treatment.

The compounds of the present invention can be evaluated for their TAPIa activity by known procedures, such as the method described in the test procedures described hereinafter.

The methods of producing the compounds of the present invention are hereinafter described in detail but they are not particularly limited to the examples shown below. The solvents to be used in reactions may be of any kinds that do not interfere with the respective reactions and they are not particularly limited to the following description.

### Production Method 1

The compound (I) of the present invention, in which A is a benzene ring or a pyridine ring; X is the formula -(CH₂)-, the formula -(CH₂)₂-, or an oxygen atom; Y is the formula -(CH₂)₃-NH-R³ (where R³ is a hydrogen atom); R¹ is a hydrogen atom, a halogen atom, a C₁₋₄ alkyl group substituted by 1-3 halogen atoms, a C₁₋₁₀ alkyl group, a C₁₋₈ alkoxy group, a C₃₋₈ cycloalkyl group, a C₃₋₈ cycloalkoxy group, a C₄₋₁₄ cycloalkylalkyl group, or a phenyl group; R² is CO₂R⁸ (where R⁸ is a hydrogen atom); m is an integer of zero and n is an integer of one, can be synthesized by the following method (scheme 1). In scheme 1, R^{a} is R¹ or a hydroxyl group, a benzyloxy group, a triflate group, a 1-cyclohexen-1-yl group, a (1E)-1-propen-1-yl group, a (1Z)-1-propen-1-yl group, or a 1-propen-2-yl group.

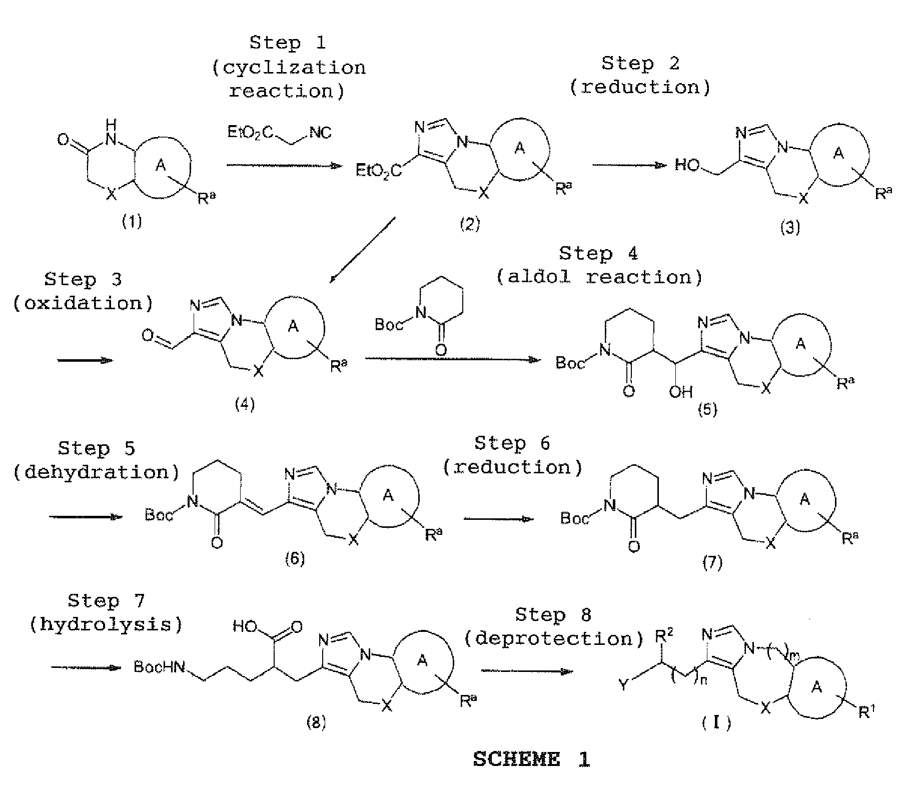

### (1) Step 1 (cyclization reaction)

Compound (1) is reacted with a suitable amide activator such as diethyl chlorophosphate in the presence of a suitable base, whereupon an intermediate can be obtained in the reaction system. The intermediate can be reacted with ethyl isocyanoacetate in the presence of a suitable base, to give compound (2). The bases to be used in this step include potassium tert-butoxide, sodium hydride, n-butyllithium, lithium diisopropylamide, lithium hexamethyldisilazane, etc. The solvents to be used in the reactions include tetrahydrofuran, diethyl ether, dioxane, toluene, etc.; the reactions can be carried out at temperatures ranging from - 78°C to room temperature.

### (2) Step 2 (reduction)

Compound (2) is reduced with a reducing agent such as lithium aluminum hydride, to give compound (3). The solvents to be used in this reaction include tetrahydrofuran, diethyl ether, dioxane, toluene, etc. The reaction can be carried out at temperatures ranging from -78°C to room temperature. Alternatively, compound (2) may be reduced with a reducing agent such as diisobutyl aluminum hydride, diisopropyl aluminum hydride, etc., to directly synthesize compound (4). The solvents to be used in this reaction include tetrahydrofuran, diethyl ether, dioxane, toluene, dichloromethane, chloroform, etc.; the reaction can be carried out at temperatures ranging from -78°C to room temperature.

### (3) Step 3 (oxidation)

Compound (3) can be reacted with a suitable oxidizing agent, optionally using a suitable base such as triethylamine or diisopropylethylamine, to give compound (4). The oxidizing agents to be used in this step include dimethyl sulfoxide-oxalyl chloride, dimethyl sulfoxide-N,N'-dicyclohexylcarbodiimide (DCC), dimethyl sulfoxide-1-chloropyrrolidine-2,5-dione (NCS), dimethyl sulofixde-acetic anhydride, manganese dioxide, Dess-Martin periodinane, piridinium chlorochromate (PCC), piridinium dichromate (PDC), etc. The solvents to be used in this reaction include dichloromethane, chloroform, 1,2-dichloroethane, tetrahydrofuran, diethyl ether, dioxane, toluene, etc.; the reaction can be carried out at temperatures ranging from -78°C to room temperature.

### (4) Step 4 (aldol reaction)

Compound (4) can be reacted with a 6-membered cyclic lactam in the presence of a suitable base, to give compound (5). The bases to be used in this step include potassium tert-butoxide, sodium hydride, n-butyllithium, lithium diisopropylamide, lithium hexamethyldisilazane, etc. The solvents to be used in the reaction include tetrahydrofuran, diethyl ether, dioxane, toluene, etc.; the reaction can be carried out at temperatures ranging from -78°C to room temperature.

### (5) Step 5 (dehydration)

A suitable electrophile is allowed to act on the hydroxyl group of compound (5) and reaction is performed using a suitable base, to give compound (6). The electrophiles to be used in this step include methanesulfonyl chloride, p-toluenesulfonyl chloride, acetyl chloride, trifluoromethanesulfonyl chloride, acetic anhydride, etc. The bases to be used in the reaction include triethylamine, diisopropylethylamine, etc. The solvents to be used in the reaction include dichloromethane, chloroform, 1,2-dichloroethane, tetrahydrofuran, toluene, etc.; the reaction can be carried out at temperatures ranging from 0°C to room temperature.

### (6) Step 6 (reduction)

Compound (6) is catalytically hydrogenated in a hydrogen atmosphere using a catalyst such as palladium-activated carbon, palladium hydroxide, or platinum-activated carbon, to give compound (7). The solvents to be used in this reaction include methanol, ethanol, isopropanol, ethyl acetate, tetrahydrofuran, mixtures thereof, etc; the reaction can be carried out at temperatures ranging from room temperature to the reflux temperature.

### (7) Step 7 (hydrolysis)

Compound (7) is hydrolyzed using a suitable base, to give compound (8). The bases to be used in this step include lithium hydroxide, sodium hydroxide, potassium hydroxide, etc. The solvents to be used in this reaction include methanol, ethanol, isopropanol, tetrahydrofuran, water, mixtures thereof, etc; the reaction can be carried out at temperatures ranging from 0°C to the reflux temperature.

### (8) Step 8 (deprotection)

Compound (8) is deprotected using a suitable acid, whereupon the compound (I) of the present invention can be synthesized. The suitable acids to be used in this step include hydrochloric acid, sulfuric acid, hydrobromic acid, trifluoroacetic acid, methanesulfonic acid, benzenesulfonic acid, p-toluenesulfonic acid, 10-camphorsulfonic acid, etc. The solvents to be used in this reaction include chloroform, dichloromethane, methanol, ethanol, isopropanol, ethyl acetate, tetrahydrofuran, diethyl ether, dioxane, toluene, water, etc.; the reaction can be carried out at temperatures ranging from 0°C to the reflux temperature.
In the case where R^{a} is a halogen atom, a hydroxyl group, a benzyloxy group, a triflate group, a 1-cyclohexen-1-yl group, a (1E)-1-propen-1-yl group, a (1Z)-1-propen-1-yl group, a 1-propen-2-yl group or the like, a suitable intermediate in scheme 1 may be subjected to transformation of a functional group, as by a coupling reaction typified by the Suzuki coupling, the Mitsunobu reaction, an alkylation reaction or a reduction reaction, so as to yield the compound of the present invention (I).

### Production Method 2

The compound (I) of the present invention, in which A is a benzene ring or a pyridine ring; X is a nitrogen atom; Y is the formula -(CH₂)₃-NH-R³ (where R³ is a hydrogen atom); R¹ is a hydrogen atom, a halogen atom, a C₁₋₄ alkyl group substituted by 1-3 halogen atoms, a C₁₋₁₀ alkyl group, a C₁₋₈ alkoxy group, a C₃₋₈ cycloalkyl group, a C₃₋₈ cycloalkoxy group, a C₄₋₁₄ cycloalkylalkyl group, or a phenyl group; R² is CO₂R^{B} (where R⁸ is a hydrogen atom); m is an integer of zero and n is an integer of one, can be synthesized by the following method (scheme 2). In scheme 2, R^{a} is R¹ or a hydroxyl group, a benzyloxy group, a triflate group, a 1-cyclohexen-1-yl group, a (1E)-1-propen-1-yl group, a (1Z)-1-propen-1-yl group, or a 1-propen-2-yl group.

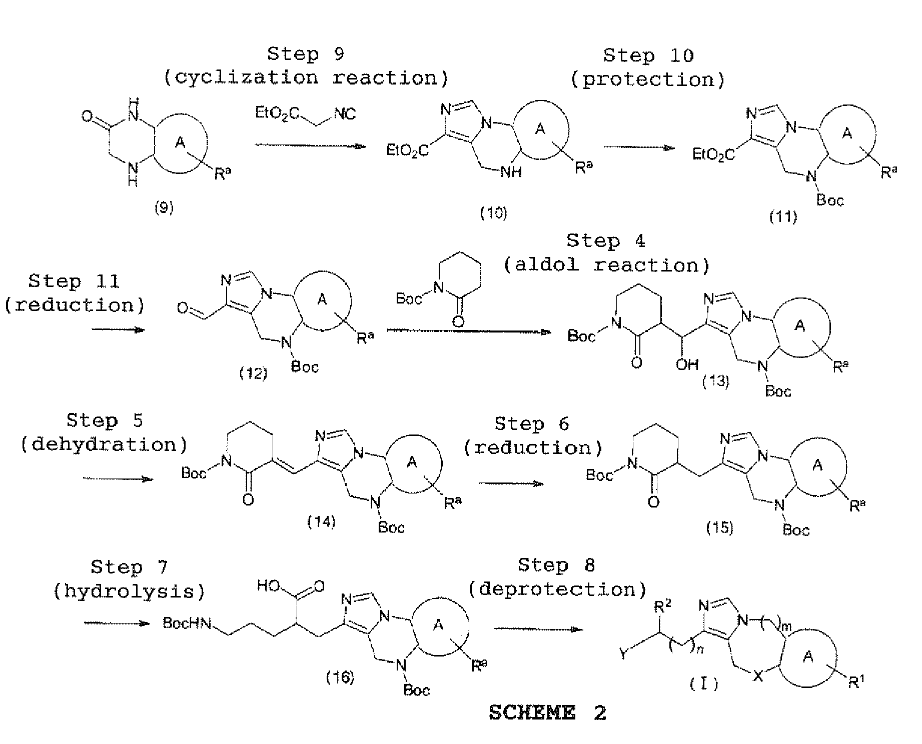

### (9) Step 9 (cyclization reaction)

Compound (9) is reacted with a suitable amide activator such as diethyl chlorophosphate in the presence of a suitable base, whereupon an intermediate can be obtained in the reaction system. The intermediate can be reacted with ethyl isocyanoacetate in the presence of a suitable base, to give compound (10). The bases to be used in this step include potassium tert-butoxide, sodium hydride, n-butyllithium, lithium diisopropylamide, lithium hexamethyldisilazane, etc. The solvents to be used in the reactions include tetrahydrofuran, diethyl ether, dioxane, toluene, etc.; the reactions can be carried out at temperatures ranging from - 78°C to room temperature.

### (10) Step 10 (protection)

Compound (10) can be reacted with di-tert-butyl dicarbonate in the presence of a suitasble base, to give compound (11). The bases to be used in this step include triethylamine, diisopropylethylamine, pyridine, 4-dimethylaminopyridine, potassium tert-butoxide, sodium hydride, n-butyllithium, lithium diisopropylamide, lithium hexamethyldisilazane, etc. The solvents to be used in the reaction include tetrahydrofuran, diethyl ether, dioxane, toluene, etc.; the reaction can be carried out at temperatures ranging from -78°C to the reflux temperature.

### (11) Step 11 (reduction)

Compound (11) is reduced with a reducing agent such as diisobutylaluminum hydride or diisopropylaluminum hydride, to give compound (12). The solvents to be used in this reaction include tetrahydrofuran, diethyl ether, dioxane, toluene, dichloromethane, chloroform, etc.; the reaction can be carried out at temperatures ranging from 78°C to room temperature. Alternatively, compound (12) may be synthesized from compound (11) via an alcohol form, as in steps 2 and 3 described in production method 1.
From compound (12), the compound (I) of the present invention can be synthesized by the same procedures as steps 4 to 8 described in production method 1.
In the case where R^{a} is a halogen atom, a hydroxyl group, a benzyloxy group, a triflate group, a 1-cyclohexen-1-yl group, a (1E)-1-propen-1-yl group, a (1Z)-1-propen-1-yl group, a 1-propen-2-yl group or the like, a suitable intermediate in scheme 2 may be subjected to transformation of a functional group, as by a coupling reaction typified by the Suzuki coupling, the Mitsunobu reaction, an alkylation reaction or a reduction reaction, so as to yield the compound (I) of the present invention.

### Production Method 3

The compound (I) of the present invention, in which A is a benzene ring or a pyridine ring; X is the formula -(CH₂)-, the formula -(CH₂)₂-, or an oxygen atom; Y is the formula -(CH₂)₄-NH-R³ (where R³ is a hydrogen atom); R¹ is a hydrogen atom, a halogen atom, a C₁₋₄ alkyl group substituted by 1-3 halogen atoms, a C₁₋₁₀ alkyl group, a C₁₋₈ alkoxy group, a C₃₋₈ cycloalkyl group, a C₃₋₈ cycloalkoxy group, a C₄₋₁₄ cycloalkylalkyl group, or a phenyl group; R² is CO₂R⁸ (where R⁸ is a hydrogen atom); m is an integer of zero and n is an integer of zero, can be synthesized by the following method (scheme 3). In scheme 3, R^{a} is R¹ or a hydroxyl group, a benzyloxy group, a triflate group, a 1-cyclohexen-1-yl group, a (1E)-1-propen-1-yl group, a (1Z)-1-propen-1-yl group, or a 1-propen-2-yl group.

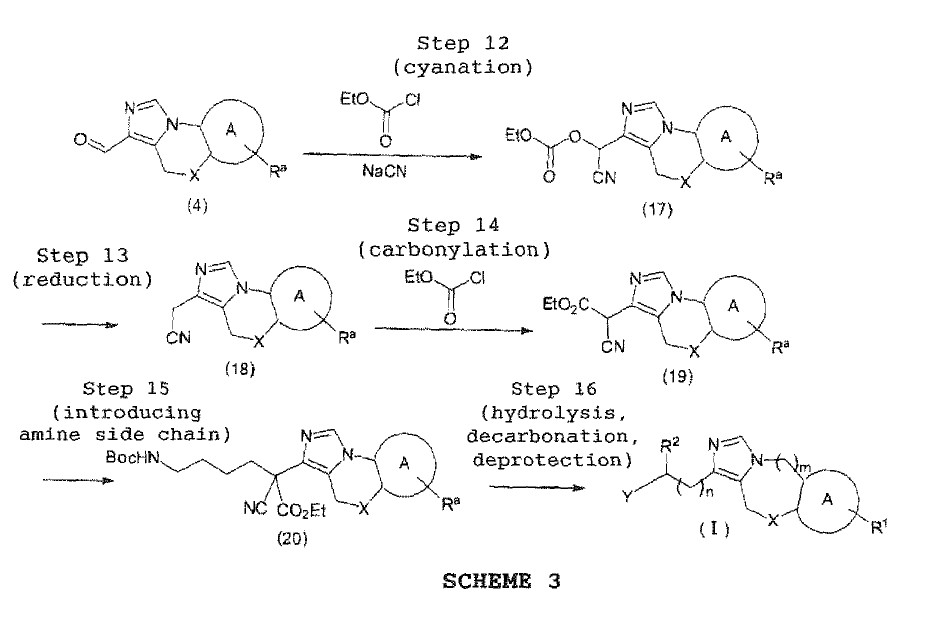

### (12) Step 12 (cyanation)

Compound (4) can be reacted with a cyanation agent such as sodium cyanide or potassium cyanide or a carbonation agent such as ethyl chloroformate, optionally in the presence of a phase transfer catalyst such as tetrabutylammonium chloride, to give compound (17). The solvents to be used in this reaction include dichloromethane, chloroform, 1,2-dichloroethane, tetrahydrofuran, toluene, water, etc.; the reaction can be carried out at temperatures ranging from 0°C to room temperature.

### (13) Step 13 (reduction)

Compound (17) is catalytically hydrogenated in a hydrogen atmosphere using a catalyst such as palladium-activated carbon, palladium hydroxide, or platinum-activated carbon, to give compound (18). The solvents to be used in this reaction include methanol, ethanol, isopropanol, ethyl acetate, tetrahydrofuran, mixtures thereof, etc; the reaction can be carried out at temperatures ranging from room temperature to the reflux temperature.

### (14) Step 14 (carbonylation)

Compound (18) can be reacted with ethyl chloroformate in the presence of a suitasble base, to give compound (19). The bases to be used in this step include potassium tert-butoxide, sodium hydride, lithium diisopropylamide, lithium hexamethyldisilazane, etc. The solvents to be used in the reaction include tetrahydrofuran, diethyl ether, dioxane, toluene, etc.; the reaction can be carried out at temperatures ranging from -78°C to room temperature.

### (15) Step 15 (reaction for introducing amine side chain)

Compound (19) can be reacted with an alkyl halide such as tert-butyl 4-iodobutyl carbamate in the presence of a suitable base, to give compound (20). The bases to be used in this step include potassium tert-butoxide, sodium hydride, etc. The solvents to be used in the reaction include N,N-dimethylformamide, N,N-dimethyl acetamide, N-methylpyrrolidone etc.; the reaction can be carried out at temperatures ranging from -78°C to the reflux temperature.

### (16) Step 16 (hydrolysis, decarbonation, deprotection)

Compound (20) is hydrolyzed, decarbonated and deprotected simultaneously using a suitable acid, to give the compound (I) of the present invention. The suitable acids to be used in this step include hydrochloric acid, sulfuric acid, hydrobromic acid, trifluoroacetic acid, methanesulfonic acid, benzenesulfonic acid, p-toluenesulfonic acid, 10-camphorsulfonic acid, etc. The solvents to be used in these reactions include chloroform, dichloromethane, methanol, ethanol, isopropanol, ethyl acetate, tetrahydrofuran, diethyl ether, dioxane, toluene, water, etc.; the reactions can be carried out at temperatures ranging from 0°C to the reflux temperature.
In the case where R^{a} is a halogen atom, a hydroxyl group, a benzyloxy group, a triflate group, a 1-cyclohexen-1-yl group, a (1E)-1-propen-1-yl group, a (1Z)-1-propen-1-yl group, a 1-propen-2-yl group or the like, a suitable intermediate in scheme 3 may be subjected to transformation of a functional group, as by a coupling reaction typified by the Suzuki coupling, the Mitsunobu reaction, an alkylation reaction or a reduction reaction, so as to yield the compound (I) of the present invention.

### Production Method 4

The compound (V) of the present invention, in which R¹ is a hydrogen atom, a halogen atom, a C₁₋₄ alkyl group substituted by 1-3 halogen atoms, a C₁₋₁₀ alkyl group, a C₁₋₈ alkoxy group, a C₃₋₈ cycloalkyl group, a C₃₋₈ cycloalkoxy group, a C₄₋₁₄ cycloalkylalkyl group, or a phenyl group; R³ is a hydrogen atom, and R⁸ is a hydrogen atom, can be synthesized by the following method (scheme 4).

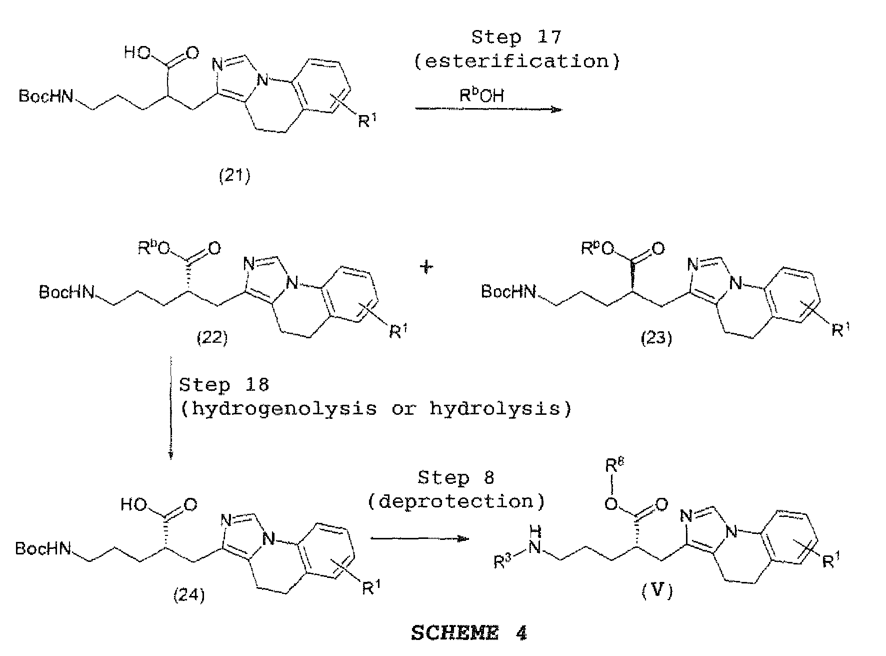

### (17) Step 17 (esterification)

Compound (21) synthesized by the same procedures as steps 1 to 7 described in Production Method 1 can be reacted with a chiral alcohol (R^{b}OH) such as (1R,2S)-1-phenyl-2-(pyrrolidin-1-yl)propan-1-ol, (1R)-1-phenylethanol, (1S,2R,5S)-5-methyl-2-(propan-2-yl)cyclohexanol, or (3R)-3-hydroxy-4,4-dimethyldihydrofuran-2(3H)-one using a condensing agent in the presence or absence of a suitable base, to give compound (22) and compound (23) that are diastereomers separable by silica gel column chromatography. Suitable bases include triethylamine, diisopropylamine, pyridine, 4-dimethylaminopyridine, etc. The condensing agents to be used in this step include N-[3-(dimethylamino)propyl]-N'-ethylcarbodiimide hydrochloride, N,N'-dicyclohexylcarbodiimide, di-1H-imidazol-1-yl-methanone, etc. The solvents to be used in the reaction include dichloromethane, chloroform, 1,2-dichloroethane, tetrahydrofuran, toluene, N,N-dimethylformamide, etc.; the reaction can be carried out at temperatures ranging from 0°C to the reflux temperature.

### (18) Step 18 (hydrogenolysis or hydrolysis)

Compound (22) is catalytically hydrogenated in a hydrogen atmosphere using a catalyst such as palladium-activated carbon, palladium hydroxide, or platinum-activated carbon, to give compound (24). The solvents to be used in this reaction include methanol, ethanol, isopropanol, ethyl acetate, tetrahydrofuran, mixtures thereof, etc; the reaction can be carried out at temperatures ranging from room temperature to the reflux temperature. Alternatively, compound (22) may be hydrolyzed using a suitable base, to give compound (24) can. The bases to be used in this step include lithium hydroxide, sodium hydroxide, potassium hydroxide, etc. The solvents to be used in this reaction include methanol, ethanol, isopropanol, tetrahydrofuran, water, mixtures thereof, etc; the reactions can be carried out at temperatures ranging from 0°C to the reflux temperature.
From compound (24), the compound (V) of the present invention can be synthesized by the same procedure as step 8 described in production method 1.

### Production Method 5

The compound (IV) of the present invention, in which R¹ is a hydrogen atom, a halogen atom, a C₁₋₄ alkyl group substituted by 1-3 halogen atoms, a C₁₋₁₀ alkyl group, a C₁₋₈ alkoxy group, a C₃₋₈ cycloalkyl group, a C₃₋₈ cycloalkoxy group, a C₄₋₁₄ cycloalkylalkyl group, or a phenyl group; R³ is a hydrogen atom; and R⁸ is a C₁₋₁₀ alkyl group, can be synthesized by the following method (scheme 5).

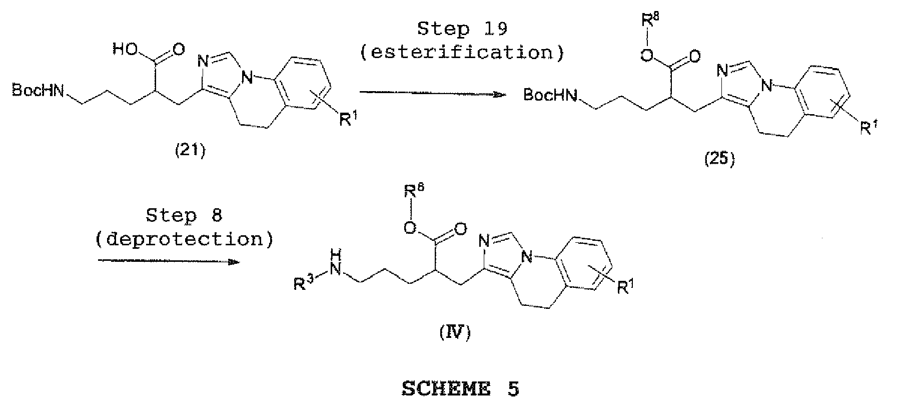

### (19) Step 19 (esterification)

Compound (21) can be reacted with an alcohol such as methanol, ethanol or propanol using a condensing agent in the presence or absence of a suitable base, to give compound (25). Suitable bases include triethylamine, diisopropylamine, pyridine, 4-dimethylaminopyridine, etc. The condensing agents to be used in this step include N-[3-(dimethylamino)propyl]-N'-ethylcarbodiimide hydrochloride, N,N'-dicyclohexylcarbodiimide, di-1H-imidazol-1-yl-methanone, etc. The solvents to be used in the reaction include dichloromethane, chloroform, 1,2-dichloroethane, tetrahydrofuran, toluene, N,N-dimethylformamide, etc.; the reaction can be carried out at temperatures ranging from 0°C to the reflux temperature. Alternatively, compound (21) may be reacted with an alkyl halide such as methyl iodide, ethyl iodide or propyl iodide in the presence of a suitable base, to give compound (25). Suitable bases include potassium carbonate, cesium carbonate, etc. The solvents to be used in this reaction include tetrahydrofuran, toluene, N,N-dimethylformamide, acetone, etc.; the reaction can be carried out at temperatures ranging from 0°C to the reflux temperature. As a further approach, compound (21) may be reacted with an alcohol such as methanol, ethanol or propanol using a suitable azo reagent in the presence of a suitable phosphine reagent, to give compound (25). Suitable phosphine reagents include triphenylphosphine, tri-n-butylphosphine, tri-tert-butylphosphine, etc. Suitable azo reagents include diethyl azodicarboxylate, diisopropyl azodicarboxylate, tetramethyl azodicarboxamide, azodicarbonyl dipiperidine, etc.
From compound (25), the compound (IV) of the present invention can be synthesized by the same procedure as step 8 described in production method 1.

### Production Method 6

The compound (IV) of the present invention, in which R¹ is a hydrogen atom, a halogen atom, a C₁₋₄ alkyl group substituted by 1-3 halogen atoms, a C₁₋₁₀ alkyl group, a C₁₋₈ alkoxy group, a C₃₋₈ cycloalkyl group, a C₃₋₈ cycloalkoxy group, a C₄₋₁₄ cycloalkylalkyl group, or a phenyl group; R³ is a C₁₋₆ alkyl group; and R⁸ is a hydrogen atom, can be synthesized by the following method (scheme 6).

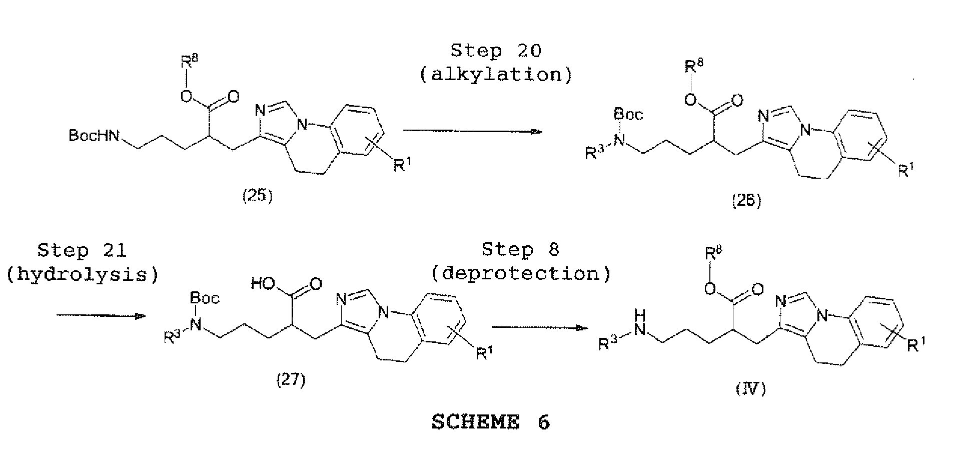

### (20) Step 20 (alkylation)

Compound (25) can be reacted with an alkyl halide such as methyl iodide or ethyl iodide in the presence of a suitable base, to give compound (26). Suitable bases include potassium tert-butoxide, sodium hydride, etc. The solvents to be used in the reaction include N,N-dimethylformamide, N,N-dimethyl acetamide, N-methylpyrrolidone, tetrahydrofuran, toluene, etc.; the reaction can be carried out at temperatures ranging from 0°C to the reflux temperature.

### (21) Step 21 (hydrolysis)

Compound (26) can be hydrolyzed with a suitable base, to give compound (27). The bases to be used in this step include lithium hydroxide, sodium hydroxide, potassium hydroxide, etc. The solvents to be used in this reaction include methanol, ethanol, isopropanol, tetrahydrofuran, water, mixtures thereof, etc; the reaction can be carried out at temperatures ranging from 0°C to the reflux temperature.
From compound (27), the compound (IV) of the present invention can be synthesized by the same procedure as step 8 described in production method 1.

### Production Method 7

The compound (II) of the present invention, in which A is a benzene ring; X is a single bond; Z is the formula -(CH₂)₃-; R³ is a hydrogen atom; R¹ is a hydrogen atom, a halogen atom, a C₁₋₄ alkyl group substituted by 1-3 halogen atoms, a C₁₋₁₀ alkyl group, a C₁₋₈ alkoxy group, a C₃₋₈ cycloalkyl group, a C₃₋₈ cycloalkoxy group, a C₄₋₁₄ cycloalkylalkyl group, or a phenyl group; R⁸ is a hydrogen atom; m is an integer of one; and n is an integer of one, can be synthesized by the following method (scheme 7).

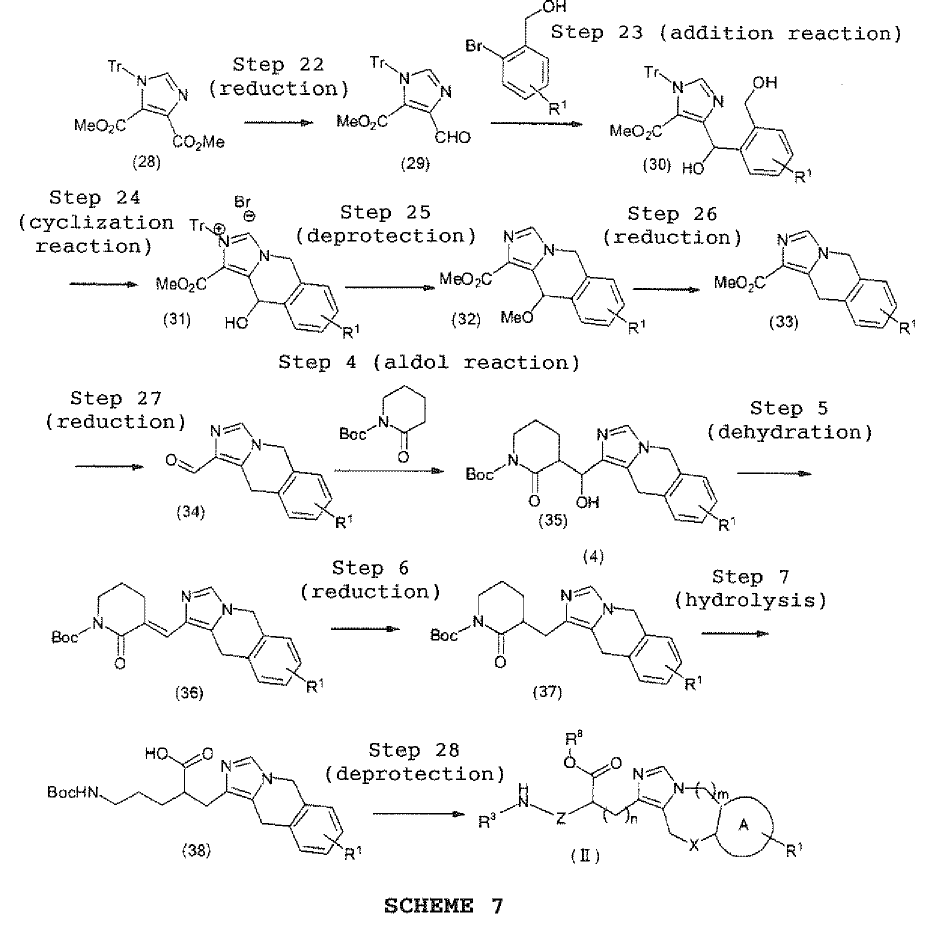

### (22) Step 22 (reduction)

Compound (28) can be reduced with a reducing agent such as diisobutyl aluminum hydride, diisopropyl aluminum hydride, etc., to give compound (29). The solvents to be used in this reaction include tetrahydrofuran, diethyl ether, dioxane, toluene, dichloromethane, chloroform, etc.; the reaction can be carried out at temperatures ranging from -78°C to room temperature. Alternatively, compound (29) may be synthesized from compound (28) via an alcohol form as in steps 2 and 3 described in production method 1.

### (23) Step 23 (addition reaction)

Compound (29) can be reacted with a bromobenzene preliminarily treated with a suitable base, to give compound (30). The bases to be used in this step include n-butyllithium, sec-butyllithium, tert-butyllithium, etc. The solvents to be used in the reaction include tetrahydrofuran, diethyl ether, dioxane, toluene, etc.; this reaction can be carried out at temperatures ranging from -78°C to room temperature.

### (24) Step 24 (cyclization reaction)

The primary hydroxyl group of compound (30) can be brominated and heated, to give compound (31). The brominating agents to be used in this step include carbon tetrabromide-triphenylphosphine, 1-bromopyrrolidine-2,5-dione (NBS)-triphenylphosphine, bromine-triphenylphosphine, hydrogen bromide-acetic acid, phosphorus tribromide, 1-bromopyrrolidine-2,5-dione (NBS), etc. The solvents to be used in the bromination reaction include dichloromethane, chloroform, tetrahydrofuran, diethyl ether, dioxane, toluene, N,N-dimethylformamide, etc.; this reaction can be carried out at temperatures ranging from 0°C to the reflux temperature. The cyclization reaction can be carried out at temperatures ranging from room temperature to the reflux temperature.

### (25) Step 25 (deprotection)

Compound (31) can be heated using methanol, to give compound (32). This reaction can be carried out at temperatures ranging from room temperature to the reflux temperature.

### (26) Step 26 (reduction)

Compound (32) is catalytically hydrogenated in a hydrogen atmosphere using a catalyst such as palladium-activated carbon, palladium hydroxide, or platinum-activated carbon, to give compound (33). The solvents to be used in this reaction include methanol, ethanol, isopropanol, ethyl acetate, tetrahydrofuran, mixtures thereof, etc; the reaction can be carried out at temperatures ranging from room temperature to the reflux temperature.

### (27) Step 27 (reduction)

Compound (33) can be reduced with a reducing agent such as diisobutyl aluminum hydride, diisopropyl aluminum hydride, etc., to give compound (34). The solvents to be used in this reaction include tetrahydrofuran, diethyl ether, dioxane, toluene, dichloromethane, chloroform, etc.; the reaction can be carried out at temperatures ranging from -78°C to room temperature. Alternatively, compound (34) may be synthesized from compound (33) via an alcohol form as in steps 2 and 3 described in production method 1.
From compound (34), the compound (II) of the present invention can be synthesized by the same procedure as steps 4 to 8 described in production method 1.

### Production Method 8

The compound (I) of the present invention, in which A is a benzene ring or a pyridine ring; X is the formula -(CH₂)-, the formula -(CH₂)₂-, an oxygen atom, or a nitrogen atom; Y is a 2-aminopyridyl group; R¹ is a hydrogen atom, a halogen atom, a C₁₋₄ alkyl group substituted by 1-3 halogen atoms, a C₁₋₁₀ alkyl group, a C₁₋₈ alkoxy group, a C₃₋₈ cycloalkyl group, a C₃₋₈ cycloalkoxy group, a C₄₋₁₄ cycloalkylalkyl group, or a phenyl group; R² is CO₂R⁸ (where R⁸ is a hydrogen atom); m is an integer of zero and n is an integer of one, can be synthesized by the following method (scheme 8). In scheme 8, R^{a} is R¹ or a hydroxyl group, a benzyloxy group, a triflate group, a 1-cyclohexen-1-yl group, a (1E)-1-propen-1-yl group, a (1Z)-1-propen-1-yl group, or a 1-propen-2-yl group.

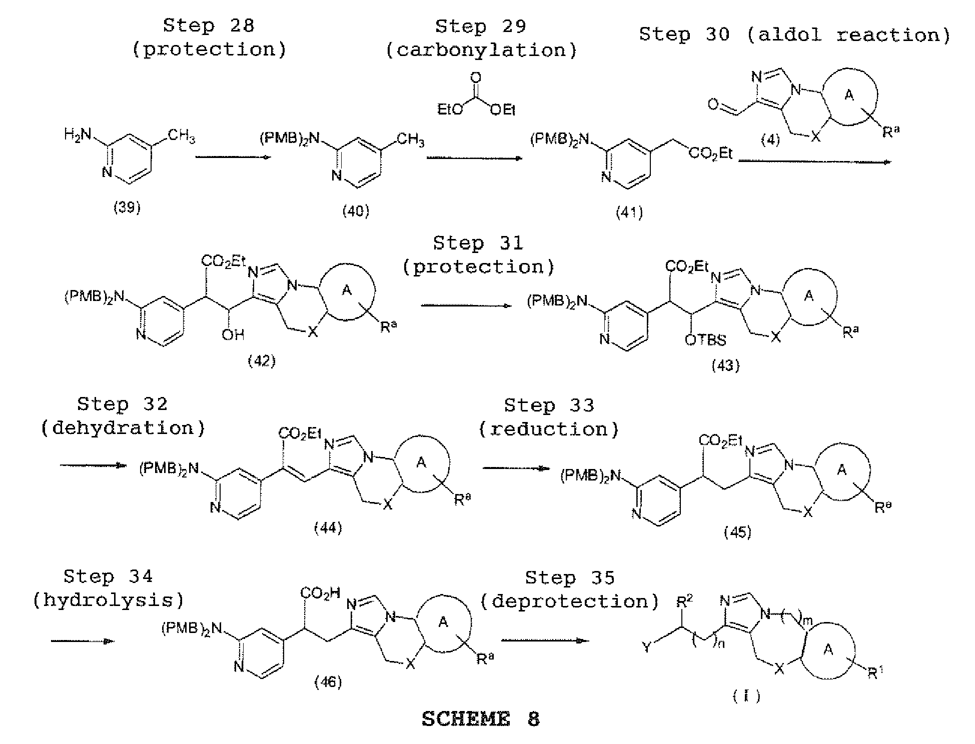

### (28) Step 28 (protection)

Compound (39) can be reacted with p-methoxybenzyl chloride in the presence of a suitasble base, to give compound (40). The bases to be used in this step include potassium tert-butoxide, sodium hydride, n-butyllithium, lithium diisopropylamide, lithium hexamethyldisilazane, etc. The solvents to be used in the reaction include N,N-dimethylformamide, N,N-dimethyl acetamide, N-methylpyrrolidone, tetrahydrofuran, diethyl ether, dioxane, toluene, etc.; the reaction can be carried out at temperatures ranging from -78°C to the reflux temperature.

### (29) Step 29 (carbonylation)

Compound (40) can be reacted with diethyl carbonate in the presence of a suitable base, to give compound (41). The bases to be used in this step include potassium tert-butoxide, sodium hydride, n-butyllithium, lithium diisopropylamide, lithium hexamethyldisilazane, etc. The solvents to be used in the reaction include tetrahydrofuran, diethyl ether, dioxane, toluene, etc.; the reaction can be carried out at temperatures ranging from -78°C to room temperature.

### (30) Step 30 (aldol reaction)

Compound (41) can be reacted with compound (4) in the presence of a suitable base, to give compound (42). The bases to be used in this step include potassium tert-butoxide, sodium hydride, n-butyllithium, lithium diisopropylamide, lithium hexamethyldisilazane, etc. The solvents to be used in the reaction include tetrahydrofuran, diethyl ether, dioxane, toluene, etc.; the reaction can be carried out at temperatures ranging from -78°C to room temperature.

### (31) Step 31 (protection)

A suitable electrophile is allowed to act on the hydroxyl group of compound (42) and reaction is performed using a suitable base, to give compound (43). The electrophiles to be used in this step include tert-butyldimethylsilyl chloride, trimethylsilyl chloride, methanesulfonyl chloride, p-toluenesulfonyl chloride, acetyl chloride, trifluoromethanesulfonyl chloride, acetic anhydride, etc. The bases to be used in the reaction include imidazole, triethylamine, diisopropylethylamine, pyridine, etc. The solvents to be used in this reaction include dichloromethane, chloroform, 1,2-dichloroethane, tetrahydrofuran, toluene, etc.; the reaction can be carried out at temperatures ranging from 0°C to room temperature.

### (32) Step 32 (dehydration)

Compound (43) can be subjected to reaction in a suitable solvent in the presence of a suitable base, to give compound (44). The bases to be used in this step include diazabicycloundecene, triethylamine, diisopropylamine, pyridine, 4-dimethylaminopyridine, potassium tert-butoxide, sodium hydride, n-butyllithium, lithium diisopropylamide, lithium hexamethyldisilazane, etc. The solvents to be used in the reaction include N,N-dimethylformamide, N,N-dimethyl acetamide, N-methylpyrrolidone, tetrahydrofuran, diethyl ether, dioxane, toluene, etc.; the reaction can be carried out at temperatures ranging from -78°C to the reflux temperature.

### (33) Step 33 (reduction)

Compound (44) is catalytically hydrogenated in a hydrogen atmosphere using a catalyst such as palladium-activated carbon, palladium hydroxide, or platinum-activated carbon, to give compound (45). The solvents to be used in this reaction include methanol, ethanol, isopropanol, ethyl acetate, tetrahydrofuran, mixtures thereof, etc; the reaction can be carried out at temperatures ranging from room temperature to the reflux temperature.

### (34) Step 34 (hydrolysis)

Compound (45) can be hydrolyzed with a suitable base, to give compound (46). The bases to be used in this step include lithium hydroxide, sodium hydroxide, potassium hydroxide, etc. The solvents to be used in this reaction include methanol, ethanol, isopropanol, tetrahydrofuran, water, mixtures thereof, etc; the reaction can be carried out at temperatures ranging from 0°C to the reflux temperature.

### (35) Step 35 (deprotection)

Compound (46) is deprotected with a suitable acid, whereupon the compound (I) of the present invention can be synthesized. The suitable acids to be used in this step include hydrochloric acid, sulfuric acid, hydrobromic acid, trifluoroacetic acid, methanesulfonic acid, benzenesulfonic acid, p-toluenesulfonic acid, 10-camphorsulfonic acid, etc. The solvents to be used in this reaction include chloroform, dichloromethane, methanol, ethanol, isopropanol, ethyl acetate, tetrahydrofuran, diethyl ether, dioxane, toluene, water, etc.; the reaction can be carried out at temperatures ranging from 0°C to the reflux temperature.
In the case where R^{a} is a halogen atom, a hydroxyl group, a benzyloxy group, a triflate group, a 1-cyclohexen-1-yl group, a (1E)-1-propen-1-yl group, a (1Z)-1-propen-1-yl group, a 1-propen-2-yl group or the like, a suitable intermediate in scheme 8 may be subjected to transformation of a functional group, as by a coupling reaction typified by the Suzuki coupling, the Mitsunobu reaction, an alkylation reaction or a reduction reaction, so as to yield the compound (I) of the present invention.

### Production Method 9

The compound (I) of the present invention, in which A is a benzene ring; X is the formula -(CH₂)-; Y is the formula - (CH₂)3-NH-R³ (where R³ is a hydrogen atom); R¹ is a hydrogen atom, a halogen atom, a C₁₋₄ alkyl group substituted by 1-3 halogen atoms, a C₁₋₁₀ alkyl group, a C₁₋₈ alkoxy group, a C₃₋₈ cycloalkyl group, a C₃₋₈ cycloalkoxy group, a C₄₋₁₄ cycloalkylalkyl group, or a phenyl group; R² is a tetrazolyl group; m is an integer of zero and n is an integer of one, can be synthesized by the following method (scheme 9).

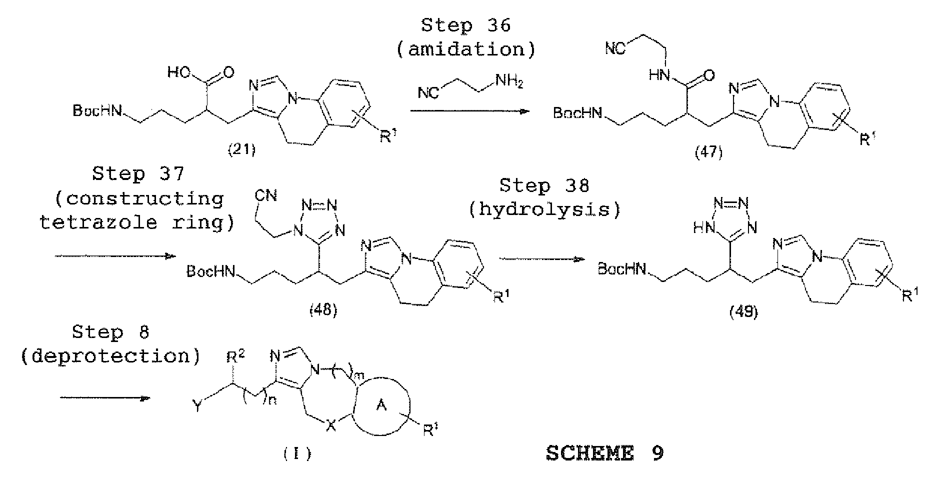

### (36) Step 36 (amidation)

Compound (21) and 3-aminopropanenitrile can be reacted using a condensing agent in the presence or absence of a suitable base and in the presence or absence of a suitable additive, to give compound (47). Suitable bases include triethylamine, diisopropylamine, pyridine, 4-dimethylaminopyridine, etc. Suitable additivs include 1H-benzotriazol-1-ol, 1-hydroxypyrrolidine-2,5-dione, 3-hydroxy-1,2,3-benzotriazin-4(3H)-one, etc. The condensing agents to be used in this step include N-[3-(dimethylamino)propyl]-N'-ethylcarbodiimide hydrochloride, N,N'-dicyclohexylcarbodiimide, di-1H-imidazol-1-ylmethanone, etc. The solvents to be used in the reaction include dichloromethane, chloroform, 1,2-dichloroethane, tetrahydrofuran, toluene, N,N-dimethylformamide, etc.; the reaction can be carried out at temperatures ranging from 0°C to the reflux temperature.

### (37) Step 37 (constructing tetrazole ring)

Compound (47) and trimethylsilyl azide can be reacted using a suitable azo reagent in the presence of a suitable phosphine reagent, to give compound (48). Suitable phosphine reagents include triphenylphosphine, tri-n-butylphosphine, tri-tert-butylphosphine, etc. Suitable azo reagents include diethyl azodicarboxylate, diisopropyl azodicarboxylate, tetramethyl azodicarboxamide, azodicarbonyl dipiperidine, etc. Alternatively, compound (47) and trimethylsilyl azide may be reacted with a phosphorane reagent such as cyanomethylene trimethyl phosphorane, cyanomethylene tributyl phosphorane, etc. to give compound (48). The solvents to be used in the reaction include dichloromethane, chloroform, 1,2-dichloroethane, tetrahydrofuran, toluene, N,N-dimethylformamide, etc.; the reactions can be carried out at temperatures ranging from 0°C to the reflux temperature.

### (38) Step 38 (hydrolysis)

Compound (48) can be hydrolyzed using a suitable base, to give compound (49). The bases to be used in this step include lithium hydroxide, sodium hydroxide, potassium hydroxide, etc. The solvents to be used in this reaction include methanol, ethanol, isopropanol, tetrahydrofuran, water, mixtures thereof, etc; the reaction can be carried out at temperatures ranging from 0°C to the reflux temperature.
From compound (49), the compound (I) of the present invention can be synthesized by the same procedure as step 8 described in production method 1.

### Production Method 10

The compound (V) of the present invention, in which R³ is a hydrogen atom; R¹ is a hydrogen atom, a halogen atom, a C₁₋₄ alkyl group substituted by 1-3 halogen atoms, a C₁₋₁₀ alkyl group, a C₁₋₈ alkoxy group, a C₃₋₈ cycloalkyl group, a C₃₋₈ cycloalkoxy group, a C₄₋₁₄ cycloalkylalkyl group, or a phenyl group; and R⁸ is a C₁₋₁₀ alkyl group or a group having the structure represented by the following formula Ib or Ic, can be synthesized by the following method (scheme 10).

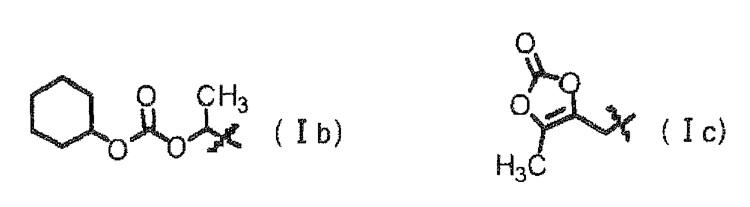

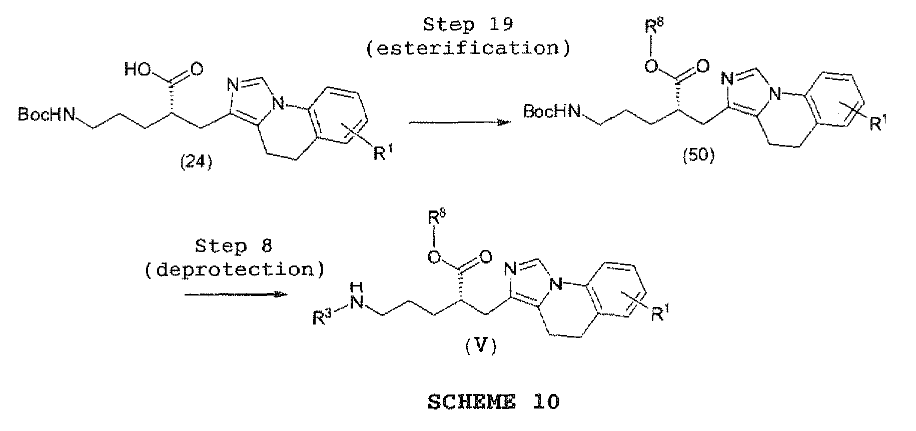

From compound (24), the compound (V) of the present invention can be synthesized by the same procedure as steps 19 and 8 described in production method 5.

### Production Method 11

The compound (V) of the present invention, in which R³ is the formula -CO₂R⁴ (where R⁴ is a C₁₋₆ alkyl group, the formula -CHR⁵OC(O)R⁶, or a substituent having the structure represented by the following formula Ia 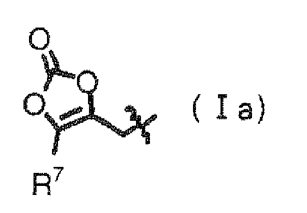 R⁵ is C₁₋₆ alkyl group; R⁶ is C₁₋₆ alkyl group, a C₃₋₈ cycloalkyl group or a phenyl group; R⁷ is a C₁₋₆ alkyl group or a phenyl group); R¹ is a hydrogen atom, a halogen atom, a C₁₋₄ alkyl group substituted by 1-3 halogen atoms, a C₁₋₁₀ alkyl group, a C₁₋₈ alkoxy group, a C₃₋₈ cycloalkyl group, a C₃₋₈ cycloalkoxy group, a C₄₋₁₄ cycloalkylalkyl group, or a phenyl group; and R⁸ is a hydrogen atom, can be synthesized by the following method (scheme 11).

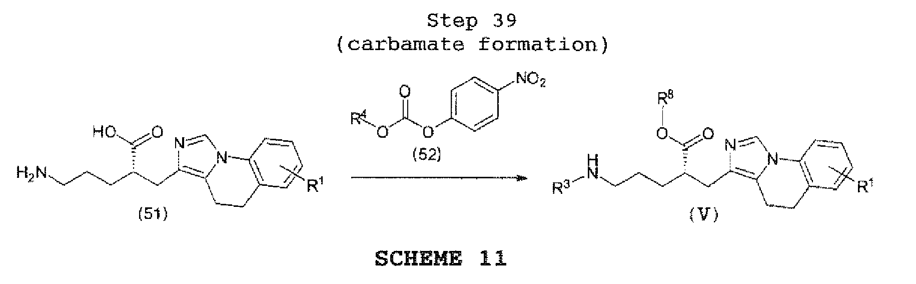

### (39) Step 39 (carbamate formation)

Compound (51) can be reacted with active carbonate (52), to give the compound (V) of the present invention. The solvents to be used in this reaction include N,N-dimethylformamide, N,N-dimethyl acetamide, N-methylpyrrolidone, tetrahydrofuran, toluene, dichloromethane, chloroform, water, etc.; the reaction can be carried out at temperatures ranging from 0°C to the reflux temperature.
On the following pages, the present invention is described even more specifically by showing Examples of the invention and Tests.

### EXAMPLES

The NH silica gel column chromatography as referred to in the following Examples means purification by column chromatographic separation using an NH2 type silica gel (Chromatorex NH2 type; FUJI SILYSIA CHEMICAL LTD.) The optical purities of compounds of the present invention were calculated based on measurements under the following conditions:
Column: CHIRALPACK AD-3, 4Φ x 250 mm, 3 µm (DAICEL CHEMICAL INDUSTRIES, LTD.)
temp.: 10°C, 24°C or 25°C
flow rate: 1 mL/min
det.: UV, 240 nm
sample conc: 1.0 mg/mL
inj. Vol.: 2 µL
Mobile phase: n-Hexane:IPA:TFA:DEA = 90:10:0.5:0.5; n-Hexane: IPA:TFA:DEA = 85:15:0.5:0.5; or n-Hexane:IPA:TFA:DEA = 80:20:0.5:0.5;

### Example 1

### Synthesis of 5-amino-2-(4,5-dihydroimidazo[1,5-a]quinolin-3-ylmethyl)pentanoic acid

### (1) Synthesis of ethyl 4,5-dihydroimidazo[1,5-a]quinoline-3-carboxylate

To a solution of 3,4-dihydroquinolin-2(1H)-one (50 g) in tetrahydrofuran (1 L), potassium tert-butoxide (46 g) was added under cooling with ice and the mixture was stirred for 30 minutes at the same temperature. Diethyl chlorophosphate (70 g) was added and after stirring the mixture for 30 minutes at the same temperature, ethyl isocyanoacetate (31 g) and potassium tert-butoxide (46 g) were added at -30°C and the mixture was stirred for an hour at room temperature. To the reaction mixture, an aqueous solution of 15% citric acid was added and extracting with ethyl acetate and washing with brine were performed. After drying over anhydrous sodium sulfate, the desiccant was filtered off and the solvent was removed in vacuo. The resulting residue was purified by silica gel column chromatography (eluent; n-hexane/ethyl acetate = 1:1 to 1:3) to give the titled compond (64.4 g) as a brown powder. MS(ESI/APCI Dual): 243 (M+H)
¹H NMR (300 MHz, CHLOROFORM-d) δ ppm 1.43 (t, J=7.2 Hz, 3H), 2.96 (t, J=7.2 Hz, 2 H), 3.35 (t, J=7.2 Hz, 2 H), 4.41 (q, J=7.2 Hz, 2 H), 7.20 - 7.30 (m, 1 H), 7.30 - 7.41 (m, 2 H), 7.42 - 7.52 (m, 1H), 8.03 (s, 1 H)

### (2) Synthesis of 4,5-dihydroimidazo[1,5-a]quinolin-3-ylmethanol

To a solution of ethyl 4,5-dihydroimidazo[1,5-a]quinoline-3-carboxylate (56.4 g) in tetrahydrofuran (583 ml), lithium aluminum hydride (10.6 g) was added under cooling with ice and the mixture was stirred for an hour at the same temperature. Ethyl acetate and water were added to the reaction system and the mixture was filtered; brine was added to the filtrate and the mixture was subjected to extraction with chloroform. After drying over anhydrous sodium sulfate, the desiccant was filtered off and the solvent was removed in vacuo to give the titled compound (50.1 g) as a brown oil.
¹H NMR (300 MHz, CHLOROFORM-d) δ ppm 2.84 - 3.07 (m, 4 H), 4.64 (s, 2 H), 7.14 - 7.25 (m, 1 H), 7.27 - 7.37 (m, 2 H), 7.40 - 7.45 (m, 1 H), 8.00 (s, 1 H)

### (3) Synthesis of 4,5-dihydroimidazo[1,5-a]quinoline-3-carbaldehyde

To a solution of 4,5-dihydroimidazo[1,5-a]quinolin-3-ylmethanol (50.1 g) in chloroform (777 ml), manganese dioxide (101 g) was added and the mixture was stirred for 15 hours at room temperature. The reaction system was filtered through Celite and the solvent was removed in vacuo. The resulting powder was washed with 1:1 n-hexane/ethyl acetate to give the titled compound (20 g) as a light brown powder.
MS(ESI/APCI Dual): 199 (M+H)
¹H NMR (300 MHz, CHLOROFORM-d) δ ppm 2.97 (t, J=7.2 Hz, 2 H), 3.35 (t, J=7.2 Hz, 2 H), 7.21 - 7.31 (m, 1 H), 7.31 - 7.42 (m, 2 H), 7.42 - 7.54 (m, 1 H), 8.07 (s, 1 H), 10.02 (s, 1 H)

### (4) Synthesis of tert-butyl (3E)-3-(4,5-dihydroimidazo[1,5-a]quinolin-3-ylmethyliden)-2-oxopiperidine-1-carboxylate

To a solution of tert-butyl 2-oxopiperidine-1-carboxylate (666 mg) in tetrahydrofuran (5.2 ml), lithium hexamethyldisilazane (3.3 ml, as 1 M solution in tetrahydrofuran) was added at -78 °C and the mixture was stirred for 30 minutes under cooling with ice. Subsequently, a suspension of 4,5-dihydroimidazo[1,5-a]quinoline-3-carbaldehyde (510 mg) in tetrahydrofuran was added at -78 °C and the mixture was stirred for an hour at the same temperature. A saturated aqueous solution of ammonium chloride was added to the reaction system and extraction was conducted with ethyl acetate. After drying over anhydrous sodium sulfate, the desiccant was filtered off and the solvent was removed in vacuo.
The resulting residue was dissolved in chloroform (5.2 ml) and after adding triethylamine (780 mg) and methanesulfonyl chloride (353 mg) under cooling with ice, the solution was stirred for an hour at room temperature. Water was added to the reaction system and extraction was conducted with chloroform. After drying over anhydrous sodium sulfate, the desiccant was filtered off and the solvent was removed in vacuo. The residue was purified by NH silica gel column chromatography (eluent: n-hexane/ethyl acetate/chloroform = 1:2:1 to 0:0:1) and the resulting powder was recrystalized with a mixture of chloroform and hexane in solution to give the titled compound (660 mg) as a pale yellow powder.
MS(ESI/APCI Dual): 380(M+H)
¹H NMR (300 MHz, CHLOROFORM-d) δ ppm 1.58 (s, 9 H), 1.86 - 2.02 (m, 2 H), 2.88 - 2.99 (m, 2 H), 3.02 - 3.13 (m, 2 H), 3.20 - 3.34 (m, 2 H), 3.70 - 3.80 (m, 2 H), 7.17 - 7.25 (m, 1 H), 7.29 - 7.40 (m, 2 H), 7.41 - 7.50 (m, 1 H), 7.70 (t, J=1.9 Hz, 1 H), 8.08 (s, 1 H)

### (5) Synthesis of tert-butyl 3-(4,5-dihydroimidazo[1,5-a]quinolin-3-ylmethyl)-2-oxopiperidine-1-carboxylate

To a solution of tert-butyl (3E)-3-(4,5-dihydroimidazo[1,5-a]quinolin-3-ylmethyliden)-2-oxopiperidine-1-carboxylate (300 mg) in a methanol-tetrahydrofuran solution (1:1, 15.8 ml), 10% palladium-activated carbon (60 mg) was added and the mixture was stirred under hydrogen purge for an hour at 60°C. The reaction system was filtered through Celite and the solvent was removed in vacuo. The resulting residue was purified by NH silica gel column chromatography (eluent: n-hexane/ethyl acetate = 1:1) to give the titled compound (301 mg) as a colorless oil.
MS(ESI/APCI Dual): 382(M+H)
¹H NMR (300 MHz, CHLOROFORM-d) δ ppm 1.52 (s, 9 H), 1.55 - 1.69 (m, 1 H), 1.69 - 1.95 (m, 2 H), 1.97 - 2.14 (m, 1 H), 2.67 (dd, J=14.4, 8.3 Hz, 1 H), 2.79 - 3.01 (m, 5 H), 3.21 (dd, J=14.4, 4.7 Hz, 1 H), 3.49 - 3.65 (m, 1 H), 3.72 - 3.91 (m, 1 H), 7.11 - 7.22 (m, 1 H), 7.23 - 7.34 (m, 2 H), 7.35 - 7.45 (m, 1 H), 7.95 (s, 1 H)

### (6) Synthesis of 5-amino-2-(4,5-dihydroimidazo[1,5-a]quinolin-3-ylmethyl)pentanoic acid

To a solution of tert-butyl 3-(4,5-dihydroimidazo[1,5-a]quinolin-3-ylmethyl)-2-oxopiperidine-1-carboxylate (301 mg) in tetrahydrofuran (4.0 ml), an aqueous solution of 0.9 M lithium hydroxide (2.6 ml) was added and the mixture was stirred for 15 hours at room temperature. An aqueous solution of 6 M hydrochloric acid (4.0 ml) was added and the mixture was stirred for 5 hours at room temperature. The reaction system was purified by cation exchange chromatography (DOWEX 50WX8-100; ammonia/water = 0:1 to 3:97) to give the titled compound (compound 1, 110 mg) as a colorless powder.
MS(ESI/APCI Dual): 300(M+H)
¹H NMR (600 MHz, DEUTERIUM OXIDE) δ ppm 1.48 - 1.62 (m, 2 H), 1.62 - 1.72 (m, 2 H), 2.55 - 2.64 (m, 2 H), 2.74 - 2.90 (m, 5 H), 2.92 - 3.04 (m, 2 H), 7.22 - 7.27 (m, 1 H), 7.30 - 7.40 (m, 2 H), 7.51 (d, J=7.8 Hz, 1 H), 8.11 (s, 1 H)

### Example 2

### Synthesis of (2S)-5-amino-2-(4,5-dihydroimidazo[1,5-a]quinolin-3-ylmethyl)pentanoic acid

### (1) Synthesis of 5-[(tert-butoxycarbonyl)amino]-2-(4,5-dihydroimidazo[1,5-a]quinolin-3-ylmethyl)pentanoic acid

To a solution of tert-butyl 3-(4,5-dihydroimidazo[1,5-a]quinolin-3-ylmethyl)-2-oxopiperidine-1-carboxylate (11.8 g) in tetrahydrofuran (155 ml), an aqueous solution of 0.9 M lithium hydroxide (103 ml) was added and the mixture was stirred for 15 hours at room temperature. Water was added to the reaction system and the aqueous layer was washed with diethyl ether. The aqueous layer was neutralized with an aquoeus solution of 15% citric acid and extraction was conducted with chloroform. After drying over anhydrous sodium sulfate, the desiccant was filtered off and the solvent was removed in vacuo. The residue was purified by silica gel column chromatography (eluent: chloroform/methanol = 10:1) to give the titled compound (11.7 g) as a colorless powder.
MS(ESI/APCI Dual): 400(M+H)
¹H NMR (300 MHz, CHLOROFORM-d) δ ppm 1.41 (s, 9 H), 1.46 - 1.68 (m, 3 H), 1.68 - 1.87 (m, 1 H), 2.68 - 3.00 (m, 7 H), 3.01 - 3.21 (m, 2 H), 4.72 (br. s., 1 H), 7.13 - 7.24 (m, 1 H), 7.24 - 7.35 (m, 2 H), 7.35 - 7.47 (m, 1 H), 8.11 (s, 1 H)

### (2) Synthesis of (1R,2S)-1-phenyl-2-(pyrrolidin-1-yl)propyl (2S)-5-[(tert-butoxycarbonyl)amino]-2-(4,5-dihydroimidazo[l,5-a]quinolin-3-ylmethyl)pentanoate and (1R,2S)-1-phenyl-2-(pyrrolidin-1-yl)propyl (2R)-5-[(tert-butoxycarbonyl)amino]-2-(4,5-dihydroimidazo[1,5-a]quinolin-3-ylmethyl)pentanoate

To a solution of 5-[(tert-butoxycarbonyl)amino]-2-(4,5-dihydroimidazo[1,5-a]quinolin-3-ylmethyl)pentanoic acid (2.00 g) in chloroform (50.1 ml), (1R,2S)-1-phenyl-2-(pyrrolidin-1-yl)propan-1-ol (1.54 g), 4-dimethylaminopyridine (61 mg) and N-[3-(dimethylamino)pxopyl]-N'-ethylcarbodiimide hydrochloride (1.44 g) were added and the mixture was stirred for 15 hours at room temperature. A saturated aqueous solution of sodium hydrogencarbonate was added to the reaction system and extraction was conducted with chloroform. After drying over anhydrous sodium sulfate, the desiccant was filtered off and the solvent was removed in vacuo. The residue was purified by silica gel column chromatography (eluent: ethyl acetate/methanol = 10:1), then by NH silica gel column chromatography (eluent: n-hexane/2-propanol = 40:1) to give the two titled compounds as a pale yellow oil, i.e., the low-polarity compound (1R,2S)-1-phenyl-2-(pyrrolidin-1-yl)propyl (2S)-5-[(tert-butoxycarbonyl)amino]-2-(4,5-dihydroimidazo[1,5-a]quinolin-3-ylmethyl)pentanoate (1.17 g) and the high-polarity compound (1R,2S)-1-phenyl-2-(pyrrolidin-1-yl)propyl (2R)-5-[(tert-butoxycarbonyl)amino]-2-(4,5-dihydroimidazo[1,5-a]quinolin-3-ylmethyl)pentanoate (590 mg).

### (1R,2S)-1-phenyl-2-(pyrrolidin-1-yl)propyl (2S)-5-[(tert-butoxycarbonyl)amino]-2-(4,5-dihydroimidazo[1,5-a]quinolin-3-ylmethyl)pentanoate

MS(ESI/APCI Dual): 587(M+H)
¹H NMR (300 MHz, CHLOROFORM-d) δ ppm 0.94 (d, J=6.7 Hz, 3 H), 1.31 - 1.56 (m, 10 H), 1.58 - 1.85 (m, 7 H), 1.93 - 2.12 (m, 1 H), 2.34 - 2.47 (m, 1 H), 2.47 - 2.72 (m, 8 H), 2.77 - 2.96 (m, 1 H), 3.08 - 3.31 (m, 3 H), 5.31 (br. s., 1 H), 6.04 - 6.17 (m, 1 H), 6.66 - 6.80 (m, 1 H), 6.80 - 6.99 (m, 4 H), 7.08 - 7.18 (m, 2 H), 7.24 - 7.34 (m, 1 H), 7.35 - 7.46 (m, 1 H), 7.98 (s, 1 H)

### (1R,2S)-1-phenyl-2-(pyrrolidin-1-yl)propyl (2R)-5-[(tert-butoxycarbonyl)amino]-2-(4,5-dihydroimidazo[1,5-a]quinolin-3-ylmethyl)pentanoate

MS(ESI/APCI Dual): 587(M+H)
¹H NMR (300 MHz, CHLOROFORM-d) δ ppm 1.00 (d, J=6.5 Hz, 3 H), 1.43 (s, 9 H), 1.45 - 1.92 (m, 9 H), 2.43 - 2.69 (m, 5 H), 2.69 - 2.88 (m, 4 H), 2.88 - 3.20 (m, 4 H), 5.12 (br. s., 1 H), 6.02 - 6.10 (m, 1 H), 7.08 - 7.35 (m, 8 H), 7.35 - 7.43 (m, 1 H), 7.94 (s, 1 H)

### (3) Synthesis of (2S)-5-[(tert-butoxycarbonyl)aminol-2-(4,5-dihydroimidazo[1,5-a]quinolin-3-ylmethyl)pentanoic acid

To a solution of (1R,2S)-1-phenyl-2-(pyrrolidin-1-yl)propyl (2S)-5-[(tert-butoxycarbonyl)amino]-2-(4,5-dihydroimidazo[1,5-a]quinolin-3-ylmethyl)pentanoate (2.62 g) in methanol (44.7 ml), 10% palladium-activated carbon (524 mg) was added and the mixture was stirred under hydrogen purge for 10 hours at 60°C. The reaction mixture was filtered through Celite and after washing with methanol, the solvent was removed in vacuo. To the resulting residue, a saturated aqueous solution of sodium hydrogencarbonate was added and the aqueous layer was washed with ethyl acetate. The aqueous layer was neutralized with an aqueous solution of 15% citric acid and extraction was conducted with chloroform. After drying over anhydrous sodium sulfate, the desiccant was filtered off and the solvent was removed in vacuo. The residue was purified by silica gel column chromatography (eluent: chloroform/methanol = 10:1) to give the titled compound (1.50 g) as a colorless powder. ¹H NMR (300 MHz, CHLOROFORM-d) δ ppm 1.41 (s, 9 H), 1.46 - 1.68 (m, 3 H), 1.68 - 1.87 (m, 1 H), 2.68 - 3.00 (m, 7 H), 3.01 - 3.21 (m, 2 H), 4.53 - 4.70 (m, 1 H), 7.13 - 7.28 (m, 1 H), 7.28 - 7.38 (m, 2 H), 7.38 - 7.47 (m, 1 H), 8.15 (s, 1 H)

### (4) Synthesis of (2S)-5-amino-2-(4,5-dihydroimidazo[1,5-alquinolin-3-ylmethyl)pentanoic acid

To a solution of (2S)-5-[(tert-butoxycarbonyl)amino]-2-(4,5-dihydroimidazo[1,5-a]quinolin-3-ylmethyl)pentanoic acid (700 mg) in tetrahydrofuran (4.4 ml), an aqueous solution of 6 M hydrochloric acid (8.8 ml) was added and the mixture was stirred for 3 hours at room temperature. The reaction system was concentrated in vacuo and the resulting residue was purified by cation exchange chromatography (DOWEX 50WX8-200; ammonia/water = 0:1 to 3:97) to give the titled compound (compound 2, 360 mg) as a pale brown powder.
MS(ESI/APCI Dual): 300(M+H)
¹H NMR (600 MHz, DEUTERIUM OXIDE) δ ppm 1.48 - 1.62 (m, 2 H), 1.62 - 1.72 (m, 2 H), 2.51 - 2.64 (m, 2 H), 2.74 - 2.90 (m, 5 H), 2.92 - 3.04 (m, 2 H), 7.18 - 7.27 (m, 1 H), 7.30 - 7.40 (m, 2 H), 7.46 (d, J=7.8 Hz, 1 H), 8.11 (s, 1 H)
[α]_{D}²⁰ = -10.3 (c = 1.0, H₂O)
Optical purity: 99.34% ee
temp.: 24°C.
Mobile phase: n-Hexane:IPA:TFA:DEA=90:10:0.5:0.5
r.t. : 26.56 min

### Example 3

### Synthesis of (2R)-5-amino-2-(4,5-dihydroimidazo[1,5-a]quinolin-3-ylmethyl)pentanoic acid

### (i) Synthesis (2R)-5-[(tert-butoxycarbonyl)amino]-2-(4,5-dihydroimidazo[1,5-alquinolin-3-ylmethyl)pentanoic acid

To a solution of (1R, 2S)-1-phenyl-2-(pyrrolidin-1-yl)propyl (2R)-5-[(tert-butoxycarbonyl)amino]-2-(4,5-dihydroimidazo[1,5-a]quinolin-3-ylmethyl)pentanoate (590 mg) in methanol (20.1 ml), an aqueous solution of 33% sodium hydroxide (3.4 ml) was added and the mixture was stirred for 39 hours at room temperature. The reaction system was neutralized with an aqueous solution of 2 M hydrochloric acid and the solvent was removed in vacuo. To the resulting residue, a saturated aqueous solution of sodium hydrogencarbonate was added and the aqueous layer was washed with diethyl ether. The aqueous layer was neutralized with an aqueous solution of 15% citric acid and extraction was conducted with chloroform. After drying over anhydrous sodium sulfate, the desiccant was filtered off and the solvent was removed in vacuo to give the titled compound (340 mg) as a pale yellow powder.
¹H NMR (300 MHz, CHLOROFORM-d) δ ppm 1.41 (s, 9 H), 1.46 - 1.68 (m, 3 H), 1.68 - 1.87 (m, 1 H), 2.68 - 3.00 (m, 7 H), 3.01 - 3.21 (m, 2 H), 4.62 - 4.80 (m, 1 H), 7.13 - 7.28 (m, 1 H), 7.28 - 7.38 (m, 2 H), 7.38 - 7.47 (m, 1 H), 8.26 (s, 1 H)

### (2) Synthesis of (2R)-5-amino-2-(4,5-dihydroimidazo[1,5-a]quinolin-3-ylmethyl)pentanoic acid

Using (2R)-5-[(tert-butoxycarbonyl)amino]-2-(4,5-dihydroimidazo[1,5-a]quinolin-3-ylmethyl)pentanoic acid (300 mg), reaction and purification were performed by the same procedures as in Example 2(4) to give the titled compound (compound 3, 90 mg) as a pale brown powder.
MS(ESI/APCI Dual): 300(M+H)
¹H NMR (600 MHz, DEUTERIUM OXIDE) δ ppm 1.48 - 1.62 (m, 2 H), 1.62 - 1.72 (m, 2 H), 2.51 - 2.64 (m, 2 H), 2.74 - 2.90 (m, 5 H), 2.92 - 3.04 (m, 2 H), 7.21 - 7.27 (m, 1 H), 7.30 - 7.40 (m, 2 H), 7.51 (d, J=7.8 Hz, 1 H), 8.13 (s, 1 H)
[α]_{D}²⁰ = +8.2 (c = 1.0, H₂O)

### Example 4

### Synthesis of 5-amino-2-(5,6-dihydro-4H-imidazo[1,5-a][1]benzazepin-3-ylmethyl)pentanoic acid

### (1) Synthesis of N-hydroxy-3,4-dihydronaphthalen-1(2H)-imine

To a solution of 3,4-dihydronaphthalen-1(2H)-one (6.00 g) in a mixture of methanol (30 ml) and pyridine (30 ml), hydroxylamine hydrochloride (5.99 g) was added at room temperature and the mixture was stirred for 2 hours with heating under reflux. The reaction system was concentrated in vacuo and, after being dissolved in chloroform, it was washed with a saturated aqueous solution of ammonium chloride and brine. After drying the organic layer with sodium carbonate, the desiccant was filtered off and the solvent was removed in vacuo. After azeotropic distillation with toluene, to the crystal rusulting from evaporation in vacuuo for removing solvent, n-hexane was added and the mixture was stirred and filtered to recover the titled compound (5.73 g) as a pale brown powder.
MS(ESI/APCI Dual): 162(M+H)
¹H NMR (300 MHz, CHLOROFORM-d) δ ppm 1.81 - 1.97 (m, 2 H), 2.70 - 2.90 (m, 4H), 7.09 - 7.35 (m, 3 H), 7.82 - 7.96 (m, 1 H)

### (2) Synthesis of 1,3,4,5-tetrahydro-2H-1-benzazepin-2-one

To N-hydroxy-3,4-dihydronaphthalen-1(2H)-imine (5.73g), polyphosphoric acid (57 g) was added and the mixture was stirred for an hour udner heating at 120°C. Water (50 ml) was added to the reaction mixture under cooling with ice. The precipipcating crystal was recovered by filtration and, after being washed with water, it was dried in vacuo to give the titled compound (3.95 g) as a brown powder.
MS(ESI/APCI Dual): 162(M+H)
¹H NMR (300 MHz, CHLOROFORM-d) δppm 2.17 - 2.31 (m, 2 H), 2.32 - 2.43 (m, 2 H), 2.81 (t, J=7.1 Hz, 2 H), 6.96 (d, J=7.6 Hz, 1 H), 7.11 - 7.36 (m, 3 H), 7.48 (br. s., 1 H)

### (3) Synthesis of ethyl 5,6-dihydro-4H-imidazo[1,5-a][1]benzazepin-3-carboxylate

Using 1,3,4,5-tetrahydro-2H-1-benzazepin-2-one (4.18 g), reaction and purificaton were performed by the same procedures as in Example 1(1) to give the titled compound (1.80 g) as a pale yellow powder.
MS(ESI/APCI Dual): 257(M+H)
¹H NMR (300 MHz, CHLOROFORM-d) δppm 1.43 (t, J=7.1 Hz, 3 H), 2.22 (quin, J=7.1 Hz, 2 H), 2.59 (t, J=7.1 Hz, 2 H), 3.06 (t, J=7.1 Hz, 2 H), 4.41 (q, J=7.1 Hz, 2 H), 7.29 - 7.45 (m, 4 H), 7.69 (s, 1 H)

### (4) Synthesis of 5,6-dihydro-4H-imidazo[1,5-a] [1]benzazepin-3-ylmethanol

Using ethyl 5,6-dihydro-4H-imidazo[1,5-a][I]benzazepin-3-carboxylate (1.80 g), reaction and purificaton were performed by the same procedures as in Example 1(2) to give the titled compound (1.48 g) as an orange powder.
MS(ESI/APCI Dual): 215(M+H)
¹H NMR (300 MHz, CHLOROFORM-d) δppm 2.06 - 2.22 (m, 2 H), 2.56 - 2.70 (m, 4 H), 4.66 (s, 2 H), 7.28 - 7.42 (m, 4 H), 7.71 (s, 1 H)

### (5) Synthesis of 5,6-dihydro-4H-imidazo[1,5-a] [1] benzazepin-3-carbaldehyde

Using 5,6-dihydro-4H-imidazo[1,5-a][1]benzazepin-3-ylmethanol (1.48 g), reaction and purificaton were performed by the same procedures as in Example 1(3) to give the titled compound (730 mg) as a pale orange oil.
MS(ESI/APCI Dual): 213(M+H)
¹H NMR (300 MHz, CHLOROFORM-d) δppm 2.19 - 2.33 (m, 2 H), 2.53 - 2.69 (m, 2 H), 2.96 - 3.11 (m, 2 H), 7.30 - 7.51 (m, 4 H), 7.76 (s, 1 H), 10.04 (s, 1 H)

### (6) Synthesis of tert-butyl (3E)-3-(5,6-dihydro-4H-imidazo[1,5-a][1]benzazepin-3-ylmethyliden)-2-oxopiperidine-1-carboxylate

To tert-butyl 2-oxopiperidine-1-carboxylate (891 mg) in tetrahydrofuran (6.9 ml), lithium hexamethyldisilazane (4.47 ml as 1 M solution in tetrahydrofuran) was added and the mixture was stirred for 30 minutes at the same temperature. At -78°C, 5,6-dihydro-4H-imidazo[1,5-a][1]benzazepin-3-carbaldehyde (730 mg) in tetrahydrofuran was added and the mixture was stirred for an hour at the same temperature. To the reaction system, a saturated aqueous solution of ammonium chloride was added and extraction was conducted with ethyl acetate. After drying over anhydrous magnesium sulfate, the desiccant was filtered off and the solvent was removed in vacuo. The resulting residue was dissolved in chloroform (6.7 ml) and triethylamine (1.40 ml) and methanesulfonyl chloride (0.3 ml) were added under cooling with ice, and the mixture was stirred for 50 minutes at room temperautre. To the reaction system, water was added and extraction was conducted with ethyl acetate. After drying over anhydrous magnesium sulfate, the desiccant was filtered off and the solvent was removed in vacuo. The residue was purified by silica gel collumn chromatography (eluent: n-hexane/ethyl acetate = 1:1) to give the titled compound (750 mg) as a pale yellow oil. MS(ESI/APCI Dual): 394(M+H)
¹H NMR (300 MHz, CHLOROFORM-d) δppm 1.57 (s, 9 H), 1.89 - 2.00 (m, 2 H), 2.09 - 2.22 (m, 2 H), 2.55 - 2.64 (m, 2 H), 2.71 - 2.81 (m, 2 H), 3.25 - 3.35 (m, 2 H), 3.73 - 3.81 (m, 2 H), 7.29 - 7.43 (m, 4 H), 7.71 - 7.75 (m, 1 H), 7.78 (s, 1 H)

### (7) Synthesis of tert-butyl 3-(5,6-dihydro-4H-imidazo[1,5-a][1]benzazepin-3-ylmethyl)-2-oxopiperidine-1-carboxylate

To tert-butyl (3E)-3-(5,6-dihydro-4H-imidazo[1,5-a][1]benzazepin-3-ylmethyliden)-2-oxopiperidine-1-carboxylate (610 mg) in tetrahydrofuran (30 ml), 10% palladium-activated carbon (300 mg) was added and the mixture was stirred under hydrogen purge for an hour at 60°C. The reaction mixture was filtered and the solvent was removed in vacuo. The resulting residue was purified by silica gel column chromatography (elutent: chloroform/methanol = 20:1) to give the titled compound (560 mg) as a colorless oil.
MS(ESI/APCI Dual): 396 (M+H)
¹H NMR (300 MHz, CHLOROFORM-d) δppm 1.53 (s, 9 H), 1.77 - 1.91 (m, 2 H), 2.00 - 2.18 (m, 2 H), 2.47 - 2.70 (m, 6 H), 2.82 - 2.95 (m, 1H), 3.26 (dd, J=14.5, 4.7 Hz, 1 H), 3.51 - 3.70 (m, 2 H), 3.78 - 3.88 (m, 1 H), 7.27 - 7.40 (m, 4 H), 7.64 (s, 1 H)

### (8) Synthesis of 5-[(tert-butoxycarbonyl)amino]-2-(5,6-dihydro-4H-imidazo[1,5-a][1]benzazepin-3-ylmethyl)pentanoic acid

Using tert-butyl 3-(5,6-dihydro-4H-imidazo[1,5-a][1]benzazepin-3-ylmethyl)-2-oxopiperidine-1-carboxylate (560 mg), reaction and purification were performed by the same procedures as in Example 2(1) to give the titled compound (330 mg) as a colorless oil.
MS(ESI/APCI Dual): 414(M+H)
¹H NMR (300 MHz, CHLOROPORM-d) δppm 1.34 - 1.68 (m, 13 H), 1.71 - 1.89 (m, 1 H), 2.03 - 2.19 (m, 2 H), 2.49 - 2.65 (m, 4 H), 2.71 - 2.94 (m, 3 H), 3.05 - 3.20 (m, 2 H), 4.56 - 4.73 (m, 1 H), 7.28 - 7.43 (m, 4 H), 7.77 (s, 1 H)

### (9) Synthesis of 5-amino-2-(5,6-dihydro-4H-imidazo[1,5-a][1]benzazepin-3-ylmethyl)pentanoic acid

Using 5-[(tert-butoxycarbonyl)amino]-2-(5,6-dihydro-4H-imidazo[1,5-a][1]benzazepin-3-ylmethyl)pentanoic acid (330 mg), reaction and purification were performed by the same procedures as in Example 2(4) to give the titled compound (coompound 4, 81 mg) as a pale brown amorphous mass. MS(ESI/APCI Dual): 314(M+H)
¹H NMR (600 MHz, DEUTERIUM OXIDE) δ ppm 1.47 - 1.73 (m, 4 H), 2.01 - 2.17 (m, 2 H), 2.47 - 2.68 (m, 6 H), 2.83 (dd, J=14.0, 8.0 Hz, 1 H), 2.93 - 3.06 (m, 2 H), 7.37 - 7.49 (m, 4 H), 7.87 (s, 1 H)

### Example 5

### Synthesis of 5-amino-2-(4H-imidazo[5,1-c][1,4]benzoxazin-3-ylmethyl)pentanoic acid

### (1) Synthesis of ethyl 4H-imidazo[5,1-c][1,4]benzoxazin-3-carboxylate

Using 2H-1,4-benzoxazin-3(4H)-one (6.00 g), reaction and purification were perormed by the same procedures as in Example 1(1) to give the titled comound (8.29 g) as a brown powder.
MS(ESI/APCI Dual): 245(M+H)
¹H NMR (300 MHz, CHLOROFORM-d) δppm 1.43 (t, J=7.1 Hz, 3 H), 4.41 (q, J=7.1 Hz, 2 H), 5.53 (s, 2 H), 7.06 - 7.17 (m, 2 H), 7.19 - 7.26 (m, 1 H), 7.48 (dd, J=8.1, 1.4 Hz, 1H), 8.01 (s, 1 H)

### (2) Synthesis of 4H-imidazo[5,1-c][1,4]benzoxazin-3-ylmethanol

Using ethyl 4H-imidazo[5,1-c][1,4]benzoxazin-3 carboxylate (3.00 g), reaction and purification were performed by the same procedures as in Example 1(2) to give the titled compound (1.23 g) as a pale brown powder.
MS(ESI/APCI Dual): 203(M+H)
¹H NMR (300 MHz, CHLOROPORM-d) δppm 4.68 (s, 2 H), 5.25 (s, 2 H), 7.03 - 7.23 (m, 3 H), 7.45 (dd, J=7.9, 1.6 Hz, 1 H), 7.97 (s, 1 H)

### (3) Synthesis of 4H-imidazo[5,1-c][1,4]benzoxazine-3-carbaldehyde

Using 4H-imidazo[5,1-c][1,4]benzoxazin-3-ylmethanol (1.23 g), reaction and purification were performed by the same procedures as in Example 1(3) to give the titled compound (1.03 g) as a brown powder.
¹H NMR (300 MHz, CHLOROFORM-d) δppm 5.54 (s, 2 H), 7.07 - 7.18 (m, 2 H), 7.22 - 7.32 (m, 1 H), 7.49 (dd, J=7.9, 1.6 Hz, 1 H), 8.04 (s, 1 H), 9.99 (s, 1 H)

### (4) Synthesis of tert-butyl (3E)-3-(4H-imidazo[5,1-c][1,4]benzoxazin-3-ylmethyliden)-2-oxopiperadine-1-carboxylate

Using 4H-imidazo[5,1-c][1,4]benzoxazine-3-carbaldehyde (1.03 g), reaction and purification were performed by the same procedures as in Example 1(4) to give the titled compound (1.90 g) as a brown powder.
MS(ESI/APCI Dual): 382(M+H)
¹H NMR (300 MHz, CHLOROPORM-d) δppm 1.57 (s, 9 H), 1.89 - 2.00 (m, 2 H), 3.20 - 3.30 (m, 2 H), 3.73 - 3.80 (m, 2 H), 5.30 (s, 2 H), 7.05 - 7.23 (m, 3 H), 7.44 - 7.50 (m, 1H), 7.55 - 7.60 (m, 1 H), 8.05 (s, 1 H)

### (5) Synthesis of tert-butyl 3-(4H-imidazo[5,1-c][1,4]benzoxazin-3-ylmethyl)-2-oxopiperidine-1-carboxylate

Using tert-butyl (3E)-3-(4H-imidazo[5,1-c][1,4]benzoxazin-3-ylmethyliden)-2-oxopiperidine-1-carboxylate (590 mg), reaction and purification were performed by the same procedures as in Example 1(5) to give the titled compound (610 mg) as a pale brown oil.
MS(ESI/APCI Dual): 384(M+H)
¹H NMR (300 MHz, CHLOROFORM-d) δppm 1.39 - 1.76 (m, 10 H), 1.77 - 1.93 (m, 2 H), 2.03 - 2.20 (m, 1 H), 2.66 - 2.93 (m, 2 H), 3.04 - 3.17 (m, 1 H), 3.49 - 3.65 (m, 1 H), 3.73 - 3.87 (m, 1 H), 5.18 - 5.27 (m, 2 H), 6.99 - 7.22 (m, 3 H), 7.38 - 7.48 (m, 1 H), 7.89 - 7.96 (m, 1 H)

### (6) Synthesis of 5-amino-2-(4H-imidazo[5,1-c][1,4]benzoxazin-3-ylmethyl)pentanoic acid

Using tert-butyl 3-(4H-imidazo[5,1-c][1,4]benzoxazin-3-ylmethyl)-2-oxopiperidine-1-carboxylate (610 mg), reaction and purification were performed by the same procedures as in Example 1(6) to give the titled compound (compound 5, 280 mg) as a pale brown powder.
MS(ESI/APCI Dual): 302(M+H)
¹H NMR (300 MHz, DEUTERIUM OXIDE) δ ppm 1.49 - 1.75 (m, 4 H), 2.48 - 2.69 (m, 2 H), 2.72 - 2.84 (m, 1 H), 2.93 - 3.06 (m, 2 H), 5.07 - 5.24 (m, 2 H), 7.09 - 7.31 (m, 3 H), 7.62 (d, J=9.3 Hz, 1 H), 8.17 (s, 1 H)

### Example 6

### Synthesis of 5-amino-2-(4,5-dihydroimidazo[1,5-a][1,7]naphthyridin-3-ylmethyl}pantanoio acid

### (1) Ethyl 4,5-dihydroimidazo[1,5-a][1,7]naphthyridin-3-carboxylate

Using 3,4-dihydro-1,7-naphthyridin-2(1H)-one (1.48 g), reaction and purification were performed by the same procedures as in Example 1(1) to give the titled compound (870 mg) as a pale yellow powder.
MS(ESI/APCI Dual): 244(M+H)

### (2) Synthesis of 4,5-dihydroimidazo[1,5-a][1,7]naphthyridin-3-ylmethanol

Using ethyl 4,5-dihydroimidazo[1,5-a][1,7]naphthyridin-3-carboxylate (870 mg), reaction was performed by the same procedure as in Example 1(2) to give the titled compound (820 mg) as a brown oil.
¹H NMR (300 MHz, CHLOROFORM-d) δ ppm 2.93 - 3.08 (m, 4 H), 4.66 (s, 2 H), 7.26 - 7.30 (m, 1 H), 8.12 (s, 1 H), 8.45 (d, J=5.0 Hz, 1 H), 8.78 (s, 1 H)

### (3) Synthesis of 4,5-dihydroimidazo[1,5-a][1,7]naphthyridin-3-carbaldehyde

Using 4,5-dihydroimidazo[1,5-a][1,7]naphthyridin-3-ylmethanol (820 mg), reaction and purification were performed by the same procedures as in Example 1(3) to give the titled compound (190 mg) as an orange powder.
MS(ESI/APCI Dual): 200(M+H)

### (4) Synthesis of tert-butyl (3E)-3-(4,5-dihydroimidazo[1,5-a][1,7]naphthyridin-3-ylmethyliden)-2-oxopiperidine-1-carboxylate

Using 4,5-dihydroimidazo[1,5-a][1,7]naphthyridin-3-carbaldehyde (190 mg), reaction and purification were performed by the same procedures as in Example 1(4) to give the titled compound (250 mg) as a yellow powder. MS(ESI/APCI Dual): 381(M+H)

### (5) Synthesis of tert-butyl 3-(4,5-dihydroimidazo[1,5-a][1,7]naphthyridin-3-ylmethyliden)-2-oxopiperidine-1-carboxylate

Using tert-butyl (3E)-3-(4,5-dihydroimidazo [1,5-a][1,7]naphthyridin-3-ylmethyl)-2-oxopiperidine-1-carboxylate (60 mg), reaction and purification were performed by the same procedures as in Example 1(5) to give the titled compound (53 mg) as a colorless oil.
MS(ESI/APCI Dual): 383(M+H)

### (6) Synthesis of 5-amino-2-(4,5-dihydroimidazo[1,5-a][1,7]naphthyridin-3-ylmethyl)pentanoic acid

Using tert-butyl 3-(4,5-dihydroimidazo[1,5-a][1,7]naphthyridin-3-ylmethyl)-2-oxopiperidine-1-carboxylate (53 mg), reaction and purification were performed by the same procedures as in Example 1(6) to give the titled compound (compound 6, 17 mg) as a colorless oil.
MS(ESI/APCI Dual): 301(M+H)
¹H NMR (600 MHz, DEUTERIUM OXIDE) δ ppm 1.48 - 1.70 (m, 4 H), 2.50 - 2.60 (m, 2 H), 2.71 - 2.92 (m, 5 H), 2.92 - 3.04 (m, 2 H), 7.36 (d, J=5.0 Hz, 1 H), 8.09 (s, 1 H), 8.22 (d, J=5.0 Hz, 1 H), 8.55 (s, 1 H)

### Example 7

### Synthesis of 5-amino-2-[(9-methyl-4,5-dihydroimidazo[1,5-a]quinolin-3-yl)methyl]pentanoic acid dihydrochloride

### (1) Synthesis of ethyl 9-methyl-4,5-dihydroimidazo[1,5-a]quinoline-3-carboxylate

Using 8-methyl-3,4-dihydroquinolin-2(1H)-one (1.61 g), reaction and purification were performed by the same procedures as in Example 1(1) to give the titled compound (1.63 g) as a pale yellow powder.
MS(ESI/APCI Dual): 257(M+H)

### (2) Synthesis of (9-methyl-4,5-dihydroimidazo[1,5-a]quinolin-3-yl)methanol

Using ethyl 9-methyl-4,5-dihydroimidazo[1,5-a]quinoline-3-carboxylate (1.63 g), reaction was performed by the same procedure as in Exmple 1(2) to give the titled compound (1.01 g) as a brown oil.
¹H NMR (300 MHz, CHLOROFORM-d) δ ppm 2.58 (s, 3 H), 2.82 - 2.94 (m, 4 H), 4.67 (s, 2 H), 7.06 - 7.23 (m, 3 H), 8.05 (s, 1 H)

### (3) Synthesis of 9-methyl-4,5-dihydroimidazo[1,5-a]quinoline-3-carbaldehyde

Using (9-methyl-4,5-dihydroimidazo[1,5-a]quinolin-3-yl)methanol (1.01 g), reaction and purification were performed by the same procedures as in Example 1(3) to give the titled compound (630 mg) as a pale yellow powder.
MS(ESI): 213(M+H)

### (4) Synthesis of tert-butyl (3E)-3-[(9-methyl-4,5-dihydroimidazo[1,5-a]quinolin-3-yl)methyliden]-2-oxopiperidine-1-carboxylate

Using 9-methyl-4,5-dihydroimidazo[1,5-a]quinoline-3-carbaldehyde (630 mg), reaction and purification were performed by the same procedures as in Example 1(4) to give the titled compound (500 mg) as a pale yellow powder. MS(ESI/APCI Dual): 394(M+H)

### (5) Synthesis of tert-butyl (3-[(9-methyl-4,5-dihydroimidazo[1,5-a]quinolin-3-yl)methyl]-2-oxopiperidine-1-carboxylate

Using tert-butyl (3E)-3-[(9-methyl-4,5-dihydroimidazo[1,5-a]quinolin-3-yl)methyliden]-2-oxopiperidine-1-carboxylate (500 mg), reaction and purification were performed by the same procedures as in Example 1(5) to give the titled compound (495 mg) as a colorless oil.
MS(ESI/APCI Dual): 396(M+H)

### (6) Synthesis of 5-amino-2-[(9-methyl-4,5-dihydroimidazol[,5-a]quinolin-3-yl)methyl]pentanoic acid dihydrochloride

Using tert-butyl (3-[(9-methyl-4,5-dihydroimidazo[1,5-a]quinolin-3-yl)methyl]-2-oxopiperidine-1-carboxylate (495 mg), reaction and purification were performed by the same procedures as in Example 1(6) to give 5-amino-2-[(9-methyl-4,5-dihydroimidazo[1,5-a]quinolin-3-yl)methyl]pentanoic acid (98 mg) as a colorless powder. To the resulting powder (45 mg), an aqueous solution of 3 M hydrochloric acid was added and the mixture was stirred for an hour at room temperature. The reaction system was concentrated in vacuo to give the titled compound (compound 7, 48 mg) as a colorless powder.
MS(ESI/APCI Dual): 314(M+H)
¹H NMR (600 MHz, DEUTERIUM OXIDE) δ ppm 1.65 - 1.88 (m, 4H), 2.55 (s, 3 H), 2.80 - 2.99 (m, 5 H), 2.99 - 3.20 (m, 4 H), 7.24 - 7.40 (m, 3 H), 9.09 (s, 1 H)

### Example 8

### Synthesis of 5-amino-2-[(6-ethyl-4,5-dihydroimidazo[1,5-a]quinolin-3-yl)methyl]pentanoic acid

### (1) Synthesis of 4-ethenyl-2,3-dihydro-1H-inden-1-one

To a solution of 4-bromo-2,3-dihydro-1H-inden-1-one (3.58 g) in dioxane (71.6 ml), triphenylphosphine (1.33 g), dibutyl vinylboronate (3.54 g), palladium acetate (381 mg) and an aqueous solution of 2 M sodium carbonate (42.5 ml) were added and the mixture was stirred for 2.5 hours at 85°C. After adding ethyl acetate, the insolubles were filtered off and the filtrate was washed with brine. After drying over anhydrous sodium sulfate, the desiccant was filtered off and the solvent was removed in vacuo. The resulting residue was purified by silica gel column chromatography (eluent: n-hexane/ethyl acetate/chloroform = 8:1:1 to 7:1:2) to give the titled compound (2.35 g) as a pale yellow powder.
MS(EI): 158(M+)
¹H NMR (300 MHz, CHLOROFORM-d) δppm 2.64 - 2.83 (m, 2 H), 3.10 - 3.28 (m, 2 H), 5.37 - 5.54 (m, 1 H), 5.73 - 5.94 (m, 1 H), 6.80 - 6.99 (m, 1H), 7.33 - 7.49 (m, 1 H), 7.62 - 7.83 (m, 2 H)

### (2) Synthesis of 4-ethenyl-N-hydroxy-2,3-dihydro-1H inden-1-imine

To a solution of 4-ethenyl-2,3-dihydro-1H-inden-1-one (5.82 g) in a mixture of methanol (29 ml) and pyridine (29 ml), hydroxylamine hydrochloride (5.37 g) was added and the resulting mixture was stirred for 3 hours with heating udner reflux. After adding a saturated aqueous solution of ammonium chloride to the reaction mixture, extraction with chloroform and washing with brine were conducted. After drying over sodium carbonate, the desiccant was filtered off and the solvent was removed in vacuo. The resulting residue was purified by silica gel column chromatography (eluent: n-hexane/ethyl acetate/chloroform = 7:1:2) to give the titled compound (2.98 g) as a colorless powder.
MS(ESI/APCI Dual): 174(M+H)
¹H NMR (300 MHz, CHLOROFORM-d) δppm 2.95 - 3.06 (m, 2 H), 3.06 - 3.17 (m, 2 H), 5.37 (d, J=11.0 Hz, 1 H), 5.76 (d, J=17.6 Hz, 1 H,), 6.80 (dd, J=17.6, 11.0 Hz, 1 H), 7.22 - 7.33 (m, 1 H), 7.51 (d, J=7.5 Hz, 1 H), 7.59 (d, J=7.5 Hz, 1 H), 7.66 - 7.91 (m, 1 H)

### (3) Synthesis of 4-ethenyl-N-{[(4-methylphenyl)sulfonyl]oxy}-2,3-dihydro-1H-inden-1-imine

To a solution of 4-ethenyl-N-hydroxy-2,3-dihydro-1H-inden-1-imine (6.06 g) in acetone (180 ml), 4-methylbenzene-1-sulfonyl chloride (7.34 g) and an aqueous solution of 6 M sodium hydroxide (18 ml) were added dropwise under cooling with ice and the mixture was stirred for an hour at the same temperature. After quenching the reaction by adding ice water (400 ml) to the reaction mixture, acetone was removed in vacuo. Extraction with chloroformm and washing with brine were performed. After drying over anhydrous magnesium sulfate, the desiccant was filtered off and the solvent was removed in vacuo. The resulting residue was purified by silica gel column chromatography (eluent: n-hexane/ethyl acetate/chloroform = 5:1:2) to give the titled compound (11.35 g) as a colorless powder.
MS(ESI/APCI Dual): 328(M+H)
¹H NMR (300 MHz, CHLOROFORM-d) δppm 2.44 (s, 3 H), 2.97 - 3.12 (m, 4 H), 5.38 (dd, J=11.1, 0.9 Hz, 1 H), 5.74 (dd, J=17.6, 0.9 Hz, 1 H), 6.74 (dd, J=17.6, 11.1 Hz, 1 H), 7.22 - 7.31 (m, 1 H), 7.35 (d, J=8.0 Hz, 2 H), 7.52 - 7.62 (m, 2 H), 7.94 (d, J=8.0 Hz, 2 H)

### (4) Synthesis of 5-ethenyl-3,4-dihydroquinolin-2(1H)-on

To a solution of 4-ethenyl-N-{[(4-methylphenyl)sulfonyl]oxy}-2,3-dihydro-1H-inden-1-imine (5.0 g) in chloroform (150 ml), trichloroaluminum (6.11 g) was added at -70°C and the mixture was stirred for an hour. After stirring the mixture for an additional 2 hours at -50°C, water was added and extraction was conducted with chloroform. After drying over sodium carbonate, the desiccant was filtered off and the solvent was removed in vacuo. The resulting residue was purified by silica gel column chromatography (eluent: n-hexane/ethyl acetate/chlroform = 1:1:1 to 1:2:1) to give the titled compound (2.1 g) as a colorless powder.
MS(ESI/APCI Dual): 174(M+H)
¹H NMR (300 MHz, CHLOROFORM-d) δppm 2.56 - 2.72 (m, 2 H), 2.96 - 3.09 (m, 2 H), 5.38 (dd, J=11.0, 1.3 Hz, 1 H), 5.66 (dd, J=17.3, 1.3 Hz, 1 H), 6.69 (dd, J=6.6, 2.3 Hz, 1 H), 6.91 (dd, J=17.3, 11.0 Hz, 1 H), 7.10 - 7.22 (m, 2 H), 8.02 (br. s., 1 H)

### (5) Synthesis of ethyl 6-ethenyl-4,5-dihydrolmidazo[1,5-a]quinoline-3-carboxylate

Using 5-ethenyl-3,4-dihydroquinolin-2(1H)-one (1.74 g), reaction and purification were performed by the same procedures as in Example 1(1) and recrystallization from ethyl acetate gave the titled compound (1.66 g) as a colorless powder.
MS(ESI/APCI Dual): 269(M+H)
¹H NMR (300 MHz, CHLOROPORM-d) δppm 1.43 (t, J=7.1 Hz, 3 H), 2.93 - 3.04 (m, 2 H), 3.28 - 3.40 (m, 2 H), 4.41 (q, J=7.1 Hz, 2 H), 5.45 (dd, J=11.0, 1.2 Hz, 1 H), 5.70 (dd, J=17.4, 1.2 Hz, 1 H), 6.98 (dd, J=17.4, 11.0 Hz, 1 H), 7.29 - 7.37 (m, 1 H), 7.38 - 7.48 (m, 2 H), 8.00 (s, 1 H)

### (6) Synthesis of (6-ethenyl-4,5-dihydroimidazo[1,5-a]quinolin-3-yl)methanol

Using ethyl 6-ethenyl-4,5-dihydroimidazo[1.5-a]quinoline-3-carboxylate (2.4 g), reaction and purification were performed by the same procedures as in Example 1(2) to give the titled compound (480 mg) as a pale yellow oil. MS(ESI/APCI Dual): 227(M+H)
¹H NMR (300 MHz, CHLOROFORM-d) δppm 2.96 (s, 4 H), 4.65 (s, 2 H), 5.43 (dd, J=11.0, 1.2 Hz, 1 H), 5.68 (dd, J=17.3, 1.2 Hz, 1 H), 6.98 (dd, J=17.3, 11.0 Hz, 1 H), 7.28 - 7.34 (m, 1 H), 7.34 - 7.41 (m, 2 H), 7.98 (s, 1 H)

### (7) Synthesis of 6-ethenyl-4,5-dihydroimidazo[1,5-a]quinoline-3-carbaldehyde

Using (6-ethenyl-4,5-dihydroimidazo[1,5-a]quinolin-3-yl)methanol (480 mg), reaction and after-treatment were performed by the same procedures as in Example 1(3) and the resulting residue was purified by silica gel column chromatography (eluent: chloroform/methanol = 9:1) to give the titled compound (360 mg) as a pale yellow powder.
MS(ESI/APCI Dual): 225(M+H)
¹H NMR (300 MHz, CHLOROFORM-d) δppm 2.95 - 3.04 (m, 2 H), 3.29 - 3.38 (m, 2 H), 5.47 (dd, J=11.0, 1.1 Hz, 1 H), 5.71 (dd, J=17.4, 1.1 Hz, 1 H), 6.98 (dd, J=17.4, 11.0 Hz, 1 H), 7.31 - 7.49 (m, 3 H), 8.05 (s, 1 H), 10.02 (s, 1 H)

### (8) Synthesis of tert-butyl (3E)-3-[(6-ethenyl-4,5-dihydroimidazo[1,5-a]quinolin-3-yl)methylidenl-2-oxopiperidine-1-carboxylate

Using 6-ethenyl-4,5-dihydrolmidazo[1,5-alquinoline-3-carbaldehyde (530 mg), reaction and after-treatment were performed by the same procedures as in Example 1(4) to give the titled compound (397 mg) as a pale yellow powder. MS(ESI): 406(M+H)
¹H NMR (300 MHz, CHLOROFORM-d) δppm 1.57 (s, 9 H), 1.88 - 2.00 (m, 2 H), 2.89 - 3.11 (m, 4 H), 3.22 - 3.33 (m, 2 H), 3.73 - 3.81 (m, 2 H), 5.44 (dd, J=11.0, 1.2 Hz, 1 H), 5.69 (dd, J=17.3, 1.2 Hz, 1H), 6.98 (dd, J.17.3, 11.0 Hz, 1 H), 7.30 - 7.36 (m, 1 H), 7.36 - 7.44 (m, 2 H), 7.67 - 7.74 (m, 1 H), 8.06 (s, 1 H)

### (9) Synthesis of tert-butyl 3-[(6-ethyl-4,5-dihydroimidazo[1,5-a]quinolin-3-yl)methyl]-2-oxopiperidine-1-carboxylate

To a solution of tert-butyl (3E)-3-[(6-ethenyl-4,5-dihydroimidazo[1,5-a]quinolin-3-yl)methyliden]-2-oxopiperidine-1-carboxylate (397 mg) in tetrahydrofuran (15 ml), 10% palladium-activated carbon (200 mg) was added and the mixture was stirred under hydrogen purge for 16 hours at 60°C. The reaction mixture was filtered through Celite and the solvent was removed in vacuo. To a solution of the resulting residue in acetonitrile (0.77 ml), di-tert-butyl dicarbonate (65 mg) and 4-dimethylaminopyridine (1.5 mg) were added and the mixture was stirred for 5 hours at 55°C, then overnight at room temperatuare. After adding a saturated aqueous solution of sodium hydrogencarbonate to the reaction mixture, extraction with ethyl acetate and washing with brine were performed. After drying over sodium carbonate, the desiccant was filtered off and the solvent was removed in vacuo. The resulting residue was purified by silica gell column chromagography (eluent: chloroform/methanol = 20:1) to give the titled compound (74 mg) as an orange oil.
MS(ESI/APCI Dual): 410(M+H)
¹H NMR (300 MHz, CHLOROFORM-d) δppm 1.21 (t, J=7.5 Hz, 3 H), 1.52 (s, 9 H), 1.54 - 1.69 (m, 2 H), 1.70 - 1.93 (m, 2 H), - 2.15 (m, 1 H), 2.60 - 2.76 (m, 3 H), 2.77 - 3.00 (m, 4 H), 3.21 (dd, J=14.4, 4.7 Hz, 1 H), 3.50 - 3.63 (m, 1 H), 3.74 - 3.85 (m, 1 H), 7.06 (dd, J=7.3, 1.6 Hz, 1 H), 7.17 - 7.30 (m, 2 H), 7.92 (s, 1 H)

### (10) Synthesis of 5-amino-2-[(6-ethyl-4,5-dihydroimidazo[1,5-a] quinolin-3-yl)methyl]pentanoic acid

Using tert-butyl 3-[(6-ethyl-4,5-dihydroimidazo[1,5-a]quinolin-3-yl)methyl]-2-oxopiperidine-1-carboxylate (74 mg), reaction and after-treatment were performed by the same procedures as in Exmple 1(6) to give the titled compound (compound 8, 20 mg) as a brown powder.
MS(ESI/APCI Dual): 328(M+H)
¹H NMR (300 MHz, DEUTERIUM OXIDE) δ ppm 1.15 (t, J=7.5 Hz, 3 H), 1.43 - 1.79 (m, 4 H), 2.50 - 2.88 (m, 9 H), 2.94 - 3.04 (m, 2 H), 7.10 - 7.41 (m, 3 H), 8.16 (s, 1 H)

### Example 9

### Synthesis of 5-amino-2-[(6-chloro-4,5-dihydroimidazo[1,5-a]quinolin-3-yl)methyl]pentanoic acid

### (1) Synthesis of ethyl 6-chloro-4,5-dihydroimidazo[1,5-a]quinolin-3-carboxylate

Using 5-chloro-3,4-dihydroquinolin-2(1H)-one (2.00 g), reaction and purification were performed by the same procedures as in Example 1(1) to give the titled compound (2.09 g) as a pale yellow powder.
MS(ESI/APCI Dual): 277(M+H)

### (2) Synthesis of (6-chloro-4,5-dihydroimidazo[1,5-a]quinolin-3-yl)methanol

Using ethyl 6-chloro-4,5-dihydroimidazo[1,5-a]quinolin-3-carboxylate (2.09 g), reaction was performed by the same procedure as in Example 1(2) to give the titled compound (1.70 g) as a yellow powder.
MS(ESI/APCI Dual): 235(M+H)

### (3) Synthesis of 6-chloro-4,5-dihydroimidazo[1,5-a]quinolin-3-carbaldehyde

Using (6-chloro-4,5-dihydroimidazo[1,5-a]quinolin-3-yl)methanol (1.70 g), reaction and purification were performed by the same procedures as in Example 1(3) to give the titled compound (1.33 g) as a pale brown powder.
MS(ESI/APCI Dual): 233(M+H)

### (4) Synthesis of tert-butyl (3E)-3-[(6-chloro-4,5-dihydroimidazo[1,5-a]quinolin-3-yl)methyliden]-2-oxopiperidine-1-carboxylate

Using 6-chloro-4,5-dihydroimidazo[1,5-a]quinolin-3-carbaldehyde (1.32 g), reaction and purification were performed by the same procedures as in Example 1(4) and reprecipitation from n-hexane/chloroform gave the titled compound (760 mg) as a pale yellow powder.
MS(ESI/APCI Dual): 414(M+H)

### (5) Synthesis of tert-butyl 3-[(6-chloro-4,5-dihydroimidazo[1,5-a]quinolin-3-yl)methyl]-2-oxopiperidine-1-carboxylate

To a solution of tert-butyl (3E)-3-[(6-chloro-4,5-dihydroimidazo[1,5-alquinolin-3-yl)methylidenl-2-oxopiperidine-1-carboxylate (100 mg) in a mixture of methanol and tetrahydrofuran (1:1, 4.84 ml), 10% palladium-activated carbon (20 mg) was added and the mixture was stirred under hydrogen purge for 15 hours at room temperature. The reaction mixture was filtered through Celite and the solvent was removed in vacuo. The resulting residue was purified by silica gel column chromatography (eluent: ethyl acetate/methanol = 1:0 to 10:1) to give the titled compound (72 mg) as a yellow oil.
MS(ESI/APCI Dual): 416(M+H)
¹H NMR (300 MHz, CHLOROFORM-d) d ppm 1.52 (s, 9 H), 1.55 - 1.96 (m, 3 H), 1.96 - 2.16 (m, 1 H), 2.68 (dd, J=14.3, 8.2 Hz, 1 H), 2.77 - 3.11 (m, 5 H), 3.20 (dd, J=14.3, 4.8 Hz, 1 H), 3.47 - 3.68 (m, 1 H), 3.69 - 3.92 (m, 1 H), 7.20 - 7.35 (m, 3 H), 7.92 (s, 1 H)

### (6) Synthesis of 5-amino-2-[(6-chloro-4,5-dihydroimidazo[1,5-a]quinolin-3-yl)methyl]pentanoic acid

Using tert-butyl 3-[(6-chloro-4,5-dihydroimidazo[1,5-a]quinolin-3-yl)methyl]-2-oxopiperidine-1-carboxylate (72 mg), reaction and purification were performed by the same procedures as in Example 1(6) to give the titled compound (compound 9, 20 mg) as a pale brown powder.
MS(ESI/APCI Dual): 334(M+H)
¹H NMR (600 MHz, DEUTERIUM OXIDE) 5 ppm 1.45 - 1.75 (m, 4 H), 2.48 - 2.64 (m, 2 H), 2.64 - 2.89 (m, 5 H), 2.91 - 3.07 (m, 2 H), 7.03 - 7.18 (m, 2 H), 7.19 - 7.33 (m, 1 H), 8.09 (br. s., 1 H)

### Example 10

### Synthesis of 5-amino-2-[(6-methyl-4,5-dihydroimidazo[1,5-a]quinolin-3-yl)methyl]pentanoic acid

### (1) Synthesis of ethyl 6-methyl-4,5-dihydroimidazo[1,5-a] quinoline-3-carboxylate

Using 5-methyl-3,4-dihydroquinolin-2(1H)-one (390 mg), reaction and purification were performed by the same procedures as in Example 1(1) to give the titled compound (423 mg) as a colorless powder.
MS(ESI/APCI Dual) : 257(M+H)

### (2) Synthesis of (6-methyl-4,5-dihydroimidazo[1,5-a]quinolin-3-yl)methanol

Using ethyl 6-methyl-4,5-dihydroimidazo[1,5-a]quinoline-3-carboxylate (420 mg), reaction was performed by the same procedure as in Example 1(2) to give the titled compound (334 mg) as a yellow powder.
MS(ESI/APCI Dual): 215(M+H)

### (3) Synthesis of 6-methyl-4,5-dihydroimidazo[1,5-a]quinoline-3-carbaldehyde

Using (6-methyl-4,5-dihydroimidazo[1,5-a]quinolin-3-yl)methanol (330 mg), reaction and purification were performed by the same procedures as in Example 1(3) to give the titled compound (252 mg) as a colorless powder,
MS(ESI/APCI Dual): 213(M+H)

### (4) Synthesis of tert-butyl (3E)-3-[(6-methyl-4,5-dihydroimidazo[1,5-a]quinolin-3-yl)methyliden]-2-oxopiperidine-1-carboxylate

Using 6-methyl-4,5-dihydroimidazo[1,5-a]quinoline-3-carbaldehyde (245 mg), reaction and purification were performed by the same procedures as in Example 1(4) to give the titled compound (269 mg) as a pale yellow powder.
MS(ESI/APCI Ducal) : 394(M+H)

### (5) Synthesis of tert-butyl 3-[(6-methyl-4,5-dihydroimidazo[1,5-a]quinolin-3-yl)methyl]-2-oxopiperidine-1-carboxylate

Using tert-butyl (3E)-3-[(6-methyl-4,5-dihydroimidazo[1,5-a]quinolin-3-yl)methyliden]-2-oxopiperidine-1-carboxylate (265 mg), reaction and purification were performed by the same procedures as in Example 1(5) to give the titled compound (195 mg) as a colorless viscous substance.
MS(ESI/APCI Dual): 396(M+H)

### (6) Synthesis of 5-amino-2-[(6-methyl-4,5-dihydroimidazo[1,5-a]quinolin-3-yl)methyl]pentanoic acid

Using tert-butyl 3-[(6-methyl-4,5-dihydroimidazo[1,5-alquinolin-3-yl)methyl]-2-oxopiperidine-1-carboxylate (190 mg), reaction and purification were performed by the same procedures as in Example 1(6). Since a crystal precipitated within water, it was recovered by filtration to give the titled compound (compound 10, 68 mg) as a colorless powder. MS(ESI/APCI Dual): 314(M+H)
¹H NMR (300 MHz, METHANOL-d4) δ ppm 1.40 - 1.80 (m, 4 H), 2.35 (s, 3 H), 2.48 - 2.68 (m, 2 H), 2.80 - 3.02 (m, 7 H), 7.08 (d, J=7.8 Hz, 1 H), 7.19 (t, J=7.8 Hz, 1 H), 7.43 (d, J=7.8 Hz, 1 H), 8.13 (s, 1H)

### Example 11

### Synthesis of 5-amino-2-[(7-methyl-4,5-dihydroimidazo[1,5-a]quinolin-3-yl)methyl]pentanoic acid dihydrochloride

### (1) Synthesis of ethyl 7-methyl-4,5-dihydroimidazo[1,5-a]quinoline-3-carboxylate

Using 6-methyl-3,4-dihydroquinolin-2(1H)-one (710 mg), reaction and purification were performed by the same procedures as in Example 1(1) to give the titled compound (752 mg) as a pale yellow powder.
MS(ESI/APCI Dual): 257(M+H)

### (2) Synthesis of (7-methyl-4,5-dihydroimidazo[1,5-a]quinolin-3-yl)methanol

Using ethyl 7-methyl-4,5-dihydroimidazo[1,5-a]quinoline-3-carboxylate (737 mg), reaction was performed by the same procedure as in Example 1(2) to give the titled compound (510 mg) as a pale yellow powder.
MS(ESI/APCI Dual): 215(M+H)

### (3) Synthesis of 7-methyl-4,5-dihydroimidazo[1,5-a]quinoline-3-carbaldehyde

Using (7-methyl-4,5-dihydroimidazo[1,5-a]quinolin-3-yl)methanol (490 mg), reaction and purification were performed by the same procedures as in Example 1(3) to give the titled compound (379 mg) as a colorless powder.
MS(ESI/APCI Dual): 213(M+H)

### (4) Synthesis of tert-butyl (3E)-3-[(7-methyl-4,5-dihydroimidazo[1,5-a]quinolin-3-yl)methyliden]-2-oxopiperidine-1-carboxylate

Using 7-methyl-4,5-dihydroimidazo[1,5-a]quinoline-3-carbaldehyde (375 mg), reaction and purification were performed by the same procedures as in Example 1(4) to give the titled compound (60 mg) as a pale brown powder. MS(ESI/APCI Dual): 394(M+H)

### (5) Synthesis of tert-butyl 3-[(7-methyl-4,5-dihydroimidazo[1,5-a]quinolin-3-yl)methyl]-2-oxopiperidine-1-carboxylate

Using tert-butyl (3E)-3-[(7-methyl-4,5-dihydroimidazo[1,5-a]quinolin-3-yl)methyliden]-2-oxopiperidine-1-carboxylate (58 mg), reaction and purification were performed by the same procedures as in Example 1(5) to give the titled compound (25 mg) as a colorless amorphous mass.
MS(ESI/APCI Dual): 396(M+H)

### (6) Synthesis of 5-amino-2-[(7-methyl-4,5-dihydroimidazo[1,5-a]quinolin-3-yl)methyl]pentanoic acid dihydrochloride

To a solution of tert-butyl 3-[(7-methyl-4,5-dihydroimidazo[1,5-a]quinolin-3-yl)methyl]-2-oxopiperidine-1-carboxylate (24 mg) in tetrahydrofuran (2 ml), an aqueous solution of 0.42 M lithium hydroxide (0.5 ml) was added and the mixture was stirred for 2 hours at room temperature. To the reaction mixture, water (30 ml) was added and extraction was conducted with diethyl ether (40 ml). A saturated aqueous solution of citric acid (2 ml) was added to the aqueous layer to render it acidic and, then, extraction was conducted with chloroform (60 ml). After drying over anhydrous magnesium sulfate, the desiccant was filtered off and the solvent was removed in vacuo. To the residue, a solution of 4 M hydrochloric acid in ethyl acetate (4 ml) was added and the mixture was stirred for 20 hours at room temperature. The solvent was removed in vacuo to give the titled compound (compound 11, 15 mg) as a pale brown powder.
MS(ESI/APCI Dual): 314(M+H)
¹H NMR (300 MHz, METHANOL-d4) δ ppm 1.66 - 1.87 (m, 4 H), 2.40 (s, 3 H), 2.73 - 3.15 (m, 9 H), 7.24 - 7.34 (m, 2 H), 7.67 (d, J=8.1 Hz, 1 H), 9.49 (s, 1 H)

### Example 12

### Synthesis of 5-amino-2-[(8-methyl-4,5-dihydroimidazo[1,5-a]quinolin-3-yl)methyl]pentanoic acid

### (1) Synthesis of ethyl 8-methyl-4,5-dihydroimidazo[1,5-a]quinoline-3-carboxylate

Using 7-methyl-3,4-dihydroquinolin-2(1H)-one (710 mg), reaction and purification were performed by the same procedures as in Example 1(1) to give the titled compound (630 mg) as a pale yellow powder.
MS(ESI/APCI Dual): 257(M+H)

### (2) Synthesis of (8-methyl-4,5-dihydroimidazo[1,5-a]quinolin-3-yl)methanol

Using ethyl 8-methyl-4,5-dihydroimidazo[1,5-a]quinoline-3-carboxylate (622 mg), reaction was performed by the same procedure as in Example 1(2) to give the titled compound (420 mg) as a yellow powder.
MS(ESI/APCI Dual): 215(M+H)

### (3) Synthesis of 8-methyl-4,5-dihydroimidazo[1,5-a]quinoline-3-carbaldehyde

Using (8-methyl-4,5-dihydroimidazo[1,5-a]quinolin-3-yl)methanol (410 mg), reaction and purification were performed by the same procedures as in Example 1(3) to give the titled compound (334 mg) as a colorless powder.
MS(ESI/APCI Dual): 213(M+H)

### (4) Synthesis of tert-butyl (3E)-3-[(8-methyl-4,5-dihydroimidazo[1,5-a]quinolin-3-yl)methyliden]-2-oxopiperidine-1-carboxylate

Using 8-methyl-4,5-dihydroimidazo[1,5-a]quinoline-3-carbaldehyde (328 mg), reaction and purification were performed by the same procedures as in Example 1(4) to give the titled compound (127 mg) as a colorless powder.
MS(ESI/APCI Dual): 394(M+H)

### (5) Synthesis of tert-butyl 3-[(8-methyl-4,5-dihydroimidazo[1,5-a]quinolin-3-yl)methyl]-2-oxopiperidine-1-carboxylate

Using tert-butyl (3E)-3-[(8-methyl-4,5-dihydroimidazo[1,5-a]quinolin-3-yl)methyliden]-2-oxopiperidine-1-carboxylate (122 mg), reaction and purification were performed by the same procedures as in Example 1(5) to give the titled compound (78 mg) as a colorless oil.
MS(ESI/APCI Dual): 396(M+H)

### (6) Synthesis of 5-amino-2-[(8-methyl-4,5-dihydroimidazo[1,5-a]quinolin-3-yl)methyl]pentanoic acid

Using tert-butyl 3-[(8-methyl-4,5-dihydrolmidazo[1,5-a]quinolin-3-yl)methyl]-2-oxopiperadine-1-carboxylate (74 mg), reaction and purification were performed by the same procedures as in Example 1(6) to give the titled compound (compound 12, 31 mg) as a pale brown amorphous mass.
MS(ESI/APCI Dual): 314(M+H)
¹H NMR (300 MHz, DEUTERIUM OXIDE) δ ppm 1.30 - 1.68 (m, 4 H), 2.23 (s, 3 H), 2.34 - 2.61 (m, 2 H), 2.61 - 2.80 (m, 5 H), 2.81 - 2.98 (m, 2 H), 6.99 (d, J=7.6 Hz, 1 H), 7.15 (d, J=7.6 Hz, 1 H), 7.27 (s, 1 H), 8.21 (br. s., 1 H)

### Example 13

### Synthesis of 6-amino-2-(4,5-dihydroimidazo[1,5-a]quinolin-3-yl)hexanoic acid

### (1) Synthesis of cyano(4,5-dihydroimidazo[1,5-a]quinolin-3-yl)methyl ethyl carbonate

To a solution of (4,5-dihydroimidazo[1,5-a]quinoline-3-carbaldehyde (2.00 g) in chloroform (20.2 ml), tetrabutylammonium chloride (290 mg), ethyl chloroformate (1.31 g) and an aqueous solution of 1.1 M sodium cyanide (20.2 ml) were added and the mixture was stirred for 3 hours at room temperature. To the reaction system, water was added and extraction was conducted with chloroform. After drying over anhydrous sodium sulfate, the desiccant was filtered off and the solvent was removed in vacuo. The residue was purified by silica gel column chromatography (eluent: n-hexane/ethyl acetate = 1:1) to give the titled compound (2.46 g) as a yellow oil.
MS(ESI/APCI Dual): 298(M+H)
¹H NMR (300 MHz, CHLOROFORM-d) δ ppm 1.33 (t, J=7.1 Hz, 3 H), 2.89 - 3.03 (m, 2 H), 3.03 - 3.25 (m, 2 H), 4.20 - 4.40 (m, 2 H), 6.38 (s, 1H), 7.20 - 7.29 (m, 1 H), 7.29 - 7.40 (m, 2 H), 7.40 - 7.48 (m, 1H), 8.04 (s, 1H)

### (2) Synthesis of 4,5-dihydroimidazo[1,5-a]quinolin-3-ylacetonitrile

To a solution of cyano(4,5-dihydroimidazo[1,5-a] quinolin-3-yl)methyl ethyl carbonate (2.46 g) in ethyl acetate (82.7 ml), 10% palladium-activated carbon (246 mg) was added and the mixture was stirred under hydrogen purge for 5 hours at 70°C. The reaction mixture was filtered through Celite and the solvent was removed in vacuo. The resulting residue was purified by silica gel column chromatography (eluent: n-hexane/ethyl acetate = 1:1 to 0:1) to give the titled compound (1.15 g) as a pale yellow crystal.
MS(ESI/APCI Dual): 210(M+H)
¹H NMR (300 MHz, CHLOROFORM-d) δ ppm 2.86 - 3.09 (m, 4 H), 3.74 (s, 2 H), 7.16 - 7.25 (m, 1 H), 7.28 - 7.38 (m, 2 H), 7.38 - 7.51 (m, 1 H), 7.98 (s, 1 H)

### [4185] (3) Synthesis of ethyl cyano(4,5-dihydroimidazo[1,5-a]quinolin-3-yl)acetate

To a solution of 4,5-dihydroimidazo[1,5-a]quinolin-3-ylacetonitrile (600 mg) in tetrahydrofuran (28.7 ml), lithium hexamethyldisilazane (4.30 ml as 1 M solution in tetrahydrofuran) and the mixture was stirred for 30 minutes at the same temperature. Ethyl chloroformate (373 mg) was added and the mixture was stirred for an additional hour at the same temperature. To the reaction system, a saturated aqueous solution of ammonium chloride was added and extraction was conducted with ethyl acetate. After drying over anhydrous sodium sulfate, the desiccant was filtered off and the solvent was removed in vacuo. The residue was purified by silica gel column chromatography (eluent: n-hexane/ethyl acetate = 1:1) to give the titled compound (370 mg) as a yellow oil.
MS(ESI/APCI Dual): 282(M+H)
¹H NMR (300 MHz, CHLOROFORM-D) δ ppm 1.32 (t, J=7.1 Hz, 3 H), 2.88 - 3.05 (m, 4 H), 4.21 - 4.40 (m, 2H), 4.89 (s, 1 H), 7.17 - 7.26 (m, 1 H), 7.29 - 7.40 (m, 2H), 7.39 - 7.52 (m, 1 H), 8.00 (s, 1 H)

### (4) Synthesis of ethyl 6-[(tert-butoxycarbonyl)amino]-2-cyano-2-(4,5-dihydroimidazo[1,5-a]quinolin-3-yl)hexanoate

To a solution of ethyl cyano(4,5-dihydroimidazo[1,5-a]quinolin-3-yl)acetate (195 ml) in N,N-dimethylformamide (6.9 ml), sodium hydride (60%, 36 mg) was added under cooling with ice and the mixture was stirred for 30 minutes at the same temperature. Then, tert-butyl 4-iodobutyl carbamate (249 mg) was added and the mixture was stirred for 3 hours at room temperature. To the reaction system, a saturated aqueous solution of ammonium chloride was added and extraction was conducted with ethyl acetate. After drying over anhydrous sodium sulfate, the desiccant was filtered off and the solvent was removed in vacuo. The residue wa purified by NH silica gel column chromatography (eluent: n-hexane/ethyl acetate = 1:2) to give the titled compuond (89 mg) as a pale yellow oil.
MS(ESI/APCI Dual): 453(M+H)
¹H NMR (300 MHz, CHLOROFORM-D) δ ppm 1.29 (t, J=7.1 Hz, 3 H), 1.44 (s, 9 H), 1.51 - 1.74 (m, 4 H), 2.36 - 2.54 (m, 2 H), 2.85 - 3.31 (m, 6 H), 4.28 (q, J=7.1 Hz, 2 H), 4.78 - 4.95 (m, 1 H), 7.17 - 7.25 (m, 1 H), 7.29 - 7.37 (m, 2 H), 7.37 - 7.49 (m, 1 H), 7.98 (s, 1 H)

### (5) Synthesis of 6-amino-2-(4,5-dihydroimidazo[1,5-a]quinolin-3-yl)hexanoic acid

To a solution of ethyl 6-[(tert-butoxycarbonyl)amino]-2-cyano-2-(4,5-dihydroimidazo[1,5-a]quinolin-3-yl)hexanoate (89 mg), an aqueous solution of 6 M hydrochloric acid (2.0 ml) was added and the mixture was stirred for 10 hours at 100°C. The reaction system was concentrated in vacuo and after adding water (3 ml) and Amberlite IRA-67 (890 mg) to the residue, the mixture was stirred for 30 minutes at room temperature. The reaction system was filtered and the filtrate was concentrated in vacuo to give the titled compound (compound 13, 50 mg) as a pale yellow powder.
MS(ESI/APCI Dual): 300(M+H)
¹H NMR (600 MHz, DEUTERIUM OXIDE) δ ppm 1.30 - 1.48 (m, 2 H), 1.69 - 1.78 (m, 2 H), 1.81 - 1.91 (m, 1 H), 2.02 - 2.13 (m, 1 H), 2.77 - 2.92 (m, 4 H), 2.99 - 3.04 (m, 2 H), 3.60 (t, J=7.8 Hz, 1 H), 7.24 - 7.30 (m, 1 H), 7.30 - 7.40 (m, 2 H), 7.50 (d, J=7.8 Hz, 1 H), 8.54 (s, 1 H)

### Example 14

### Synthesis of 5-amino-2-[(7-ethoxy-4,5-dihydroimidazo[1,5-a]quinolin-3-yl)methyl]pentanoic acid

### (1) Synthesis of ethyl 7-(benzyloxy)-4,5-dihydroimidazo[1,5-a]quinoline-3-carboxylate

Using 6-(benzyloxy)-3,4-dihydroquinolin-2(1H)-one (29.9 g), reaction and purification were performed by the same procedures as in Example 1(1) to give the titled compound (8.00 g) as a pale brown powder.
MS(ESI/APCI Dual): 349(M+H)

### (2) Synthesis of [7-(benzyloxy)-4,5-dihydroimidazo[1,5-a]quinolin-3-yl]methanol

Using ethyl 7-(benzyloxy)-4,5-dihydroimidazo[1,5-a]quinoline-3-carboxylate (8.00 g), reaction was performed by the same procedure as in Example 1(2) to give the titled compound (4.67 g) as a brown powder.
MS(ESI/APCI Dual): 307(M+H)

### (3) Synthesis of 7-(benzyloxy)-4,5-dihydroimidazo[1,5-a]quinoline-3-carbaldehyde

Using [7-(benzyloxy)-4,5-dihydroimidazo[1,5-a]quinolin-3-yl]methanol (4.67 g), reaction and purification were performed by the same procedures as in Example 1(3) to give the titled compound (3.25 g) as a pale brown powder.
MS(ESI/APCI Dual): 305(M+H)

### (4) Synthesis of tert-butyl (3E)-3-{[7-(benzyloxy)-4,5-dihydroimidazo[1,5-a]quinolin-3-yl]methyliden}-2-oxopiperidine-1-carboxylate

Using 7-(benzyloxy)-4,5-dihydroimidazo[1,5-a]quinoline-3-carbaldehyde (3.25 g), reaction and purification were performed by the same procedures as in Example 1(4) to give the titled compound (2.23 g) as a yellow powder.
MS(ESI/APCI Dual): 486(M+H)

### (5) Synthesis of tert-butyl 3-[(7-hydroxy-4,5-dihydroimidazo[1,5-a] quinolin-3-yl)methyl]-2-oxopiperidine-1-carboxylate

To a solution of tert-butyl (3E)-3-{[7-(benzyloxy)-4,5-dihydroimidazo[1,5-a]quinolin-3-yl]methyliden}-2-oxopiperidine-1-carboxylate (2.23 g) in a mixture of methanol and tetrahydrofuran (1:1, 46.0 ml), 10% palladium-activated carbon (446 mg) was added and the mixture was stirred under hydrogen purge for 10 hours at 60°C. The reaction mixture was filtered through Celite and the solvent was removed in vacuo. The resulting residue was purified by silica gel column chromatography (eluent: chloroform/methanol = 10:1) to give the titled compound (1.91 g) as a pale yellow powder. MS(ESI/APCI Dual): 398(M+H)
¹H NMR (300 MHz, CHLOROFORM-d) δ ppm 1.38 - 1.68 (m, 10 H), 1.68 - 1.92 (m, 2 H), 1.92 - 2.12 (m, 1 H), 2.67 (dd, J=14.5, 7.9 Hz, 1 H), 2.73 - 2.99 (m, 5 H), 3.19 (dd, J=14.5, 5.3 Hz, 1 H), 3.44 - 3.60 (m, 1 H), 3.71 - 3.89 (m, 1 H), 6.69 - 6.85 (m, 2 H), 7.20 (d, J=8.5 Hz, 1 H), 7.88 (s, 1 H)

### (6) Synthesis of tert-butyl 3-[(7-ethoxy-4,5-dihydroimidazo[1,5-a]quinolin-3-yl)methyl]-2-oxopiperidine-1-carboxylate

To a solution of tert-butyl 3-[(7-hydroxy-4,5-dihydroimidazo[1,5-a]quinolin-3-yl)methyl]-2-oxopiperidine-1-carboxylate (100 mg) in N,N-dimethylformamide (2.5 ml), potassium carbonate (34.8 mg) and ethyl iodide (78.5 mg) were added and the mixture was stirred for 3 hours at room temperature. To the reaction system, water was added and extraction was conducted with chloroform. After drying over anhydrous sodium sulfate, the desiccant was filtered off and the solvent was removed in vacuo. The residue was purified by silica gel column chromatography (eluent: ethyl acetate to chloroform/methanol = 10:1) to give the titled compound (70 mg) as a yellow oil.
MS(ESI/APCI Dual): 426(M+H)
¹H NMR (300 MHz, CHLOROFORM-D) δ ppm 1.42 (t, J=6.9 Hz, 3 H), 1.52 (s, 9 H), 1.54 - 1.94 (m, 3 H), 1.94 - 2.14 (m, 1 H), 2.66 (dd, J=14.3, 8.3 Hz, 1 H), 2.77 - 3.00 (m, 5 H), 3.20 (dd, J=14.3, 4.7 Hz, 1 H), 3.50 - 3.66 (m, 1 H), 3.71 - 3.87 (m, 1 H), 4.04 (q, J=6.9 Hz, 2 H), 6.75 - 6.87 (m, 2 H), 7.28 - 7.36 (m, 1H), 7.87 (s, 1 H)

### (7) Synthesis of 5-[(tert-butoxycarbonyl)amino]-2-[(7-ethoxy-4,5-dihydroimidazo[1,5-a]quinolin-3-yl)methyl]pentanoic acid

To a solution of tert-butyl 3-[(7-ethoxy-4,5-dihydroimidazo[1,5-a]quinolin-3-yl)methyl]-2-oxopiperidine-1-carboxylate (70 mg) in tetrahydrofuran (0.8 ml), an aqueous solution of 0.9 M lithium hydroxide (0.55 ml) was added and the mixture was stirred for 15 hours at room temperature. To the reaction system, water was added and the aqueous layer was washed with diethyl ether. The aqueous layer was neutralized with an aqueous solution of 15% citric acid and extraction was conducted with chloroform. After drying over anhydrous sodium sulfate, the desiccant was filtered off and the solvent was removed in vacuo to give the titled compound (65 mg) as a pale yellow powder.
MS(ESI/APCI Dual): 444(M+H)
¹H NMR (300 MHz, CHLOROFORM-d) δ ppm 1.33 - 1.48 (m, 13 H), 1.48 - 1.69 (m, 2 H), 1.68 - 1.89 (m, 1 H), 2.63 - 3.26 (m, 9 H), 4.05 (q, J=6.9 Hz, 2 H), 4.77 (br. s., 1 H), 6.77 - 6.96 (m, 2 H), 7.52 (d, J=8.5 Hz, 1 H), 8.73 (s, 1 H)

### (8) Synthesis of 5-amino-2-[(7-ethoxy-4,5-dihydroimidazo[1,5-a]quinolin-3-yl)methyl]pentanoic acid

To a solution of 5-[(tert-butoxycarbonyl)amino]-2-[(7-ethoxy-4,5-dihydroimidazo[1,5-alquinolin-3-yl)methyl]pentanoic acid (65 mg) in ethyl acetate (0.7 ml), a solution of 4 M hydrochloric acid in ethyl acetate (0.7 ml) was added and the mixture was stirred for an hour at room temperature. The reaction system was concentrated in vacuo and the resulting powder was dissolved in water (3 ml) and after adding Amberlite IRA-67 (650 ml), the mixture was stirred for 30 minutes at room temperature. The reaction system was filtered and the filtrate was concentrated in vacuo to give the titled compound (compound 14, 30 mg) as a pale yellow powder.
MS(ESI/APCI Dual): 344(M+H)
¹H NMR (600 MHz, DEUTERIUM OXIDE) δ ppm 1.33 (t, J=6.9 Hz, 3 H), 1.44 - 1.61 (m, 2 H), 1.62 - 1.73 (m, 2 H), 2.48 - 2.80 (m, 7 H), 2.93 - 3.03 (m, 2 H), 3.91 (q, J=6.9 Hz, 2 H), 6.60 - 6.67 (m, 1 H), 6.71 (br. s., 1 H), 7.15 (d, J=8.3 Hz, 1 H), 7.89 (s, 1 H)

### Example 15

### Synthesis of 5-amino-2-[(7-phenyl-4,5-dihydroimidazo[1,5-a]quinolin-3-yl)methyl]pentanoic acid dihydrochloride

### (1) Synthesis of tert-butyl 2-oxo-3-[(7-{(trifluoromethyl)sulfonyl]oxy}-4,5-dihydroimidazo[1,5-a]quinolin-3-yl)methyl]piperidine-1-carboxylate

To a solution of tert-butyl 3-[(7-hydroxy-4,5-dihydroimidazo[1,5-alquinolin-3-yl)methyl]-2-oxopiperidine-1-carboxylate (100 mg) in chlorform (2.5 ml), pyridine (39.8 mg) and trifluoromethanesulfonic anhydride (85.3 mg) were added under cooling on a dry ice-acetone bath and the mixture was stirred for an hour under cooling with ice. To the reaction system, a saturated aqueous solution of sodium hydrogencarbonate was added and extraction was conducted with chloroform. After drying over anhydrous sodium sulfate, the desiccant as filtered off and the solvent was removed in vacuo. The residue was purified by silica gel column chromatography (eluent: n-hexane/ethyl acetate = 1:2 to chloroform/methanol = 10:1) to give the titled compound (110 mg) as a yellow oil.
MS(ESI/APCI Dual): 530(M+H)
¹H NMR (300 MHz, CHLOROFORM-d) δ ppm 1.50 (s, 9 H), 1.55 - 1.71 (m, 1 H), 1.71 - 1.94 (m, 2 H), 1.98 - 2.15 (m, 1 H), 2.69 (dd, J=14.3, 7.8 Hz, 1 H), 2.79 - 3.06 (m, 5 H), 3.16 (dd, J=14.3, 5.1 Hz, 1 H), 3.56 (ddd, J=12.9, 7.2, 5.3 Hz, 1 H), 3.80 (ddd, J=12.9, 7.2, 5.3 Hz, 1 H), 7.16 - 7.30 (m, 2 H), 7.41 - 7.51 (m, 1 H), 7.92 (s, 1 H)

### (2) Synthesis of tert-butyl 2-oxo-3-[(7-phenyl-4,5-dihydroimidazo[1,5-a]quinolin-3-yl)methyl]piperidine-1-carboxylate

To a solution of tert-butyl 2-oxo-3-[(7-{[(trifluoromethyl)sulfonyl]oxy}-4,5-dihydroimidazo[1,5-a]quinolin-3-yl)methyl]piperidine-1-carboxylate (110 mg) in 1,4-dioxane (1.0 ml), triphenylphosphine (16.4 mg), phenylboronic acid (27.9 mg), palladium acetate (4.67 mg) and an aqueous solution of 2 M sodium carbonate (0.52 ml) were added and the mixture was stirred for 2 hours at 80°C. To the reaction system, brine was added and extraction was conducted with ethyl acetate. After drying over anhydrous sodium sulfate, the desiccant was filtered off and the solvent was removed in vacuo. The residue was purified by NH silica gel column chromatogprahy (eluent: n-hexane/ethyl acetate = 1:1 to 0:1) to give the titled compound (46 mg) as a colorless powder.
MS(ESI/APCI Dual): 458(M+H)
¹H NMR (300 MHz, CHLOROFORM-d) d ppm 1.40 - 1.68 (m, 10 H), 1.70 - 1.95 (m, 2 H), 1.95 - 2.16 (m, 1 H), 2.61 - 2.77 (m, 1 H), 2.80 - 3.06 (m, 5 H), 3.14 - 3.30 (m, 1 H), 3.50 - 3.65 (m, 1 H), 3.72 - 3.89 (m, 1 H), 7.31 - 7.73 (m, 8 H), 7.97 (s, 1 H)

### (3) Synthesis of 5-C(tert-butoxycarbonyl)amino]-2-[(7-phenyl-4,5-dihydroimidazo[1,5-a]quinolin-3-yl)methyl]pentanoic acid

Using tert-butyl 2-oxo-3-[(7-phenyl-4,5-dihydroimidazo[1,5-a]quinolin-3-yl)methyl]piperidine-1-carboxylate (46 mg), reaction was performed by the same procedure as in Example 14(7) and recrystallization with n-hexane/ethyl acetate gave the titled compound (26 mg) as a colorless powder.
MS(ESI/APCI Dual): 476(M+H)

### (4) Synthesis of 5-amino-2-[(7-phenyl-4,5-dihydroimidazo[1,5-a]quinolin-3-yl)methyl]pentanoic acid dihydrochloride

Using 5-[(tert-butoxycarbonyl)amino-2-[(7-phenyl-4,5-dihydroimidazo[1,5-a]quinolin-3-yl)methyl]pentanoic acid (26 mg), reaction and purification were performed by the same procedures as in Example 14(8). To the resulting powder, a solution of 2 M hydrochloric acid in ethyl acetate (3 ml) was added and the mixture was stirred for an hour at room temperature. The reaction system was concentrated in vacuo to give the titled compound (compound 15, 18 mg) as a yellow powder.
MS(ESI/APCI Dual): 376(M+H)
¹H NMR (600 MHz, DEUTERIUM OXIDE) δ ppm 1.59 - 1.85 (m, 4H), 2.60 - 2.90 (m, 7 H), 2.99 - 3.10 (m, 2 H), 7.17 - 7.53 (m, 8 H), 8.75 (s, 1 H)

### Example 16

### Synthesis of 5-amino-2-{[7-(cyclohexyloxy)-4,5-dihydroimidazo[1,5-a]quinolin-3-yl)methyl]pentanoic acid

### (1) Synthesis of 6-(cyclohexyloxy)-3,4-dihydroquinolin-2(1H)-one

To a solution of 6-hydroxy-3,4-dihydroquinolin-2(1H)-one (3.00 g) in N,N-dimethylformamide (61.3 ml), cesium carbonate (5.99 g) and cyclohexylbromide (6.00 g) were added and the mixture was stirred for 5 hours at 60°C. To the reaction system, water was added and extraction was conducted with chloroform. After drying over anhydrous sodium sulfate, the desiccant was filtered off and the solvent was removed in vacuo. The residue was purified by silica gel column chromatography (eluent: n-hexane/chloroform/ethyl acetate = 1:1:2) to give the titled compound (301 mg) as a pale yellow powder.
MS(ESI/APCI Dual): 246(M+H)
¹H NMR (300 MHz, CHLOROPORM-d) δ ppm 1.22 - 1.57 (m, 6 H), 1.72 - 1.89 (m, 2 H), 1.89 - 2.05 (m, 2 H), 2.54 - 2.67 (m, 2 H), 2.86 - 3.00 (m, 2 H), 4.10 - 4.20 (m, 1 H), 6.60 - 6.80 (m, 3 H), 7.67 (br. s., 1 H)

### (2) Synthesis of ethyl 7-(cyclohexyloxy)-4,5-dihydroimidazo[1,5-a]quinoline-3-carboxylate

Using 6-(cyclohexyloxy)-3,4-dihydroquinolin-2(1H)-one (399 mg), reaction and purification were performed by the same procedures as in Example 1(1) to give the titled compound (360 mg) as a pale brown crystal.
MS(ESI/APCI Dual): 341(M+H)

### (3) Synthesis of [7-(cyclohexyloxy)-4,5-dihydroimidazo[1,5-a]quinolin-3-yl]methanol

Using ethyl 7-(cyclohexyloxy)-4,5-dihydroimidazo[1,5-a]quinoline-3-carboxylate (360 mg), reaction was performed by the same procedure as in Example 1(2) to give the titled compound (304 mg) as a brown oil.
MS(ESI/APCI Dual): 299(M+H)

### (4) Synthesis of 7-(cyclohexyloxy)-4,5-dihydroimidazo[1,5-a]quinoline-3-carbaldehyde

Using [7-(cyclohexyloxy)-4,5-dihydroimidazo[1,5-a]quinolin-3-yl]methanol (304 mg), reaction and purification were performed by the same procedures as in Example 1(3) to give the titled compound (190 mg) as a brown oil.
MS(ESI/APCI Dual): 297(M+H)

### (5) Synthesis of tert-butyl (3E)-3-{[7-(cyclohexyloxy)-4,5-dihydroimidazo[1,5-a]quinolin-3-yl]methyliden}-2-oxopiperidine-1-carboxylate

Using 7-(cyclohexyloxy)-4,5-dihydroimidazo(1,5-a]quinoline-3-carbaldehyde (190 mg), reaction and purification were performed by the same procedures as in Example 1(4) to give the titled compound (205 mg) as a pale yellow crystal.
MS(ESI/APCI Dual): 478(M+H)

### (6) Synthesis of tert-butyl 3-{[7-(cyclohexyloxy)-4,5-dihydroimidazo[1,5-a]quinolin-3-yl]methyl}-2-oxopiperidine-1-carboxylate

Using tert-butyl (3E)-3-{[7-(cyclohexyloxy)-4,5-dihydroimidazo[1,5-a]quinolin-3-yl]methyliden}-2-oxopiperidine-1-carboxylate (205 mg), reaction and purification were performed by the same procedures as in Example 1(5) to give the titled compound (188 mg) as a pale yellow oil.
MS(ESI/APCI Dual): 480(M+H)

### (7) Synthesis of 5-[(tert-butoxycarbonyl)amino]-2-{[7-(cyclohexyloxy)-4,5-dihydroimidazo[1,5-a]quinolin-3-yl]methyl}pentanoic acid

Using tert-butyl 3-{[7-(cyclohexyloxy)-4,5-dihydroimidazo[1,5-a]quinolin-3-yl]methyl}-2-oxopiperidine-1-carboxylate (188 mg), reaction was performed by the same procedure as in Example 14(7) and recrystallization with n-hexane/ethyl acetate gave the titled compound (155 mg) as a colorless powder.
MS(ESI/APCI Dual): 498(M+H)

### (8) Synthesis of 5-amino-2-{[7-(cyclohexyloxy)-4,5-dihydroimidazo[1,5-a]quinolin-3-yl]methyl}pentanoic acid

Using 5-[(tert-butoxycarbonyl)amino]-2-{[7-(cyclohexyloxy)-4,5-dihydroimidazo[1,5-a]quinolin-3-yl]methyl}pentanoic acid (155 mg), reaction and purification were performed by the same procedures as in Example 14(8) to give the titled compound (compound 16, 96 mg) as a pale yellow powder.
MS(ESI/APCI Dual: 398(M+H)
¹H NMR (600 MHz, METHANOL-d3) δ ppm 1.22 - 2.03 (m, 14 H), 2.47 - 2.67 (m, 2 H), 2.74 - 2.97 (m, 7 H), 4.19 - 4.39 (m, 1 H), 6.84 (dd, J=8.7, 2.3 Hz, 1 H), 6.88 (d, J=2.3 Hz, 1 H), 7.46 (d, J=8.7 Hz, 1 H), 8.07 (s, 1 H)

### Example 17

### Synthesis of 5-amino-2-[(7-propyl-4,5-dihydroimidazo[1,5-a]quinolin-3-yl)methyl]pentanoic acid dihydrochloride

### (1) Synthesis of ethyl 7-bromo-4,5-dihydroimidazo[1,5-a]quinoline-3-carboxylate

Using 6-bromo-3,4-dihydroquinolin-2(1H)-one (3.00 g), reaction and purification were performed by the same procedures as in Example 1(1) to give the titled compound (2.10 g) as a pale yellow powder.
MS(ESI/APCI Dual): 321(M+H)

### (2) Synthesis of ethyl 7-[(1E)-1-propen-1-yl]-4,5-dihydroimidazo[1,5-a]quinoline-3-carboxylate

To a suspension of ethyl 7-bromo-4,5-dihydroimidazo[1,5-a]quinoline-3-carboxylate (1.76 g) in toluene (54.8 ml), (1E)-1-propen-1-yl-boronic acid (2.35 g), tri-tert-butylphosphine (1.66 g), palladium acetate (615 mg) and an aqueous solution of 2 M potassium carbonate (11.0 ml) were added and the mixture was stirred for 2 hours at 144 °C. To the reaction system, water was added and extraction was conducted with ethyl acetate. After drying over anhydrous sodium sulfate, the desiccant was filtered off and the solvent was removed in vacuo. The residue was purified by silica gel column chromatography (eluent: n-hexane/ethyl acetate = 1:1) and recrystallization with a n-hexane/ethyl acetate mixture in solution gave a mixture of the titled compound and ethyl 7-(1-propen-2-yl)-4,5-dihydroimidazo[1,5-a]quinoline-3-carboxylate (453 mg, 5:3) as a pale yellow powder.
MS(ESI/APCI Dual): 283(M+H)
¹H NMR (300 MHz, CHLOROFORM-d) δ ppm 1.36 - 1.49 (m, 3 H), 1.87 - 1.96 (m, 3 H), 2.84 - 3.03 (m, 2 H), 3.27 - 3.44 (m, 2 H), 4.33 - 4.50 (m, 2 H), 6.20 - 6.42 (m, 2 H), 7.25 - 7.50 (m, 3 H), 7.99 (s, 1 H)

### (3) Synthesis of {7-[(1E)-1-propen-1-yl]-4,5-dihydroimidazo[1,5-a]quinolin-3-yl}methanol

To a solution of ethyl 7-[(1E)-1-propen-1-yl]-4,5-dihydroimidazo[1,5-a]quinoline-3-carboxylate and ethyl 7-(1-propen-2-yl)-4,5-dihydroimidazo[1,5-a]quinoline-3-carboxylate in admixture (453 mg, 5:3) in tetrahydrofuran (16.0 ml), lithium aluminum hydride (73 mg) was added under cooling with ice and the mixture was stirred for an hour at the same temperature. To the reaction system, ethyl acetate and an aqueous solution of 10% sodium potassium tartarate were added and the mixture was stirred for 3 days at room temperature; extraction was then conducted with ethyl acetate. After drying over anhydrous sodium sulfate, the desiccant was filtered off and the solvent was removed in vacuo to give a mixture of the titled compound and [7-(1-propen-2-yl)-4,5-dihydroimidazo[1,5-a]quinolin-3-yl]methanol (423 mg, 5:3) as a pale yellow oil. MS(ESI/APCI Dual): 241(M+H)
¹H NMR (300 MHz, CHLOROFORM-d) δ ppm 1.87 - 1.94 (m, 3 H), 2.80 - 3.09 (m, 4 H), 4.63 (s, 2 H), 6.16 - 6.44 (m, 2 H), 7.20 - 7.47 (m, 3 H), 8.00 (s, 1 H)

### (4) Synthesis of 7-[(1E)-1-propen-1-yl]-4,5-dihydroimidazo[1,5-a]quinoline-3-carbaldehyde

To a solution of {7-[(1E)-1-propen-1-yl]-4,5-dihydroimidazo[1,5-a]quinolin-3-yl}methanol and [7-(1-propen-2-yl)-4,5-dihydroimidazo[1,5-a]quinolin-3-yl]methanol in admixture (423 mg, 5:3) in chloroform (8.8 ml), manganese dioxide (1.53 mg) was added and the mixture was stirred for 15 hours at room temperature. The reaction system was filtered through Celite and the solvent was removed in vacuo. The residue was purified by silica gel column chromatography (eluent: n-hexane/ethyl acetate = 1:1) and recrystallization with a n-hexane/ethyl acetate mixture in solution gave the titled compound (110 mg) as a pale yellow crystal. MS(ESI/APCI Dual): 239(M+H)
¹H NMR (300 MHz, CHLOROFORM-d) δ ppm 1.91 (dd, J=6.1, 1.2 Hz, 3 H), 2.88 - 3.01 (m, 2 H), 3.28 - 3.41 (m, 2 H), 6.20 - 6.47 (m, 2 H), 7.28 - 7.43 (m, 3 H), 8.03 (s, 1 H), 10.00 (s, 1 H)

### (5) Synthesis of tert-butyl (3E)-2-oxo-3-({7-[(1E)-1-propen-1-yl]-4,5-dihydroimidazo[1,5-a]quinolin-3-yl}methyliden)piperidine-1-carboxylate

Using 7-[(1E)-1-propen-1-yl]-4,5-dihydroimidazo[1,5-a]quinoline-3-carbaldehyde (110 mg), reaction and purification were performed by the same procedures as in Example 1(4) to give the titled compound (130 mg) as a pale yellow crystal. MS(ESI/APCI Dual): 420(M+H)

### (6) Synthesis of tert-butyl 2-oxo-3-[(7-propyl-4,5-dihydroimidazo[1,5-a]quinolin-3-yl)methyl]piperidine-1-carboxylate

To a solution of tert-butyl (3E)-2-oxo-3-({7-[(1E)-1-propen-1-yl]-4,5-dihydroimidazo[1,5-a]quinolin-3-yl}methyliden)piperidine-1-carboxylate (130 mg) in methanol/tetrahydrofuran (1:1, 3.1 ml), 10% palladium-activated carbon (26 mg) was added and the mixture was stirred under hydroen purge for 3 hours at 60 °C. The reaction mixture was filtered through Celite and the solvent was removed in vacuo. The resulting residue was purified by NH silica gel column chromatography (eluent: ethyl acetate) to give the titled compound (131 mg) as a pale yellow powder.
MS(ESI/APCI Dual): 424(M+H)
¹H NMR (300 MHz, CHLOROFORM-d) δ ppm 0.95 (t, J=7.3 Hz, 3 H), 1.52 (s, 9 H), 1.55 - 1.94 (m, 5 H), 1.94 - 2.13 (m, 1 H), 2.50 - 2.73 (m, 3 H), 2.76 - 2.98 (m, 5 H), 3.14 - 3.29 (m, 1 H), 3.49 - 3.64 (m, 1 H), 3.72 - 3.88 (m, 1 H), 7.05 - 7.15 (m, 2 H), 7.27 - 7.37 (m, 1 H), 7.91 (s, 1 H)

### (7) Synthesis of 5-[(tert-butoxycarbonyl)amino]-2-[(7-propyl-4,5-dihydroimidazo[1,5-a]quinolin-3-yl)methyl]pentanoic acid

Using tert-butyl 2-oxo-3-[(7-propyl-4,5-dihydroimidazo[1,5-a]quinolin-3-yl)methyl]piperidine-1-carboxylate (131 mg), reaction was performed by the same procedure as in Example 14(7) to give the titled compound (130 mg) as a pale yellow powder.
MS(ESI/APCI Dual): 442(M+H)

### (8) Synthesis of 5-amino-2-[(7-propyl-4,5-dihydroimidazo[1,5-a]quinolin-3-yl)methyl]pentanoic acid dihydrochloride

Using 5-[(tert-butoxycarbonyl)amino]-2-[(7-propyl-4,5-dihydroimidazo[1,5-a]quinolin-3-yl)methyl]pentanoic acid (130 mg), reaction and purification were performed by the same procedures as in Exmple 15(4) to give the titled compound (compound 17, 75 mg) as a pale yellow powder.
MS(ESI/APCI Dual): 342(M+H)
¹H NMR (600 MHz, DEUTERIUM OXIDE) δ ppm 0.92 (t, J=7.3 Hz, 3 H), 1.60 (tq, J=7.6, 7.3 Hz, 2 H), 1.71 - 1.89 (m, 4 H), 2.56 (t, J=7.6 Hz, 2 H), 2.81 - 2.98 (m, 5 H), 2.98 - 3.19 (m, 4 H), 7.23 (d, J=8.3 Hz, 1 H), 7.27 (s, 1 H), 7.50 (d, J=8.3 Hz, 1 H), 9.16 (s, 1 H)

### Example 18

### Synthesis of 5-amino-2-(4,5-dihydroimidazo[1,5-a]quinoxalin-3-ylmethyl)pentanoic acid

### (1) Synthesis of ethyl 4,5-dihydroimidazo[1,5-a]quinoxaline-3-carboxylate

To a solution of 3,4-dihydroquinoxalin-2(1H)-one (1.00 g) in tetrahydrofuran (10 ml), potassium tert-butoxide (841 mg) was added under cooling with ice and the mixture was stirred for 30 minutes at room temperature. The reaction mixture was cooled to -78 °C and after adding diethyl chlorophosphate (1.29 g) dropwise, the mixture was stirred for 15 minutes at the same temperature, then for an additional 15 minutes at room temperature. The reaction mixture was cooled to -78 °C and after adding ethyl isocyanoacetate (0.82 ml) and potassium tert-butoxide (841 mg), the mixture was stirred for 1.5 hours at the same temperature. To the reaction mixture, a saturated aqueous solution of ammonium chloride (10 ml), water (30 ml) and ethyl acetate (50 ml) were added and the mixture was stirred for 30 minutes at room temperature. The compound insoluble in both layers was recovered by filtration. After the filtrate was separated into an aqueous layer and an organic layer, the latter was washed with brine (50 ml) and dried over anhydrous magnesium sulfate. The desiccant was filtered off and after removing the solvent in vacuo, the resulting residue was recrystallized from n-hexane/ethyl acetate. The crystal was combined with the previously recovered compound to give the titled compound (403 mg) as a brown powder.
MS(ESI/APCI Dual): 244(M+H)
¹H NMR (300 MHz, CHLOROFORM-D) δ ppm 1.42 (t, J=7.1 Hz, 3 H), 4.40 (q, J=7.1 Hz, 2 H), 4.83 (s, 2 H), 6.80 (dd, J=7.8, 1.3 Hz, 1 H), 6.85 (td, J=7.8, 1.3 Hz, 1 H), 7.11 (td, J=7.8, 1.3 Hz, 1 H), 7.39 (dd, J=7.8, 1.3 Hz, 1 H), 7.98 (s, 1 H)

### (2) Synthesis of 5-tert-butyl 3-ethyl imidazo[1,5-a]quinoxaline-3,5(4H)-dicarboxylate

To a suspension of ethyl 4,5-dihydroimidazo[1,5-a]quinoxaline-3-carboxylate (400 mg) in tetrahydrofuran (20 ml), di-tert-butyl dicarbonate (395 mg), 4-dimethylaminopyridine (15 mg) and triethylamine (0.25 ml) were added and the mixture was stirred for 3.5 days at room temperature and for 2.5 hours at 60 °C. Then, di-tert-butyl dicarbonate (395 mg) and triethylamine (0.25 ml) were added and the mixture was stirred for 14 hours at 60 °C. Sodium hydride (60%, 35 mg) was added and the mixture was stirred for 2 hours at 60 °C; thereafter, di-tert-butyl dicarbonate (790 mg) was added and the mixture was stirred for 2 hours at 60 °C and for 13 hours at room temperature. The reaction mixture was left to cool to room temperature and water (20 ml) was added; thereafter, brine (30 ml) and ethyl acetate (100 ml) were added for extraction. The organic layer was washed with brine (50 ml) and dried over anhydrous magnesium sulfate. The desiccant was filtered off and the solvent was removed in vacuo; thereafter, the resulting residue was purified by silica gel column chromatography (eluent: n-hexane/ethyl acetate = 1:1 to 1:4) to give the titled compound (449 mg) as a pale brown powder.
MS(ESI/APCI Dual): 344(M+H)
¹H NMR (200 MHz, CHLOROFORM-D) δ ppm 1.44 (t, J=7.0, 3 H), 1.53 (s, 9 H), 4.42 (q, J=7.0, 2 H), 5.21 (s, 2 H), 7.21 - 7.37 (m, 2 H). 7.48 - 7.55 (m, 1 H), 7.73 - 7.81 (m, 1 H), 8.02 (s, 1 H)

### (3) Synthesis of tert-butyl 3-formylimidazo[1,5-a]quinoxaline-5(4H)-carboxylate

A solution of 5-tert-butyl 3-ethyl imidazo[1,5-a]quinoxaline-3,5(4H)-dicarboxylate (341 mg) in tetrahydrofuran (10 ml) was cooled to -78 °C and after adding diisobutylaluminum hydride (4.0 ml as 0.99 M solution in toluene) dropwise, and the mixture was stirred for an hour at the same temperature. Methanol (2.0 ml) was added dropwise and, thereafter, a saturated aqueous solution of ammonium chloride (20 ml) and ethyl acetate (20 ml) were added and the mixture waas stirred for 30 minutes at room temperature. The reaction mixture was filtered through Celite and water (20 ml) and ethyl acetate (50 ml) were added to the filtrate, whereupon it separated into an aqueous layer and an organic layer. After washing the organic layer with brine (30 ml), silica gel was added to the organic layer and the solvent was removed in vacuo. The resulting residue was purified by silica gel column chromatography (eluent: n-hexane/ethyl acetate = 1:1 to 1:4) to give the titled compound (270 mg) as a colorless powder.
MS(ESI/APCI Dual): 300(M+H)
¹H NMR (300 MHz, CHLOROFORM-d) δ ppm 1.53 (s, 9 H), 5.21 (s, 2 H), 7.24 - 7.38 (m, 2 H), 7.49 - 7.55 (m, 1 H), 7.79 (dd, J=8.2, 1.2 Hz, 1 H), 8.06 (s, 1 H), 10.01 (s, 1 H)

### (4) Synthesis of tert-butyl 3-{(E)-[1-(tert-butoxycarbonyl)-2-oxopiperidin-3-ylidene]methyl}imidazo[1,5-a]quinoxaline-5(4H)- carboxylate

Using tert-butyl 3-formylimidazo[1,5-a]quinoxaline-5(4H)-dicarboxylate (263 mg), reaction and purification were performed by the same procedures as in Example 1(4) to give the titled compound (187 mg) as a colorless powder.
MS(ESI/APCI Dual): 481(M+H)
¹H NMR (300 MHz, CHLOROFORM-D) δ ppm 1.52 (s, 9 H), 1.57 (s, 9 H), 1.87 - 2.00 (m, 2 H), 3.20 - 3.30 (m, 2 H), 3.70 - 3.82 (m, 2 H), 4.96 (s, 2 H), 7.19 - 7.34 (m, 2 H), 7.46 - 7.54 (m, 1 H), 7.64 - 7.70 (m, 1 H), 7.70 - 7.79 (m, 1 H), 8.06 (s, 1 H)

### (5) Synthesis of tert-butyl 3-{[1-(tert-butoxycarbonyl)-2-oxopiperidin-3-yl]methyl}imidazo[1,5-a]quinoxaline-5(4H)- carboxylate

Using tert-butyl 3-{(E)-[1-(tert-butoxycarbonyl)-2-oxopiperidin-3-ylidene]methyl}imidazo[1,5-a]quinoxaline-5(4H)-carboxylate (180 mg), reaction and purification were performed by the same procedures as in Example 1(5) to give the titled compound (171 mg) as a colorless powder.
MS(ESI/APCI Dual): 483(M+H)
¹H NMR (300 MHz, CHLOROFORM-d) δ ppm 1.51 (s, 9 H), 1.52 (s, 9 H), 1.53 - 1.90 (m, 3 H), 1.96 - 2.08 (m, 1 H), 2.65 - 2.75 (m, 1 H), 2.81 - 2.94 (m, 1 H), 3.16 - 3.26 (m,1 H), 3.50 - 3.61 (m, 1 H), 3.76 - 3.87 (m, 1 H), 4.71 - 4.96 (m, 2 H), 7.17 - 7.27 (m, 2 H), 7.42 - 7.49 (m, 1 H), 7.66 - 7.74 (m, 1 H), 7.92 (s, 1 H)

### (6) Synthesis of 5-amino-2-(4,5-dihydroimidazo[1,5-a]quinoxalin-3-ylmethyl)pentanoic acid

Using tert-butyl 3-{[1-(tert-butoxycarbonyl)-2-oxopiperidin-3-yl]methyl}imidazo[1,5-a]quinoxaline-5(4H)-carboxylate (164 mg), reaction and purification were performed by the same procedures as in Example 1(6) and two subsequent recrystallizations from water/dioxane give the titled compound (compound 18, 56 mg) as a colorless powder.
MS(ESI/APCI Dual): 301(M+H)
¹H NMR (300 MHz, DEUTERIUM OXIDE) δ ppm 1.42 - 1.78 (m, 4 H), 2.47 - 2.68 (m, 2 H), 2.71 - 2.86 (m, 1 H), 2.90 - 3.09 (m, 2 H), 4.25 (s, 2 H), 6.90 - 7.07 (m, 2 H), 7.11 - 7.22 (m, 1 H), 7.53 (d, J=7.9 Hz, 1H), 8.15 (s, 1 H)

### Example 19

### Synthesis of 5-amino-2-(4,5-dihydroimidazo[1,5-a][1,5]naphthyridin-3-ylmethyl)pentanoic acid

### (1) Synthesis of ethyl 4,5-dihydroimidazo[1,5-a][1,5]naphthyridine-3-carboxylate

Using 3,4-dihydro-1,5-naphthyridin-2(1H)-one (1.96 g), reaction and purification were performed by the same procedures as in Example 1(1) to give the titled compound (1.03 g) as a brown oil.
MS(ESI/APCI Dual): 244(M+H)

### (2) Synthesis of 4,5-dihydroimidazo[1,5-a][2,5]naphthyridin-3-ylmethanol

Using ethyl 4,5-dihydroimidazo[1,5-a][1,5]naphthyridine-3-carboxylate (1.03 g), reaction was performed by the same procedure as in Example 1(2) to give the titled compound (610 mg) as a brown oil.
MS(ESI/APCI Dual):202(M+H)

### (3) Synthesis of 4,5-dihydroimidazo[1,5-a][1,5]naphthyridin-3-carbaldehyde

Using 4,5-dihydroimidazo[1,5-a][1,5]naphthyridine-3-ylmethanol (610 mg), reaction and purification were performed by the same procedures as in Example 1(3) to give the titled compound (312 mg) as a pale yellow powder.
MS(ESI/APCI Dual): 200(M+H)

### (4) Synthesis of tert-butyl (3E)-3-(4,5-dihydroimidzazo[1,5-a][1,5]naphthyridin-3-ylmethyliden)-2-oxopiperidine-1-carboxylate

Using 4,5-dihydroimidazo[1,5-a][1,5]naphthyridine-3-carbaldehyde (312 mg), reaction and purification were performed by the same procedures as in Exmple 1(4) to give the titled compound (320 mg) as a yellow powder.
MS(ESI/APCI Dual): 381(M+H)

### (5) Synthesis of tert-butyl 3-(4,5-dihydroimidzazo[1,5-a][1,5]naphthyridin-3-ylmethyl)-2-oxopiperidine-1-carboxylate

Using tert-butyl (3E)-3-(4,5-dihydroimidzazo[1,5-a][1,5]naphthyridin-3-ylmethyliden)-2-oxopiperidine-1-carboxylate (320 mg), reaction and purification were performed by the same procedures as in Example 1(5) to give the titled compound (280 mg) as a colorless oil.
MS(ESI/APCI Dual): 383(M+H)

### (6) Synthesis of 5-[(tert-butoxycarbonyl)amino]-2-(4,5-dihydroimidzazo[1,5-a][1,5]naphthyridin-3-ylmethyl)pentanoic acid

Using tert-butyl 3-(4,5-dihydroimidzazo[1,5-a][1,5]naphthyridin-3-ylmethyl)-2-oxopiperidine-1-carboxylate (280 mg), reaction was performed by the same procedure as in Example 14(7) to give the titled compound (253 mg) as a pale yellow powder.
MS(ESI/APCI Dual): 401(M+H)

### (7) Synthesis of 5-amino-2-(4,5-dihydroimidazo[1,5-a][1,5]naphthyridin-3-ylmethyl)pentanoic acid

Using 5-[(tert-butoxycarbonyl)amino]-2-(4,5-dihydroimidzazo[1,5-a][1,5]naphthyridin-3-ylmethyl)pentanoic acid (253 mg), reaction and purification were performed by the same procedures as in Example 14(8) to give the titled compound (compound 19, 150 mg) as a pale yellow powder.
MS(ESI/APCI Dual): 301(M+H)
¹H NMR (600 MHz, DEUTERIUM OXIDE) δ ppm 1.48 - 1.65 (m, 2 H), 1.65-1.75 (m, 2 H), 2.49 - 2.61 (m, 2 H), 2.71 - 2.96 (m, 5 H), 2.96 - 3.09 (m, 2 H), 7.28 (dd, J=8.0, 4.8 Hz, 1 H), 7.76 (d, J=8.0 Hz, 1 H), 8.01 (s, 1 H), 8.18 (d, J=4.8 Hz, 1 H)

### Example 20

### Synthesis of 2-(4,5-dihydroimidazo[1,5-a]quinolin-3-ylmethyl)-5-(methylamino)pentanoic acid

### (1) Synthesis of methyl 5-[(tert-butoxycarbonyl)amino]-2-(4,5-dihydroimidazo[1,5-a]quinolin-3-ylmethyl)pentanoate

To a solution of 5-[(t-butoxycarbonyl)amino]-2-(4,5-dihydroimidazo[1,5-a]quinolin-3-ylmethyl)pentanoic acid (511 mg) in chloroform (12.5 ml), methanol (0.080 ml), 4-dimethylaminopyridine (16 mg) and N-[3-(dimethylamino)propyl]-N'-ethylcarbodiimide hydrochloride (368 mg) were added and the mixture was stirred for 2 days at room temperature. To the reaction system, a saturated aqueous solution of sodium hydrogencarbonate (20 ml) was added and extraction was conducted twice with ethyl acetate (50 ml). The organic layer was washed with brine (40 ml) and after drying over anhydrous magnesium sulfate, the desiccant was filtered off and the solvent was removed in vacuo. The residue was purified by silica gel collumn chromatography (eluent: n-hexane/ethyl acetate = 1:1 to 0:1) to give the titled compound (425 mg) as a colorless oil.
MS(ESI/APCI Dual): 414(M+H)
¹H NMR (300 MHz, CHLOROFORM-d) δ ppm 1.42 (s, 9 H), 1.45 - 1.76 (m, 4 H), 2.65 - 2.78 (m, 1 H), 2.80 - 2.96 (m, 6 H), 3.05 - 3.19 (m, 2 H), 3.63 (s, 3 H), 4.70 - 4.81 (m, 1 H), 7.12 - 7.20 (m, 1 H), 7.25 - 7.33 (m, 2 H), 7.37 - 7.42 (m, 1 H), 7.94 (s, 1 H)

### (2) Synthesis of methyl 5-[(t-butoxycarbonyl)(methyl)amino]-2-(4,5-dihydroimidazo[1,5-a]quinolin-3-ylmethyl)pentanoate

To a solution of methyl 5-[(t-butoxycarbonyl)amino]-2-(4,5-dihydroimidazo[1,5-a]quinolin-3-ylmethyl)pentanoate (880 mg) in N,N-dimethylformamide (12 ml), sodium hydride (60%, 128 mg) was added in small portions under cooling with ice and the mixture was then stirred for 20 minutes. Methyl iodide (0.20 ml) was subsequently added and the mixture was stirred for 15 hours at room temperature. To the reaction mixture, water (50 ml) was added and extraction was conducted with ethyl acetate (80 ml). After washing the orgnaic layer with water (50 ml) and brine (50 ml), NH silica gel was added and the solvent was removed in vacuo. The residue was purified by NH silica gel column chromatography (eluent: n-hexane/ethyl acetate = 1:1 to 0:1) and by another run of NH silica gel column chromatography (eluent: n-hexane/ethyl acetate = 1:1 to 1:4) to give the titled compound (263 mg) as a colorless oil.
MS(ESI/APCI Dual): 428(M+H)
¹H NMR (300 MHz, CHLOROFORM-d) δ ppm 1.43 (s, 9 H), 1.48 - 1.65 (m, 4 H), 2.63 - 2.76 (m, 1 H), 2.81 (s, 3 H), 2.83 - 2.97 (m, 6 H), 3.14 - 3.23 (m, 2 H), 3.62 (s, 3 H), 7.12 - 7.20 (m, 1 H), 7.26 - 7.33 (m, 2 H), 7.37 - 7.44 (m, 1 H), 7.94 (s, 1 H)

### (3) Synthesis of 2-(4,5-dihydroimidazo[1,5-a]quinolin-3-ylmethyl)-5-(methylamino)pentanoic acid

To a solution of methyl 5-[(tert-butoxycarbonyl)(methyl)amino]-2-(4,5-dihydroimidazo[1,5-a]quinolin-3-ylmethyl)pentanoate (254 mg) in tetrahydrofuran (6 ml), an aqueous solution of 0.9 M lithium hydroxide (2.0 ml) was added and the mixture was stirred at room temperature for 1.5 hours. To the reaction mixture, water (40 ml) and brine (5 ml) were added and extraction was conducted with diethyl ether (40 ml). To the aqueous layer, a saturated aqueous solution of citric acid (5 ml) was added and extraction was conducted twice with ethyl acetate (70 ml, 50 ml). To the solution of the starting material in a methanol/tetrahydrofuran mixture as recovered from the organic layer (diethyl ether), an aqueous solution of 2 M sodium hydroxide (1.0 ml) was added and the mixture was stirred for 14 hours at room temperature and for an hour at 60 °C. The solvent was removed in vacuo and after adding water (40 ml), extraction was conducted twice with diethyl ether (30 ml). To the aqueous layer, a sdaturated aqueous solution of citric acid (2 ml) was added and extraction was conducted twice with chloroform (60 ml). After drying the organic layer (ethyl acetate and chloroform) with anhydrous magnesium sulfate, the desiccant was filtered off and the solvent was removed in vacuo. To the resulting residue, a solution of 2 M hydrochloric acid in ethyl acetate (20 ml) was added and the mixture was stirred for 15 hours at room temperature. The solvent was removed in vacuo and after adding water (20 ml) and Amberlite IRA-67 (2.2 g), the mixture was stirred for 30 minutes at room temperature. The reaction system was filtered and the filtrate was concentrated in vacuo to give the titled compound (compound 20, 133 mg) as a brown amorphous mass.
MS(ESI/APCI Dual): 314(M+H)
¹¹H NMR (600 MHz, DEUTERIUM OXIDE) δ ppm 1.48 - 1.56 (m, 1 H), 1.56 - 1.63 (m, 1 H), 1.65 - 1.72 (m, 2 H), 2.53 - 2.59 (m, 1 H), 2.62 - 2.67 (m, 1 H), 2.70 (s, 3 H), 2.79 - 2.90 (m, 5 H), 2.98 - 3.07 (m, 2 H), 7.25 - 7.29 (m, 1 H), 7.35 - 7.41 (m, 2 H), 7.53 - 7.56 (m, 1 H), 8.29 (s, 1 H)

### Example 21

### Synthesis of 5-amino-2-(5,10-dihydroimidazo[1,5-b]isoquinolin-1-ylmethyl)pentanoic acid

### (1) Synthesis of methyl 4-formyl-1-trityl-1H-imidazole-5-carboxylate

A solution of dimethyl 1-trityl-1H-imidazole-4,5-dicarboxylate (500 mg) in chloroform (11.7 ml) was cooled to - 60 °C and diisobutylaluminum hydride (2.36 ml as 0.99 M solution in toluene) was added dropwise, and the mixture was stirred for an hour at the same temperature. After adding methanol and an aqueous solution of 15% citric acid dropwise, extraction was conducted with chloroform. After drying the organic layer over anhydrous sodium sulfate, the desiccant was filtered off and the solvent was removed in vacuo. The resulting residue was purified by silica gel column chromatography (eluent: n-hexane/ethyl acetate = 2:1 to 1:2) to give the titled compound (350 mg) as a colorless powder.
MS(ESI/APCI Dual): 419(M+Na)
¹H NMR (300 MHz, CHLOROFORM-d) δ ppm 3.24 (s, 3 H), 7.08 - 7.24 (m, 6 H), 7.28 - 7.40 (m, 9 H), 7.62 (d, J=0.5 Hz, 1 H), 10.10 (d, J=0.5 Hz, 1 H)

### (1) Synthesis of methyl 4-{hydroxy[2-(hydroxymethyl)phenyl]methyl}-1-trityl-1H-imidazole-5-carboxylate

A solution of 2-bromobenzyl alcohol (248 mg) in tetrahydrofuran (4.4 ml) was cooled to -30 °C and dibutyl magnesium (0.662 ml as 1.0 M solution in heptane) and n-butyllithium (0.518 ml as 2.73 M solution in hexane) were sequentially added dropwise, and the mixture was stirred for an hour at 0 °C. A solution of methyl 4-formyl-1-trityl-1H-imidazole-5-carboxylate (350 ml) in tetrahydrofuran was added dropwise and the mixture was stirred for an ahour at the same temperature. After adding a saturated aqueous solution of ammonium chloride to quench the reaction, extraction was conducted with ethyl acetate. After drying the organic layer over anhydrous sodium sulfate, the desiccant was filtered off and the solvent was removed in vacuo. The resulting residue was purified by silica gel column chromatography (eluent: n-hexane/ethyl acetate = 2:1 to 1:3) to give the titled compound (210 mg) as a colorless powder.
MS(ESI/APCI Dual): 527(M+Na)
¹H NMR (300 MHz, CHLOROFORM-d) δ ppm 3.00 (s, 3 H), 4.71 - 4.90 (m, 2 H), 6.41 (s, 1 H), 7.01 - 7.43 (m, 20 H)

### (3) Synthesis of methyl 10-methoxy-5,10-dihydroimidazo[1,5-b]isoquinoline-1-carboxylate

To a solution of methyl 4-{hydroxy[2-(hydroxymethyl)phenyl]methyl}-1-trityl-1H-imidazole-5-carboxylate (500 mg) in chloroform (5.0 ml), carbon tetrabromide (362 mg) and triphenylphosphine (286 mg) were added under cooling with ice and the mixture was stirred for 10 minutes at the same temperature and for 2 hours at room temperature. The reaction mixture was concentrated in vacuo. A solution of the resulting residue in methanol (5.0 ml) was stirred for 5 hours at 60 °C. The reaction mixture was concentrated in vacuo and an aqueous solution of sodium hydrogencarbonate was added to the resulting residue; extraction was then conducted three times with chloroform. After drying the organic layer over anhydrous sodium sulfate, the desiccant was filtered off and the solvent was removed in vacuo. The resulting residue was purified by silica gel column chromatography (eluent: ethyl acetate/chloroform/methanol = 1:0:0 to 0:20:1) to give the titled compound (117 mg) as a colorless oil.
MS(ESI/APCI Dual): 259(M+H)
¹H NMR (300 MHz, CHLOROFORM-d) δ ppm 3.39 (s, 3 H), 3.95 (s, 3 H), 5.05 - 5.33 (m, 2 H), 5.97 (s, 1 H), 7.30 - 7.46 (m, 3 H), 7.50 - 7.58 (m, 1 H), 7.66 (s, 1 H)

### (4) Synthesis of methyl 5,10-dihydroimidazo[1,5-b]isoquinoline-1-carboxylate

To a solution of methyl 10-methoxy-5,10-dihydroimidazo[1,5-b]isoquinoline-1-carboxylate (117 mg) in methanol (4.5 ml), 10% palladium-activated carbon (59 mg) and conc. sulfuric acid (one drop) were added and the mixure was stirred under hydrogen purge for an hour at room temperature and for 5 hours at 60 °C. The reaction mixture was filtered through Celite and the solvent was removed in vacuo. To the resulting residue, an aqueous solution of sodium hydrogencarbonate was added and extraction was conducted three times with chloroform. After drying the organic layer over anhydrous sodium sulfate, the desiccant was filtered off and the solvent was removed in vacuo to give the titled compound (84 mg) as a colorless powder.
MS(ESI/APCI Dual): 229(M+H)
¹H NMR (300 MHz, CHLOROFORM-d) δ ppm 3.94 (s, 3 H), 4.42 (s, 2 H), 5.21 (s, 2 H), 7.23 - 7.44 (m, 4 H), 7.68 (s, 1 H)

### (5) Synthesis of 5,10-dihydroimidazo[1,5-b]isoquinoline-1-carbaldehyde

A solution of methyl 5,10-dihydroimidazo[1,5-b]isoquinoline-1-carboxylate (1.00 g) in tetrahydrofuran (43.8 ml) was cooled to -78 °C and diisobutylaluminum hydride (17.7 ml as 0.99 M solution in toluene) was added dropwise and the mixture was stirred for an hour at the same temperature. After quenching the reaction with methanol, an aqueous solution of 15% citric acid was added and the mixture was stirred for 30 minutes at room temperature. After neutralizing with an aqueous solution of 10% sodium hydroxide, extraction was conduted three times with chloroform. After drying the organic layer with anhydrous sodium sulfate, the desiccant was filtered off and the solvent was removed in vacuo. The resulting residue was purified by silica gel column chromatography (eluent: chloroform/methanol = 10:1) to give the titled compound (566 mg) as a pale yellow powder.
¹H NMR (300 MHz, CHLOROFORM-d) δ ppm. 4.39 - 4.49 (m, 2 H), 5.17 - 5.25 (m, 2 H), 7.22 - 7.46 (m, 4 H), 7.65 (s, 1 H), 9.99 (s, 1 H)

### (6) Synthesis of tert-butyl (3E)-3-(5,10-dihydroimidzazo[1,5-b]isoquinolin-1-ylmethyliden)-2-oxopiperidine-1-carboxylate

Using 5,10-dihydroimidazo[1,5-b]isoquinoline-1-carbaldehyde (566 mg), reaction and purification were performed by the same procedures as in Example 1(4) to give the titled compound (360 mg) as a pale yellow powder. MS(ESI/APCI Dual): 380(M+H)

### (7) Synthesis of tert-butyl 3-(5,10-dihydroimidzazo[1,5-b]isoquinolin-1-ylmethyl)-2-oxopiperidine-1-carboxylate

Using tert-butyl (3E)-3-(5,10-dihydroimidzazo[1,5-b]isoquinolin-1-ylmethyliden)-2-oxopiperidine-1-carboxylate (360 mg), reaction and purification were performed by the same procedures as in Example 1(5) to give the titled compound (345 mg) as a pale yellow oil.
MS(ESI/APCI Dual): 382(M+H)

### (8) Synthesis of 5-[(tert-butoxycarbonyl)amino]-2-(5,10-dihydroimidzazo[1,5-b]isoquinolin-1-ylmethyl)pentanoic acid

Using tert-butyl 3-(5,10-dihydroimidzazo[1,5-b]isoquinolin-1-ylmethyl)-2-oxopiperidine-1-carboxylate (345 mg), reaction was performed by the same procedure as in Example 14(7) to give the titled compound (332 mg) as a pale yellow powder.
MS(ESI/APCI Dual): 400(M+H)

### (9) Synthesis of 5-amino-2-(5,10-dihydroimidazo[1,5-b]isoquinolin-1-ylmethyl)pentanoic acid

Using 5-[(tert-butoxycarbonyl)amino]-2-(5,10-dihydroimidzazo[1,5-b]isoquinolin-1-ylmethyl)pentanoic acid (332 mg), reaction and purification were performed by the same procedures as in Example 14(8) to give the titled compound (compound 21, 187 mg) as a pale brown powder.
MS(ESI/APCI Dual): 300(M+H)
¹H NMR (600 MHz, DEUTERIUM OXIDE) 5 ppm 1.44 - 1.73 (m, 4 H), 2.51 - 2.63 (m, 2 H), 2.73 - 2.83 (m, 1 H), 2.91 - 3.04 (m, 2 H), 3.70 - 3.82 (m, 2 H), 4.89 (s, 2 H), 7.11 - 7.29 (m, 4 H), 7.70 (s, 1 H)

### Example 22

### Synthesis of 2-(2-aminopyridin-4-yl)-3-(4,5-dihydroimidazo[1,5-a]quinolin-3-yl)propanoic acid dihydrochloride

### (1) Synthesis of N,N-bis(4-methoxybenzyl)-4-methylpyridin-2-amine

To a solution of 2-amino-4-methylpyridine (5.00 g) in N,N-dimethylformamide (154 ml), sodium hydride (60%, 4.62 g) and p-methoxybenzyl chloride (18.2 g) were sequentially added under cooling with ice and the mixture was stirred for 20 hours at room temperature. After adding an aqueous solution of 15% citric acid to quench the reaction, extraction was conducted three times with ethyl acetate. After drying the organic layer over anhydrous sodium sulfate, the desiccant was filtered off and the solvent was removed in vacuo. The resulting residue was purified by silica gel column chromatography (eluent: n-hexane/ethyl acetate = 8:1 to 3:1) to give the titled compound (11.2 g) as a yellow oil. MS(ESI/APCI Dual): 349(M+H)
¹H NMR (300 MHz, CHLOROFORM-d) δ ppm 2.18 (s, 3 H), 3.78 (s, 6 H), 4.68 (s, 4 H), 6.28 - 6.33 (m, 1 H), 6.39 - 6.45 (m, 1 H), 6.80 - 6.86 (m, 4 H), 7.11 - 7.18 (m, 4 H), 8.04 - 8.09 (m, 1 H)

### (2) Synthesis of ethyl {2-[bis(4-methoxybenzyl)amino]pyridin-4-yl}acetate

To a solution of N,N-bis(4-methoxybenzyl)-4-methylpyridin-2-amine (5.00 g) in tetrahydrofuran (14.3 ml), diethyl carbonate (3.39 g), lithium diisopropylamide (57.2 ml as 2 M solution in heptane/tetrahydrofuran/ethylbenzene) were added and the mixture was stirred for an hour at the same temperature. After adding an aqueous solution of 15% citric acid to quench the reaction, extraction was conducted three times with ethyl acetate. After drying the organic layer over anhydrous sodium sulfate, the desiccant was filtered off and the solvent was removed in vacuo. The resulting residue was purified by silica gel column chromatography (eluent: n-hexane/ethyl acetate = 9:1 to 4:1) to give the titled compound (1.25 g) as a yellow oil.
MS(ESI/APCI Dual): 421(M+H)
¹H NMR (300 MHz, CHLOROFORM-d) δ ppm 1.20 (t, J=7.1 Hz, 3 H), 3.43 (s, 2 H), 3.79 (s, 6 H), 4.10 (q, J=7.1 Hz, 2 H), 4.69 (s, 4 H), 6.35 - 6.42 (m, 1 H), 6.48 - 6.57 (m, 1 H), 6.78 - 6.89 (m, 4 H), 7.08 - 7.20 (m, 4 H), 8.10 - 8.18 (m, 1 H)

### (3) Synthesis of ethyl 2-{2-[bis(4-methoxybenzyl)amino]pyridin-4-yl}-3-(4,5-dihydroimidazo[1,5-a]quinolin-3-yl)-3-hydroxypropanoate

To a solution of ethyl {2-[bis(4-methoxybenzyl)amino]pyridin-4-yl}acetate (100 mg) in tetrahydrofuran (1.8 ml), lithium hexamethyldisilazane (0.238 ml as 1 M solution in tetrahydrofuran) was added at -78°C and the mixture was stirred for 30 minutes at the same temperature. A solution of 4,5-dihydroimidazo[1,5-a]quinoline-3-carbaldehyde (36 mg) in tetrahydrofuran was added at -78°C and the mixture was stirred for an hour at the same temperature. To the reaction system, a saturated aqueous solution of ammonium chloride was added and extraction was conducted with ethyl acetate. After drying the organic layer over anhydrous sodium sulfate, the desiccant was filtered off and the solvetns were removd in vacuo. The resulting residue was purified by NH silica gel column chromatography (eluent: n-hexane/ethyl acetate = 1:1 to 0:1) to give the titled compound (37 mg) as a yellow oil.
MS(ESI/APCI Dual): 619(M+H)
¹H NMR (300 MHz, CHLOROFORM-d) δppm 1.16 - 1.32 (m, 3 H), 2.06 - 2.15 (m, 1 H), 2.33 - 2.54 (m, 2 H), 2.60 - 2.71 (m, 1 H), 3.74 - 3.81 (m, 6 H), 3.94 - 4.01 (m, 1 H), 4.07 - 4.33 (m, 2 H), 4.49 - 4.66 (m, 4 H), 4.97 - 5.08 (m, 1 H), 6.31 - 6.37 (m, 1 H), 6.40 - 6.46 (m, 1 H), 6.69 - 6.77 (m, 4 H), 6.99 - 7.08 (m, 4 H), 7.08 - 7.41 (m, 4 H), 7.81 (s, 1 H), 7.94 - 8.01 (m, 1 H)

### (4) Synthesis of ethyl 2-{2-[bis(4-methoxybenzyl)amino]pyridin-4-yl}-3-(tert-butyldimethylsilyloxy)-3-(4,5-dihydroimidazo[1,5-a]quinolin-3-yl)propanoate

To a solution of ethyl 2-{2-[bis(4-methoxybenzyl)amino]pyridin-4-yl}-3-(4,5-dihydroimidazo[1,5-a]quinolin-3-yl)-3-hydroxypropanoate (1.50 g) in chloroform (24.2 ml), imidazole (33 mg) and tert-butyldimethylsilyl chloride (438 mg) were added and the mixture was stirred at room temperature for 20 hours. To the reaction system, water was added and extraction was conducted three times with chloroform. After drying the organic layer over anhydrous sodium sulfate, the desiccant was filtered off and the solvent was removd in vacuo. The residue was purified by silica gel column chromatography (eluent: n-hexane/ethyl acetate = 4:1 to 1:1) to give the titled compound (1.46 g) as a colorless oil.
MS(ESI/APCI Dual): 733(M+H)
¹H NMR (300 MHz, CHLOROFORM-d) δppm 0.12 - 0.25 (m, 6 H), 0.93 - 1.04 (m, 9 H), 1.44 (t, J=7.1 Hz, 3 H), 2.38 - 2.54 (m, 1 H), 2.54 - 2.73 (m, 2 H), 2.74 - 2.93 (m, 1 H), 3.95 (s, 6 H), 4.17 - 4.49 (m, 3 H), 4.62 - 4.86 (m, 4 H), 5.28 (d, J=10.3 Hz, 1 H), 6.56 (s, 1 H), 6.70 (dd, J=5.2, 1.2 Hz, 1 H), 6.86 - 6.96 (m, 4 H), 7.15 - 7.27 (m, 4 H), 7.29 - 7.60 (m, 4 H), 7.94 (s, 1 H), 8.12 (d, J=5.2 Hz, 1 H)

### (5) Synthesis of ethyl (2Z)-2-{2-[bis(4-methoxybenzyl)amino]pyridin-4-yl}-3-(4,5-dihydroimidazo[1,5-a]quinolin-3-yl)-2-propenoate

To a solution of ethyl 2-{2-[bis(4-methoxybenzyl)amino]pyridin-4-yl}-3-(tert-butyldimethylsilyloxy)-3-(4,5-dihydroimidazo[1,5-a]quinolin-3-yl)propanoate (1.46 g) in N,N-dimethylformamide (19.9 ml), diazabicycloundecene (1.21 g) was added and the mixture was stirred for 6 hours at 70 °C. The reaction system was concentrated in vacuo and the residue was purified by silica gel column chromatography (eluent: n-hexane/ethyl acetate = 1:1 to 1:2) to give the titled compound (580 mg) as a pale yellow powder.
MS(ESI/APCI Dual): 601(M+H)
¹H NMR (300 MHz, CHLOROPORM-d) δppm 1.27 (t, J=7.1 Hz, 3 H), 2.40 - 2.56 (m, 2 H), 2.64 - 2.78 (m, 2 H), 3.74 (s, 6 H), 4.23 (q, J=7.1 Hz, 2 H), 4.62 (s, 4 H), 6.45 - 6.50 (m, 1 H), 6.58 (dd, J=5.2, 1.3 Hz, 1 H), 6.68 - 6.78 (m, 4 H), 7.04 - 7.14 (m, 4 H), 7.15 - 7.43 (m, 4 H), 7.77 (s, 1 H), 7.86 (s, 1 H), 8.18 (dd, J=5.2, 0.8 Hz, 1 H)

### (6) Synthesis of ethyl 2-{2-[bis(4-methoxybenzyl)amino]pyridin-4-yl}-3-(4,5-dihydroimidazo[1,5-a]quinolin-3-yl)propanoate

To a solution of ethyl (2Z)-2-{2-[bis(4-methoxybenzyl)amino]pyridin-4-yl}-3-(4,5-dihydroimidazo[1,5-a]quinolin-3-yl)-2-propenoate (580 mg) in ethanol (9.7 ml), 10% palladium-activated carbon (116 mg) was added and the mixture was stirred under hydrogen purge for 3 hours at 60 °C. The reaction system was filtered through Celite and the solvent was removed in vacuo. The resulting residue was purified by silica gel column chromatography (eluent: n-hexane/ethyl acetate = 1:1 to 1:4) to give the titled compound (495 mg) as a colorless oil.
MS(ESI/APCI Dual): 603(M+H)
¹H NMR (300 MHz, CHLOROFORM-d) δ ppm 1.12 (t, J=7.1 Hz, 3 H), 2.37 - 2.54 (m, 1 H), 2.57 - 2.79 (m, 3 H), 2.84 (dd, J=14.3, 7.8 Hz, 1 H), 3.20 (dd, J=14.3, 7.8 Hz, 1 H), 3.77 (s, 6 H), 3.91 (t, J=7.8 Hz, 1 H), 3.95 - 4.18 (m, 2 H), 4.53 - 4.74 (m, 4 H), 6.39 (s, 1 H), 6.55 (dd, J=5.1, 1.2 Hz, 1 H), 6.71 - 6.84 (m, 4 H), 7.03 - 7.20 (m, 4 H), 7.22 - 7.42 (m, 4 H), 7.86 (s, 1 H), 8.09 (d, J=5.1 Hz, 1 H)

### (7) Synthesis of 2-{2-[bis(4-methoxybenzyl)amino]pyridin-4-yl}-3-(4,5-dihydroimidazo[1,5-a]quinolin-3-yl)propanoic acid

To a solution of ethyl 2-(2-[bis(4-methoxybenzyl)amino]pyridin-4-yl}-3-(4,5-dihydroimidazo[1,5-a]quinolin-3-yl)propanoate (270 mg) in methanol (9.0 ml), an aqueous solution of 33% sodium hydroxide (1.5 ml) was added and the mixture was stirred for 15 hours at room temperature. The reaction system was concentrated in vacuo and after adding an aqueous solution of 15% citric acid to the residue to neutralize it, extraction was conducted three times with chloroform. After drying the organic layer with anhydrous sodium sulfate, the desiccant was filtered off and the solvent was removed in vacuo to give the titled compound (241 mg) as a colorless oil.
MS(ESI/APCI Dual): 575(M+H)
¹H NMR (300 MHz, CHLOROFORM-d) δ ppm 2.32 - 2.77 (m, 4 H), 2.83 - 2.99 (m, 1 H), 3.16 - 3.33 (m, 1 H), 3.69 - 3.79 (m, 6 H), 3.88 (t, J=6.8 Hz, 1 H), 4.53 - 4.71 (m, 4 H), 6.48 (s, 1 H), 6.57 (d, J=5.3 Hz, 1 H), 6.70 - 6.79 (m, 4 H), 7.02 - 7.10 (m, 4 H), 7.12 - 7.34 (m, 4 H), 7.86 (s, 1 H), 8.04 (d, J=5.3 Hz, 1 H)

### (8) Synthesis of 2-(2-aminopyridin-4-yl)-3-(4,5-dihydroimidazo[1,5-a]quinolin-3-yl)propanoic acid dihydrochloride

[248] A solution of 2-{2-[bis(4-methoxybenzyl)amino]pyridin-4-yl}-3-(4,5-dihydroimidazo[1,5-a]quinolin-3-yl)propanoic acid (241 mg) in trifluoroacetic acid (4.2 ml) was stirred for 20 hours at room temperature. The reaction system was concentrated in vacuo and after adding water to the residue, extraction was conducted three times with chloroform. The aqueous layer was concentrated in vacuo and after adding water (4.2 ml) and Amberlite IRA-67 (2.41 g) to the residue, the mixture was stirred for 30 minutes at room temperature. The reaction system was filtered and after washing with methanol, the filtrate was concentrated in vacuo. To the resulting residue, an aqoueous solution of 6 M hydrochloric acid was added and the mixture was stirred for 30 minutes at room temperature. The reaction system was concentrated in vacuo to give the titled compound (compound 22, 40 mg) as a pale yellow powder.
MS(ESI/APCI Dual): 335(M+H)
¹H NMR (600 MHz, DEUTERIUM OXIDE) δ ppm 2.79- 3.00 (m, 4 H), 3.28 - 3.34 (m, 1 H), 3.46 - 3.53 (m, 1 H), 4.08 (t, J=7.8 Hz, 1 H), 6.90 (dd, J=6.7, 1.6 Hz, 1 H), 6.95 (d, J=0.9 Hz, 1 H), 7.40 - 7.50 (m, 3 H), 7.65 - 7.70 (m, 1 H), 7.80 (d, J=6.7 Hz, 1 H), 9.21 (s, 1 H)

### Example 23

### Synthesis of 5-(4,5-dihydroimidazo[1,5-a]quinolin-3-yl)-4-(1H-tetrazol-5-yl)pentan-1amine

### (1) Synthesis of tert-butyl {5-[(2-cyanoethyl)amino]-4-(4,5-dihydroimidazo[1,5-a]quinolin-3-ylmethyl)-5-oxopentyl}carbamate

To a solution of 5-[(tert-butoxycarbonyl)amino]-2-(4,5-dihydroimidazo[1,5-a]quinolin-3-ylmethyl)pentanoic acid (400 mg) in chloroform (5.0 ml), 3-aminopropanenitrile (105 mg) and N-[3-(dimethylamino)propyl]-N'-ethylcarbodiimide hydrochloride (290 mg) and 1H-benzotriazol-1-ol hydrate (207 mg) were added and the mixture was stirred for 3 hours at room temperature. To the reaction mixture, ethyl acetate (70 ml) was added and the mixture was washed with water (30 ml) and brine (30 ml) in that order. Silica gel was added to the organic layer and the solvent was concentrated in vacuo. The resulting residue was purified by silica gel column chromatography (eluent: chloroform/methanol = 95:5 to 90:10) to give the titled compound (413 mg) as a colorless amorphous mass.
MS(ESI/APCI Dual): 452(M+H)
¹H NMR (300 MHz, CHLOROFORM-d) δ ppm 1.42 (s, 9 H), 1.47 - 1.59 (m, 3 H), 1.70 - 1.79 (m, 1 H), 2.38 - 2.58 (m, 2 H), 2.63 - 2.97 (m, 7 H), 3.01 - 3.22 (m, 2 H), 3.31 - 3.50 (m, 2 H), 4.60 - 4.73 (m, 1 H), 7.10 - 7.22 (m, 2 H), 7.25 - 7.34 (m, 2 H), 7.39 - 7.45 (m, 1 H), 7.96 (s, 1 H)

### (2) Synthesis of tert-butyl {4-[1-(2-cyanoethyl)-1H-tetrazol-5-yl]-5-(4,5-dihydroimidazo[1,5-a]quinolin-3-yl)pentyl}carbamate

To a solution of tert-butyl {5-[(2-cyanoethyl)amino]-4-(4,5-dihydroimidazo[1,5-a]quinolin-3-ylmethyl)-5-oxopentyl}carbamate (220 mg), trimethylsilylazide (160 mg) and triphenylphosphine (256 mg) in tetrahydrofuran (6 ml), diethyl azodicarboxylate (0.45 ml as a 2.2 M solution in toluene) was added under cooling with ice and the mixture was stirred for 22 hours at room temperature. Trimethylsilylazide (160 mg), triphenylphosphine (256 mg) and diethyl azodicarboxylate (0.45 ml as 2.2 M solution in toluene) were added and the mixture was stirred for 5 days at room temperature. Silica gel was added to the reaction system and the solvent was concentrated in vacuo. The resulting residue was purified by silica gel column chromatography (eluent: chloroform/methanol = 97:3 to 90:10) and subsequently by NH silica gel column chromatography (eluent: ethyl acetate/methanol = 100:0 to 97:3) to give the titled compound (32 mg) as a colorless amorphous mass.
MS(ESI/APCI Dual): 477(M+H)
¹H NMR (300 MHz, CHLOROFORM-d) δ ppm 1.42 (s, 9 H), 1.45 - 1.60 (m, 2 H), 1.92 - 2.06 (m, 1 H), 2.07 - 2.19 (m, 1 H), 2.37 - 2.47 (m, 1 H), 2.51 - 2.67 (m, 2 H), 2.70 - 2.85 (m, 2 H), 2.86 - 3.02 (m, 2 H), 3.03 - 3.26 (m, 3 H), 3.50 - 3.62 (m, 1 H), 4.20 - 4.32 (m, 1 H), 4.39 - 4.52 (m, 1 H), 4.55 - 4.65 (m, 1 H), 7.12 - 7.19 (m, 1 H), 7.21 - 7.29 (m, 2 H), 7.30 - 7.38 (m, 1 H), 7.91 (s, 1 H)

### (3) Synthesis of 5-(4,5-dihydroimidazo[1,5-a]quinolin-3-yl)-4-(1H-tetrazol-5-yl)pentan-1-amine

To a solution of tert-butyl {4-[1-(2-cyanoethyl)-1H-tetrazol-5-yl]-5-(4,5-dihydroimidazo[1,5-a]quinolin-3-yl)pentyl}carbamate (59 mg) in tetrahydrofuran (3 ml), an aqueous solution of 1 M sodium hydroxide (0.25 ml) was added and the mixture was stirred for 2.5 hours at room temperature. Water (20 ml) was added to the reaction mixture and extraction was conducted with ethyl acetate (25 ml). Conc. hydrochloric acid (2 ml) was added to the aqueous layer and the mixture was stirred for 3 hours at room temperature. The solvent was concentrated in vacuo, and the resulting residue was dissolved in water (2 ml) and purified by cation exchange chromatography (DOWEX 50WX8-200, ammonia/water = 0:1 to 5:95) to give the titled compound (compound 23, 24 mg) as a colorless amorphous mass.
MS(ESI/APCI Dual): 324(M+H)
¹H NMR (600 MHz, DEUTERIUM OXIDE) 5 ppm 1.39 - 1.49 (m, 1 H), 1.55 - 1.65 (m, 1 H), 1.86 - 1.99 (m, 2 H), 2.03 - 2.10 (m, 1 H), 2.26 - 2.32 (m, 1 H), 2.38 - 2.45 (m, 1 H), 2.57 - 2.64 (m, 1 H), 2.86 - 3.00 (m, 4 H), 3.34 - 3.41 (m, 1 H), 7.13 - 7.17 (m, 1 H), 7.22 - 7.27 (m, 2 H), 7.36 - 7.40 (m, 1 H), 8.03 (s, 1 H)

### Example 24

### Synthesis of (2S)-5-amino-2-{[7-(2-cyclohexylethyl)-4,5-dihydroimidazo[1,5-a]quinolin-3-yl]methyl}pentanoic acid dihydrochloride

### (1) Synthesis of 6-(2-cyclohexylethyl)-3,4-dihydroquinolin-2(1H)-one

To a solution of 6-(cyclohexylacetyl)-3,4-dihydroquinolin-2(1H)-one (50 mg) in methanol (1.8 ml), conc. sulfuric acid (5 mg) and 10% palladium-activated carbon (25 mg) were added and the mixture was stirred under hydrogen purge for 15 hours at room temperature. The reaction mixture was filtered through Celite and after adding a saturated aqueous solution of sodium hydrogencarbonate to the filtrate, extraction was conducted three times with chloroform. After drying over anhydrous sodium sulfate, the desiccant was filtered off and the solvent was removed in vacuo to give the titled compound (44 mg) as a colorless powder.MS(ESI/APCI Dual): 258(M+H)
¹H NMR (300 MHz, CHLOROFORM-d) δ ppm 0.83 - 1.00 (m, 2 H), 1.08 - 1.35 (m, 4 H), 1.40 - 1.80 (m, 7 H), 2.45 - 2.71 (m, 4 H), 2.85 - 3.04 (m, 2 H), 6.57 - 6.71 (m, 1 H), 6.92 - 7.03 (m, 2 H), 7.56 (br. s., 1 H)

### (2) Synthesis of ethyl 7-(2-cyclohexylethyl)-4,5-dihydroimidazo[1,5-a]quinoline-3-carboxylate

Using 6-(2-cyclohexylethyl)-3,4-dihydroquinolin-2(1H)-one (7.00 g), reaction and purification were performed by the same procedures as in Example 1(1) to give the titled compound (4.60 g) as a pale yellow powder.
MS(ESI/APCI Dual): 353(M+H)

### (3) Synthesis of [7-(2-cyclohexylethyl)-4,5-dihydroimidazo[1,5-a]quinolin-3-yl]methanol

Using ethyl 7-(2-cyclohexylethyl)-4,5-dihydroimidazo[1,5-a]quinoline-3-carboxylate (4.50 g), reaction was performed by the same procedure as in Example 1(2) to give the titled compound (3.97 g) as a pale yellow powder.
MS(ESI/APCI Dual): 311(M+H)

### (4) Synthesis of 7-(2-cyclohexylethyl)-4,5-dihydroimidazo[1,5-a]quinoline-3-carbaldehyde

Using [7-(2-cyclohexylethyl)-4,5-dihydroimidazo[1,5-a]quinolin-3-yl]methanol (3.98 g), reaction and purification were performed by the same procedures as in Example 1(3) to give the titled compound (3.35 g) as a pale brown powder.
MS(ESI/APCI Dual): 309(M+H)

### (5) Synthesis of tert-butyl (3E)-3-{[7-(2-cyclohexylethyl)-4,5-dihydroimidazo[1,5-a]quinolin-3-yl]methyliden}-2-oxopiperidine-1-carboxylate

Using 7-(2-cyclohexylethyl)-4,5-dihydroimidazo[1,5-a]quinoline-3-carbaldehyde (3.35 g), reaction and purification were performed by the same procedures as in Example 1(4) to give the titled compound (2.38 g) as a pale yellow powder.
MS(ESI/APCI Dual): 490(M+H)

### (6) Synthesis of tert-butyl 3-{[7-(2-cyclohexylethyl)-4,5-dihydroimidazo[1,5-a]quinolin-3-yl]methyl}-2-oxopiperidine-1-carboxylate

Using tert-butyl (3E)-3-{[7-(2-cyclohexylethyl)-4,5-dihydroimidazo[1,5-a]quinolin-3-yl]methyliden}-2-oxopiperidine-1-carboxylate (2.38 g), reaction and purification were performed by the same procedures as in Example 1(5) to give the titled compound (2.39 g) as a colorless powder.
MS(ESI/APCI Dual): 492(M+H)

### (7) Synthesis of 5-[(tert-butoxycarbonyl)amino]-2-{[7-(2-cyclohexylethyl)-4,5-dihydroimidazo[1,5-a]quinolin-3-yl]methyl}pentanoic acid

Using tert-butyl 3-{[7-(2-cyclohexylethyl)-4,5-dihydroimidazo[1,5-a]quinolin-3-yl]methyl}-2-oxopiperidine-1-carboxylate (2.39 g), reaction and purification were performed by the same procedures as in Example 14(7) to give the titled compound (2.33 g) as a colorless powder.
MS(ESI/APCI Dual): 510(M+H)

### (8) Synthesis of (1R,2S)-1-phenyl-2-(pyrrolidin-1-yl)propyl (2S)-5-[(tert-butoxycarbonyl)amino]-2-{[7-(2-cyclohexylethyl)-4,5-dihydroimidazo[1,5-a]quinolin-3-yl]methyl}pentanoate

Using 5-[(tert-butoxycarbonyl)amino]-2-{[7-(2-cyclohexylethyl)-4,5-dihydroimidazo[1,5-a]quinolin-3-yl]methyl}pentanoic acid (100 mg), reaction and purification were performed by the same procedures as in Example 2(2) to give the titled compound (59 mg) as a pale brown oil.
MS(ESI/APCI Dual): 698(M+H)

### (9) Synthesis of (2S)-5-[(tert-butoxycarbonyl)amino]-2-{[7-(2-cyclohexylethyl)-4,5-dihydroimidazo[1,5-a]quinolin-3-yl]methyl}pentanoic acid

Using (1R,2S)-1-phenyl-2-(pyrrolidin-1-yl)propyl (2S)-5-[(tert-butoxycarbonyl)amino]-2-{[7-(2-cyclohexylethyl)-4,5-dihydroimidazo[1,5-a]quinolin-3-yl]methyl}pentanoate (1.00 g), reaction and purification were performed by the same procedures as in Example 2(3) to give the titled compound (720 mg) as a colorless powder.
MS(ESI/APCI Dual): 510(M+H)

### (10) Synthesis of (2S)-5-amino-2-{[7-(2-cyclohexylethyl)-4,5-dihydroimidazo[1,5-a]quinolin-3-yl]methyl}pentanoic acid dihydrochloride

To a solution of (2S)-5-[(tert-butoxycarbonyl)amino]-2-{[7-(2-cyclohexylethyl)-4,5-dihydroimidazo[1,5-a]quinolin-3-yl]methyl}pentanoic acid (720 mg) in ethyl acetate (7.1 ml), a solution of 4 M hydrochloric acid in ethyl acetate (7.1 ml) was added and the mixture was stirred for an hour at room temperature. The solvent was removed by decantation and the resulting powder was dried in vacuo to give the titled compound (compound 24, 550 mg) as a pale yellow powder.
MS(ESI/APCI Dual): 410(M+H)
¹H NMR (600 MHz, DEUTERIUM OXIDE) δ ppm 0.86 - 1.00 (m, 2 H), 1.10 - 1.29 (m, 4 H), 1.38 - 1.54 (m, 2 H), 1.59 - 1.87 (m, 9 H), 2.54 (t, J=7.6 Hz, 2 H), 2.71 - 2.81 (m, 1 H), 2.81 - 2.96 (m, 5 H), 2.96 - 3.14 (m, 3 H), 7.15 - 7.20 (m, 2 H), 7.55 - 7.59 (m, 1 H), 9.18 (s, 1 H)

### Example 25

### Synthesis of (2S)-5-amino-2-[(7-cyclohexyl-4,5-dihydroimidazo[1,5-a]quinolin-3-yl)methyl]pentanoic acid dihydrochloride

### (1) Synthesis of 6-(1-cyclohexen-1-yl)-3,4-dihydroquinolin-2(1H)-one

To a solution of 6-bromo-3,4-dihydroquinolin-2(1H)-one (3.39 g) in methanol (85 ml), sodium methoxide (2.43 g), 2-(1-cyclohexen-1-yl)-4,4,5,5-tetramethyl-1,3,2-dioxaborane (3.39 g) and dichloro(bistriphenylphosphine)palladium (526 mg) were added and the mixture was heated under reflux for an hour. To the reaction system, water and ethyl acetate were added and after filtering the insolubles through Celite, the organic layer was recovered from the filtrate. After drying the resulting organic layer over anhydrous sodium sulfate, the desiccant was filtered off and the solvent was removed in vacuo. The residue was purified by silica gel column chromatography (eluent: n-hexane/ethyl acetate = 4:1 to 2:1) to give the titled compound (2.90 g) as a colorless powder.
MS(ESI/APCI Dual): 228(M+H)
¹H NMR (300 MHz, CHLOROFORM-d) δ ppm 1.60 - 1.71 (m, 2 H), 1.72 - 1.82 (m, 2 H), 2.15 - 2.24 (m, 2 H), 2.33 - 2.41 (m, 2 H), 2.59 - 2.67 (m, 2 H), 2.92 - 3.00 (m, 2 H), 6.03 - 6.09 (m, 1 H), 6.65 - 6.71 (m, 1 H), 7.16 - 7.22 (m, 2 H), 7.77 (br. s., 1 H)

### (2) Synthesis of ethyl 7-(1-cyclohexen-1-yl)-4,5-dihydroimidazo[1,5-a]quinoline-3-carboxylate

Using 6-(1-cyclohexen-1-yl)-3,4-dihydroquinolin-2(1H)-one (578 mg), reaction and purification were performed by the same procedures as in Example 1(1) to give the titled compound (390 mg) as a colorless powder.
MS(ESI/APCI Dual): 323(M+H)
¹H NMR (300 MHz, CHLOROFORM-d) δ ppm 1.43 (t, J=7.1 Hz, 3 H), 1.63 - 1.72 (m, 2 H), 1.76 - 1.84 (m, 2 H), 2.19 - 2.27 (m, 2 H), 2.36 - 2.43 (m, 2 H), 2.94 (t, J=7.1 Hz, 2 H), 3.31 - 3.37 (m, 2 H), 4.40 (q, J=7.1 Hz, 2 H), 6.13 - 6.18 (m, 1 H), 7.31 - 7.42 (m, 3 H), 7.99 (s, 1 H)

### (3) Synthesis of 7-(1-cyclohexen-1-yl)-4,5-dihydroimidazo[1,5-a]quinoline-3-carbaldehyde

Using ethyl 7-(1-cyclohexen-1-yl)-4,5-dihydroimidazo[1,5-a]quinoline-3-carboxylate (2.52 g), reaction and purification were performed by the same procedures as in Example 18(3) to give the titled compound (1.79 g) as a colorless powder.
MS(ESI/APCI Dual): 279(M+H)
¹H NMR (300 MHz, CHLOROFORM-d) δ ppm 1.63 - 1.73 (m, 2 H), 1.75 - 1.85 (m, 2 H), 2.19 - 2.28 (m, 2 H), 2.37 - 2.45 (m, 2 H), 2.95 (t, J=7.2 Hz, 2 H), 3.30 - 3.38 (m, 2 H), 6.14 - 6.19 (m, 1 H), 7.33 - 7.43 (m, 3 H), 8.04 (s, 1 H), 10.01 (s, 1 H)

### (4) Synthesis of tert-butyl (3E)-3-{[7-(1-cyclohexen-1-yl)-4,5-dihydroimidazo[1,5-a]quinolin-3-yl]methyliden}-2-oxopiperidine-1-carboxylate

Using 7-(1-cyclohexen-1-yl)-4,5-dihydroimidazo[1,5-alquinoline-3-carbaldehyde (230 mg), reaction and purification were performed by the same procedures as in Example 1(4) to give the titled compound (250 mg) as a pale orange powder.
MS(ESI/APCI Dual): 460(M+H)
¹H NMR (300 MHz, CHLOROFORM-d) δ ppm 1.57 (s, 9 H), 1.61 - 1.72 (m, 2 H), 1.75 - 1.85 (m, 2 H), 1.88 - 1.99 (m, 2 H), 2.18 - 2.28 (m, 2 H), 2.35 - 2.44 (m, 2 H), 2.89 - 2.98 (m, 2 H), 3.02 - 3.10 (m, 2 H), 3.22 - 3.30 (m, 2 H), 3.71 - 3.80 (m, 2 H), 6.11 - 6.18 (m, 1 H), 7.30 - 7.40 (m, 3 H), 7.68 - 7.72 (m, 1 H), 8.05 (s, 1 H)

### (5) Synthesis of tert-butyl 3-[(7-cyclohexyl-4,5-dihydroimidazo[1,5-a]quinolin-3-yl)methyl]-2-oxopiperidine-1-carboxylate

Using tert-butyl (3E)-3-{[7-(1-cyclohexen-1-yl)-4,5-dihydroimidazo[1,5-a]quinolin-3-yl]methyliden}-2-oxopiperidine-1-carboxylate (870 mg), reaction and purification were performed by the same procedures as in Example 1(5) to give the titled compound (770 mg) as a colorless powder.
MS(ESI/APCI Dual): 464(M+H)
¹H NMR (300 MHz, CHLOROFORM-d) δ ppm 1.16 - 1.65 (m, 17 H), 1.71 - 1.93 (m, 6 H), 1.97 - 2.09 (m, 1 H), 2.41 - 2.55 (m, 1 H), 2.61 - 2.70 (m, 1 H), 2.79 - 2.95 (m, 4 H), 3.16 - 3.24 (m, 1 H), 3.51 - 3.63 (m, 1 H), 3.74 - 3.84 (m, 1 H), 7.09 - 7.14 (m, 2 H), 7.29 - 7.33 (m, 1 H), 7.90 (s, 1 H)

### (6) Synthesis of 5-[(tert-butoxycarbonyl)amino]-2-[(7-cyclohexyl-4,5-dihydroimidazo[1,5-a]quinolin-3-yl)methyl] pentanoic acid

Using tert-butyl 3-[(7-cyclohexyl-4,5-dihydroimidazo[1,5-a]quinolin-3-yl)methyl]-2-oxopiperidine-1-carboxylate (770 mg), reaction and purification were performed by the same procedures as in Example 14(7) to give the titled compound (730 mg) as a colorless powder.
MS(ESI/APCI Dual) : 482(M+H)
¹H NMR (300 MHz, CHLOROFORM-d) δ ppm 1.15 - 1.48 (m, 14 H), 1.49 - 1.67 (m, 3 H), 1.69 - 1.94 (m, 6 H), 2.41 - 2.59 (m, 1 H), 2.83 - 3.22 (m, 9 H), 4.77 - 4.91 (m, 1 H), 7.16 (d, J=1.9 Hz, 1 H), 7.21 - 7.25 (m, 1 H), 7.62 (d, J=8.2 Hz, 1 H), 9.18 (s, 1 H)

### (7) Synthesis of (1R,2S)-1-phenyl-2-(pyrrolidin-1-yl)propyl_(2S)-5-[(tert-butoxycarbonyl)amino]-2-[(7-cyclohexyl-4,5-dihydroimidazo[1,5-a]quinolin-3-yl)methyl]pentanoate

Using 5-[(tert-butoxycarbonyl)amino]-2-[(7-cyclohexyl-4,5-dihydroimidazo[1,5-a]quinolin-3-yl)methyl]pentanoic acid (620 mg), reaction and purification were performed by the same procedures as in Example 2(2) to give the titled compound (400 mg) as a colorless powder.
MS(ESI/APCI Dual) : 669(M+H)
¹H NMR (600 MHz, CHLOROFORM-d) δ ppm 0.93 (d, J=6.4 Hz, 3 H), 1.22 - 1.30 (m, 1 H), 1.35 - 1.48 (m, 15 H), 1.66 - 1.79 (m, 8 H), 1.82 - 1.90 (m, 4 H), 1.96 - 2.06 (m, 1 H), 2.37 - 2.43 (m, 1 H), 2.44 - 2.56 (m, 4 H), 2.57 - 2.68 (m, 5 H), 2.79 - 2.91 (m, 1 H), 3.08 - 3.23 (m, 2 H), 5.25 - 5.32 (m, 1 H), 6.09 (br. s., 1 H), 6.68 - 6.74 (m, 1 H), 6.82 - 6.92 (m, 4 H), 6.95 (s, 1 H), 7.10 - 7.15 (m, 1 H), 7.29 (d, J=8.3 Hz, 1 H), 7.93 (s, 1 H)

### (8) Synthesis of (2S)-5-[(tert-butoxycarbonyl)amino]-2-[(7-cyclohexylethyl-4,5-dihydroimidazo[1,5-a]quinolin-3-yl)methyl]pentanoic acid

Using (1R,2S)-1-phenyl-2-(pyrrolidin-1-yl)propyl (2S)-5-[(tert-butoxycarbonyl)amino]-2-[(7-cyclohexylethyl-4,5-dihydroimidazo[1,5-a]quinolin-3-yl)methyl]pentanoate (400 mg), reaction and purification were performed by the same procedures as in Example 2(3) to give the titled compound (230 mg) as a colorless powder.
MS(ESI/APCI Dual) : 482(M+H)
¹H NMR (600 MHz, CHLOROFORM-d) δ ppm 1.22 - 1.30 (m, 2 H), 1.32 - 1.50 (m, 12 H), 1.52 - 1.61 (m, 2 H), 1.70 - 1.91 (m, 7 H), 2.44 - 2.53 (m, 1 H), 2.76 - 2.93 (m, 7 H), 3.03 - 3.15 (m, 2 H), 4.58 - 4.66 (m, 1 H), 7.10 - 7.17 (m, 2 H), 7.31 (d, J=8.3 Hz, 1 H), 7.98 (s, 1 H)

### (9) Synthesis of (2S)-5-amino-2-[(7-cyclohexyl-4,5-dihydroimidazo[1,5-a]quinolin-3-yl)methyl]pentanoic acid dihydrochloride

To a solution of (2S)-5-[(tert-butoxycarbonyl)amino]-2-[(7-cyclohexylethyl-4,5-dihydroimidazo[1,5-a]quinolin-3-yl)methyl]pentanoic acid (230 mg) in ethyl acetate (10 ml), a solution of 4 M hydrochloric acid in ethyl acetate (5 ml) was added and the mixture was stirred for 3 hours at room temperature. The solvent was concentrated in vacuo and after washing the resulting residue with ethyl acetate and diethyl ether, decantation was performed. After three azeotropic distillations with water, the residue was dried to give the titled compound (compound 25, 190 mg) as a pale brown amorphous mass.
MS(ESI/APCI Dual): 382(M+H)
¹H NMR (600 MHz, DEUTERIUM OXIDE) 5 ppm 1.16 - 1.28 (m, 1 H), 1.29 - 1.43 (m, 4 H), 1.64 - 1.86 (m, 9 H), 2.43 - 2.58 (m, 1 H), 2.75 - 2.85 (m, 1 H), 2.87 - 3.12 (m, 8 H), 7.22 - 7.36 (m, 2 H), 7.51 - 7.60 (m, 1 H), 9.19 (s, 1 H)

### Example 26

### Synthesis of (2S)-5-amino-2-[(7-fluoro-4,5-dihydroimidazo[1,5-a]quinolin-3-yl]methyl}pentanoic acid dihydrochloride

### (1) Synthesis of ethyl 7-fluoro-4,5-dihydroimidazo[1,5-a]quinoline-3-carboxylate

Using 6-fluoro-3,4-dihydroquinolin-2(1H)-one (10.0 g), reaction and purification were performed by the same procedures as in Example 1(1) to give the titled compound (6.23 g) as a pale yellow powder.
MS(ESI): 261(M+H)

### (1) Synthesis of 7-fluoro-4,5-dihydroimidazo[1,5-a]quinoline-3-carbaldehyde

Using ethyl 7-fluoro-4,5-dihydroimidazo[1,5-a]quinoline-3-carboxylate (6.23 g), reaction and purification were performed by the same procedures as in Example 18(3) to give the titled compound (4.55 g) as a colorless powder.
MS(ESI/APCI Dual): 217(M+H)

### (3) Synthesis of tert-butyl (3E)-3-[(7-fluoro-4,5-dihydroimidazo[1,5-a]quinolin-3-yl)methyliden]-2-oxopiperidine-1-carboxylate

Using 7-fluoro-4,5-dihydroimidazo[1,5-a]quinoline-3-carbaldehyde (4.55 g), reaction and purification were performed by the same procedures as in Exmple 1(4) to give the titled compound (5.58 g) as a pale yellow powder.
MS(ESI/APCI Dual): 398(M+H)

### (4) Synthesis of tert-butyl 3-[(7-fluoro-4,5-dihydroimidazo[1,5-a]quinolin-3-yl)methyl]-2-oxopiperidine-1-carboxylate

Using tert-butyl (3E)-3-[(7-fluoro-4,5-dihydroimidazo[1,5-a]quinolin-3-yl)methyliden]-2-oxopiperidine-1-carboxylate (5.58 g), reaction and purification were performed by the same procedures as in Example 1(5) to give the titled compound (3.51 g) as a pale yellow powder.
MS(ESI/APCI Dual): 400(M+H)

### (5) Synthesis of 5-[(tert-butoxcarbonyl)amino]-2-[(7-fluoro-4,5-dihydroimidazo[1,5-a]quinolin-3-yl)methyl]pentanoic acid

Using tert-butyl 3-[(7-fluoro-4,5-dihydroimidazo[1,5-a]quinolin-3-yl)methyl]-2-oxopiperidine-1-carboxylate (3.51 g), reaction and purification were performed by the same procedures as in Example 14(7) to give the titled compound (3.55 g) as a colorless powder.
MS(ESI/APCI Dual): 418(M+H)

### (6) Synthesis of (1R,2S)-1-phenyl-2-(pyrrolidin-1-yl)propyl_(2S)-5-[(tert-butoxycarbonyl)amino]-2-[(7-fluoro-4,5-dihydroimidazo[1,5-a]quinolin-3-yl)methyl]pentanoate

Using 5-[(tert-butoxycarbonyl)amino]-2-[(7-fluoro-4,5-dihydroimidazo[1,5-a]quinolin-3-yl)methyl]pentanoic acid (2.66 g), reaction and purification were performed by the same procedures as in Example 2(2) to give the titled compound (970 mg) as a pale brown oil.
MS(ESI/APCI Dual): 605(M+H)

### (7) Synthesis of (2S)-5-[(tert-butoxycarbonyl)amino]-2-[(7-fluoro-4,5-dihydroimidazo[1,5-a]quinolin-3-yl)methyl]pentanoic acid

Using (1R,2S)-1-phenyl-2-(pyrrolidin-1-yl)propyl (2S)-5-[(tert-butoxycarbonyl)amino]-2-[(7-fluoro-4,5-dihydroimidazo[1,5-a]quinolin-3-yl)methyl]pentanoate (970 mg), reaction and purification were performed by the same procedures as in Example 2(3) to give the titled compound (623 mg) as a colorless powder.
MS(ESI/APCI Dual): 418(M+H)

### (8) Synthesis of (2S)-5-amino-2-[(7-fluoro-4,5-dihydroimidazo[1,5-a]quinolin-3-yl)methyl]pentanoic acid dihydrochloride

Using (2S)-5-[(tert-butoxycarbonyl)amino]-2-[(7-fluoro-4,5-dihydroimidazo[1,5-a]quinolin-3-yl)methyl]pentanoic acid (313 mg), reaction and purification were performed by the same procedures as in Example 24 (10) to give the titled compound (compound 26, 215 mg) as a pale yellow amorphous mass.
MS(ESI/APCI Dual): 318(M+H)
¹H NMR (600 MHz, DEUTERIUM OXIDE) 5 ppm 1.64 - 1.83 (m, 4 H), 2.78 - 2.88 (m, 1 H), 2.93 - 3.13 (m, 8 H), 7.15 - 7.23 (m, 1 H), 7.23 - 7.29 (m, 1 H), 7.65 - 7.75 (m, 1 H), 9.20 (s, 1 H)

### Example 27

### Synthesis of 5-amino-2-{[7-(trifluoromethyl)-4,5-dihydroimidazo[1,5-a]quinolin-3-yl]methyl}pentanoic acid dihydrochloride

### (1) Synthesis of ethyl 7-(trifluoromethyl)-4,5-dihydroimidazo[1,5-a]quinoline-3-carboxylate

Using 6-(trifluoromethyl)-3,4-dihydroquinolin-2(1H)-one (7.09 g), reaction and purification were performed by the same procedures as in Example 1(1) to give the titled compound (6.00 g) as a pale brown powder.
MS(ESI/APCI Dual): 311(M+H)
¹H NMR (300 MHz, CHLOROFORM-d) δ ppm 1.43 (t, J=7.1 Hz, 3 H), 3.03 (t, J=7.3 Hz, 2 H), 3.29 - 3.49 (m, 2 H), 4.42 (q, J=7.1 Hz, 2 H), 7.51 - 7.68 (m, 3 H), 8.06 (s, 1 H)

### (2) Synthesis of 7-(trifluoromethyl)-4,5-dihydroimidazo[1,5-a]quinoline-3-carbaldehyde

Using ethyl 7-(trifluoromethyl)-4,5-dihydroimidazo[1,5-a]quinoline-3-carboxylate (6.00 g), reaction and purification were performed by the same procedures as in Example 18(3) to give the titled compound (4.26 g) as a pale yellow powder.
MS(ESI/APCI Dual): 267(M+H)
¹H NMR (300 MHz, CHLOROFORM-d) δ ppm 2.97 - 3.10 (m, 2 H), 3.34 - 3.45 (m, 2 H), 7.53 - 7.70 (m, 3 H), 8.10 (s, 1 H), 10.03 (s, 1 H)

### (3) Synthesis of tert-butyl (3E)-2-oxo-3-{[7-(trifluoromethyl)-4,5-dihydroimidazo[1,5-a]quinolin-3-yl]methylidene}piperidine-1-carboxylate

Using 7-(trifluoromethyl)-4,5-dihydroimidazo[1,5-a]quinoline-3-carbaldehyde (130 mg), reaction and purification were performed by the same procedures as in Example 1(4) to give the titled compound (100 mg) as a colorless powder.
MS(ESI/APCI Dual): 448(M+H)
¹H NMR (300 MHz, CHLOROFORM-d) δ ppm 1.57 (s, 9 H), 1.88 - 2.00 (m, 2 H), 2.95 - 3.05 (m, 2 H), 3.06 - 3.14 (m, 2 H), 3.21 - 3.30 (m, 2 H), 3.72 - 3.80 (m, 2 H), 7.51 - 7.57 (m, 1 H), 7.58 - 7.64 (m, 2 H), 7.65 - 7.71 (m, 1 H), 8.10 (s, 1 H)

### (4) Synthesis of tert-butyl 2-oxo-3-{[7-(trifluoromethyl)-4,5-dihydroimidazo[1,5-a]quinolin-3-yl]methyl}piperidine-1-carboxylate

Using tert-butyl (3E)-2-oxo-3-{[7-(trifluoromethyl)-4,5-dihydroimidazo[1,5-a]quinolin-3-yl]methylidene}piperidine-1-carboxylate (100 mg), reaction and purification were performed by the same procedures as in Example 1(5) to give the titled compound (80 mg) as a colorless powder.
MS(ESI/APCI Dual): 450(M+H)
¹H NMR (300 MHz, CHLOROFORM-d) δ ppm 1.51 (s, 9 H), 1.55 - 1.70 (m, 1 H), 1.72 - 1.94 (m, 2 H), 2.00 - 2.13 (m, 1 H), 2.63 - 2.75 (m, 1 H), 2.80 - 3.04 (m, 5 H), 3.12 -3.23 (m, 1 H), 3.51 - 3.64 (m, 1 H), 3.76 - 3.84 (m, 1 H), 7.45 - 7.51 (m, 1 H), 7.53 - 7.59 (m, 2 H), 7.96 (s, 1 H)

### (5) Synthesis of 5-[(tert-butoxcarbonyl)amino]-2-{[7-(trifluoromethyl)-4,5-dihydroimidazo[1,5-a]quinolin-3-yl]methyl}pentanoic acid

Using tert-butyl 2-oxo-3-{[7-(trifluoromethyl)-4,5-dihydroimidazo[1,5-a]quinolin-3-yl]methyl}piperidine-1-carboxylate (130 mg), reaction and purification were performed by the same procedures as in Example 14(7) to give the titled compound (80 mg) as a colorless powder.
MS(ESI/APCI Dual): 468(M+H)
¹H NMR (300 MHz, CHLOROFORM-d) δ ppm 1.35 - 1.45 (m, 9 H), 1.47 - 1.66 (m, 3 H), 1.71 - 1.87 (m, 1 H), 2.73 - 3.04 (m, 7 H), 3.06 - 3.19 (m, 2 H), 4.59 - 4.72 (m, 1 H), 7.48 - 7.63 (m, 3 H), 8.13 (s, 1 H)

### (6) Synthesis of 5-amino-2-{[7-(trifluoromethyl)-4,5- dihydroimidazo[1,5-a]quinolin-3-yl]methyl}pentanoic acid dihydrochloride

Using 5-[(tert-butoxcarbonyl)amino]-2-{[7-(trifluoromethyl)-4,5-dihydroimidazo[1,5-a]quinolin-3-yl]methyl}pentanoic acid (80 mg), reaction and purification were performed by the same procedures as in Example 24(10) to give the titled compound (compound 27, 57 mg) as a pale brown amorphous mass.
MS(ESI/APCI Dual): 368(M+H)
¹H NMR (600 MHz, DEUTERIUM OXIDE) δ ppm 1.55 - 1.75 (m, 4 H), 2.55 - 2.64 (m, 1 H), 2.83 - 2.90 (m, 1 H), 2.93 - 3.11 (m, 7 H), 7.75 - 7.81 (m, 1 H), 7.82 - 7.86 (m, 2 H), 9.25 (s, 1 H)

### Example 28

### Synthesis of (2S)-5-amino-2-[(7-isopropyl-4,5-dihydroimidazo[1,5-a]quinolin-3-yl)methyl]pentanoic acid dihydrochloride

### (1) Synthesis of ethyl 7-(benzyloxy)-4,5-dihydroimidazo[1,5-a]quinoline-3-carboxylate

Using 6-(benzyloxy)-3,4-dihydroquinolin-2(1H)-one (40.0 g), reaction and purification were performed by the same procedures as in Example 1(1) to give the titled compound (22.9 g) as a yellow powder.
MS(ESI/APCI Dual): 349(M+H)

### (2) Synthesis of 7-(benzyloxy)-4,5-dihydroimidazo[1,5-a]quinoline-3-carbaldehyde

Using ethyl 7-(benzyloxy)-4,5-dihydroimidazo[1,5-a]quinoline-3-carboxylate (21.9 g), reaction and purification were performed by the same procedures as in Example 18(3) to give the titled compound (13.6 g) as a colorless powder. MS(ESI/APCI Dual): 305(M+H)

### (3) Synthesis of tert-butyl (3E)-3-{[7-(benzyloxy)-4,5-dihydroimidazo[1,5-a]quinolin-3-yl]methyliden}-2-oxopiperidine-1-carboxylate

Using 7-(benzyloxy)-4,5-dihydroimidazo[1,5-a]quinoline-3-carbaldehyde (24.4 g), reaction and purification were performed by the same procedures as in Example 1(4) to give the titled compound (20.8 g) as a pale yellow powder.
MS(ESI/APCI Dual): 486(M+H)

### (4) Synthesis of tert-butyl 3-[(7-hydroxy-4,5-dihydroimidazo[1,5-a]quinolin-3-yl)methyl]-2-oxopiperidine-1-carboxylate

Using tert-butyl (3E)-3-{[7-(benzyloxy)-4,5-dihydroimidazo[1,5-a]quinolin-3-yl]methyliden}-2-oxopiperidine-1-carboxylate (20.8 g), reaction and purification were performed by the same procedures as in Example 1(5) to give the titled compound (16.1 g) as a pale yellow powder.
MS(ESI/APCI Dual): 398(M+H)

### (5) Synthesis of tert-butyl 2-oxo-3-[(7-{[(trifluoromethyl)sulfonyl]oxy}-4,5-dihydroimidazo[1,5-a]quinolin-3-yl)methyl]piperidine-1-carboxylate

To a solution of tert-butyl 3-[(7-hydroxy-4,5-dihydroimidazo[1,5-a]quinolin-3-yl)methyl]-2-oxopiperidine-1-carboxylate (1.00 g) in chloroform (8.4 ml), triethylamine (510 mg) and 1,1,1-trifluoro-N-phenyl-N-[(trifluoromethyl)sulfonyl]methanesulfonamide (1.08 g) were added and the mixture was stirred for 5 hours at room temperature. To the reaction system, a saturated aqueous solution of sodium hydrogencarbonae was added and extraction was conducted three times with chloroforml. After drying the organic layer with anhydrous sodium sulfate, the desiccant was filtered off and the solvent was removed in vacuo. The resulting residue was purified by silica gel column chromatography (eluent: n-hexane/ethyl acetate = 1:1 to 0:1) to give the titled compound (1.32 g) as a colorless powder.
MS(ESI/APCI Dual): 530(M+H)
¹H NMR (300 MHz, CHLOROPORM-d) δppm 1.50 (s, 9 H), 1.55 - 1.71 (m, 1 H), 1.71 - 1.94 (m, 2 H), 1.98 - 2.15 (m, 1 H), 2.69 (dd, J=14.3, 7.8 Hz, 1 H), 2.79 - 3.06 (m, 5 H), 3.16 (dd, J=14.3, 5.1 Hz, 1 H), 3.56 (ddd, J=12.9, 7.2, 5.3 Hz, 1 H), 3.80 (ddd, J=12.9, 7.2, 5.3 Hz, 1 H), 7.16 - 7.30 (m, 2 H), 7.41 - 7.51 (m, 1 H), 7.92 (s, 1 H)

### (6) Synthesis of tert-butyl 2-oxo-3-{[7-(1-propen-2-yl)-4,5-dihydroimidazo[1,5-a]quinolin-3-yl]methyl}piperidine-1-carboxylate

To a solution of tert-butyl 2-oxo-3-[(7-{[(trifluoromethyl)sulfonyl]oxy}-4,5-dihydroimidazo[1,5-a]quinolin-3-yl)methyl]piperidine-1-carboxylate (5.00 g) in tetrahydrofuran (94.4 ml), 4,4,5,5-tetramethyl-2-(1-propen-2-yl)-1,3,2-dioxaborane (3.17 g), cesium carbonate (12.3 g) and 1,1-bis(diphenylphosphino)ferrocene-palladium(II) dichloride dichloromethane complex (2.31 g) were added and the mixture was heated under reflux for 30 minutes. The reaction system was concentrated in vacuo and the residue was purified by NH silica gel column chromatography (eluent: ethyl acetate). After concentrating in vacuo, an aqueous solution of 15% citric acid was added to the residue and back extraction was conducted with diethyl ether. The aqueous layer was neutralized with a saturated aqueous solution of sodium hydrogencarbonate and extraction was conducted three times with chloroform. After drying the organic layer over anhydrous sodium sulfate, the desiccant was filtered off and the solvent was removed in vacuo. The resulting residue was purified by silica gel column chromatography (eluent: n-hexane/ethyl acetate = 1:4) to give the titled compound (2.74 g) as a colorless powder.
¹H NMR (300 MHz, CHLOROFORM-d) δ ppm 1.50 (s, 9 H), 1.55 - 1.71 (m, 1 H), 1.71 - 1.95 (m, 2 H), 1.99 - 2.12 (m, 1 H), 2.13 - 2.21 (m, 3 H), 2.67 (dd, J=14.5, 8.4 Hz, 1 H), 2.79 - 3.02 (m, 5 H), 3.21 (dd, J=14.5, 4.7 Hz, 1 H), 3.50 - 3.65 (m, 1 H), 3.74 - 3.88 (m, 1 H), 5.08 - 5.12 (m, 1 H), 5.36 - 5.40 (m, 1 H), 7.31 - 7.45 (m, 3 H), 7.93 (s, 1 H)

### (7) Synthesis of 5-[(tert-butoxycarbonyl)amino]-2-{[7-(1-propen-2-yl)-4,5-dihydroimidazo[1,5-a]quinolin-3-yl]methyl}pentanoic acid

Using tert-butyl 2-oxo-3-{[7-(1-propen-2-yl)-4,5-dihydroimidazo[1,5-a]quinolin-3-yl]methyl}piperidine-1-carboxylate (2.74 g), reaction and purification were performed by the same procedures as in Example 14(7) to give the titled compound (2.85 g) as a colorless powder.
MS(ESI/APCI Dual): 440(M+H)

### (8) Synthesis of (1R,2S)-1-phenyl-2-(pyrrolidin-1-yl)propyl

### (2S)-5-[(tert-butoxycarbonyl)amino]-2-{[7-(1-propen-2-yl)-4,5-dihydroimidazo[1,5-a]quinolin-3-yl]methyl}pentanoate

Using 5-[(tert-butoxycarbonyl)amino]-2-{[7-(1-propen-2-yl)-4,5-dihydroimidazo[1,5-a]quinolin-3-yl]methyl}pentanoic acid (200 mg), reaction and purification were performed by the same procedures as in Example 2(2) to give the titled compound (100 mg) as a pale brown powder.
MS(ESI/APCI Dual): 627(M+H)

### (9) Synthesis of (2S)-5-[(tert-butoxycarbonyl)amino]-2-[(7-isopropyl-4,5-dihydroimidazo[1,5-a]quinolin-3-yl)methyl]pentanoic acid

Using (1R,2S)-1-phenyl-2-(pyrrolidin-1-yl)propyl (2S)-5-[(tert-butoxycarbonyl)amino]-2-{[7-(1-propen-2-yl)-4,5-dihydroimidazo[1,5-a]quinolin-3-yl]methyl}pentanoate (1.88 g), reaction and purificatin were performed by the same procedures as in Example 2(3) to give the titled compound (1.11 g) as a colorless amorphous mass.
MS(ESI/APCI Dual): 442(M+H)

### (10) Synthesis of (2S)-5-amino-2-[(7-isopropyl-4,5-dihydroimidazo[1,5-a]quinolin-3-yl)methyl]pentanoic acid dihydrochloride

To a solution of (2S)-5-[(tert-butoxycarbonyl)amino]-2-[(7-isopropyl-4,5-dihydroimidazo[1,5-a]quinolin-3-yl)methyl]pentanoic acid (600 mg) in ethyl acetate (13.6 ml), a solution of 4 M hydrochloric acid in ethyl acetate (13.6 ml) was added and the mixture was stirred for 2 hours at room temperature. The solvent was removed in vacuo to give the titled compound (compound 28, 485 mg) as a colorless amorphous mass.
MS(ESI/APCI Dual): 342(M+H)
¹H NMR (600 MHz, DEUTERIUM OXIDE) δppm 1.24 (d, J=7.3 Hz, 6 H), 1.57 - 1.78 (m, 4 H), 2.60 - 2.70 (m, 1 H), 2.85 - 2.92 (m, 1 H), 2.92 - 3.09 (m, 8 H), 7.37 (dd, J=8.3, 1.8 Hz, 1 H), 7.40 (d, J=1.8 Hz, 1 H), 7.61 (d, J=8.3 Hz, 1 H), 9.14 (s, 1 H)

### Example 29

### Synthesis of (2S)-5-amino-2-[(7-methyl-4,5-dihydroimidazo[1,5-a]quinolin-3-yl)methyl]pentanoic acid dihydrochloride

### (1) Synthesis of tert-butyl 3-[(7-methyl-4,5-dihydroimidazo(1,5-a]quinolin-3-yl)methyl]-2-oxapiperidine-1-carboxylate

To a solution of tert-butyl 2-oxo-3-[(7-{[(trifluoromethyl)sulfonyl]oxy}-4,5-dihydroimidazo[1,5-a]quinolin-3-yl)methyl]piperidine-1-carboxylate (5.00 g) in tetrahydrofuran (200 ml), methylboronic acid (2.26 g), cesium carbonate (12.3 g) and 1,1-bis(diphenylphosphino)ferrocene-palladium(II) dichloride dichloromethane complex (2.36 g) were added and the mixture was heated under reflux for 4.5 hours. The reaction system was concentrated in vacuo and after adding ethyl acetate (300 ml) and water (100 ml) to the residue, the mixture was filtered through Celite. After the filtrate separated into an aqueous layer and an organic layer, the latter was washed with an aqueous solution of 15% citric acid three times, and with an aqueous solution of 1 M hydrochloric acid three times. After neutralizing the combined aqueous layers with a saturated aqueous solution of sodium hydrogencarbonate, extraction was conducted with ethyl acetate. After drying the organic layer over anhydrous magnesium sulfate, the desiccant was filtered off and the solvent was removed in vacuo. The resulting residue was purified by NH silica gel column chromatography (eluent: n-hexane/ethyl acetate = 35:65 to 0:100) and subsequent recrystallization from n-hexane/chloroform gave the titled compound (1.46 g) as a colorless powder.
MS(ESI/APCI Dual): 396(M+H)
¹H NMR (300 MHz, CHLOROFORM-d) δ ppm 1.52 (s, 9 H), 1.54 - 1.64 (m, 1 H), 1.70 - 1.92 (m, 2 H), 1.97 - 2.13 (m, 1 H), 2.34 (s, 3 H), 2.61 - 2.72 (m, 1 H), 2.79 - 2.97 (m, 5 H), 3.16 - 3.26 (m, 1 H), 3.51 - 3.62 (m, 1 H), 3.74 - 3.86 (m, 1 H), 7.10 (s, 1 H), 7.25 - 7.29 (m, 2 H), 7.90 (s, 1 H)

### (2) Synthesis of 5-[(tert-butoxycarbonyl)amino]-2-[(7-methyl-4,5-dihydroimidazo[1,5-a]quinolin-3-yl)methyl]pentanoic acid

Using tert-butyl 3-[(7-methyl-4,5-dihydroimidazo[1,5-a]quinolin-3-yl)methyl]-2-oxapiperidzne-1-carboxylate (1.58 g), reaction and purification were performed by the same procedures as in Example 14(7) to give the titled compound (16.7 g) as a colorless amorphous mass.
MS(ESI/APCI Dual): 414(M+H)
¹H NMR (300 MHz, CHLOROFORM-d) δppm 1.41 (s, 9 H), 1.43 - 1.65 (m, 3 H), 1.69 - 1.88 (m, 1 H), 2.36 (s, 3 H), 2.72 - 2.93 (m, 7 H), 3.05 - 3.17 (m, 2 H), 4.57 - 4.70 (m, 1 H), 7.08 - 7.15 (m, 2 H), 7.27 - 7.32 (m, 1 H), 8.02 (s, 1 H)

### (3) Synthesis of (1R,2S)-1-phenyl-2-(pyrrolidin-1-yl)propyl (2S)-5-[(tert-butoxycarbonyl)amino]-2-[(7-methyl-4,5-dihydroimidazo[1,5-a]quinolin-3-yl)methyl]pentanoate

To a solution of 5-[(tert-butoxycarbonyl)amino]-2-[(7-methyl-4,5-dihydroimidazo[1,5-a]quinolin-3-yl)methyl]pentanoic acid (1.67 g) in chloroform (20 ml), (1R,2S)-1-phenyl-2-(pyrrolidin-1-yl)propan-1-ol (1.23 g), 4-dimethylaminopyridine (99 mg) and N-[3-(dimethylamino)propyl]-N'-ethylcarbadiimide hydrochloride (1.15 g) were added and the mixture was stirred for 16 hours at room temperature. The reaction mixture was purified by silica gel column chromatography (eluent: chloroform/methanol = 90:10 to 80:20), then by three runs of NH silica gel column chromatography (eluent: n-hexane/2-propanol = 95:5), subsequently by yet another run of NH silica gel column chromatography (eluent: n-hexane/2-propanol = 96:4), finally by silica gel column chromatography (eluent: chloroform/methanol = 85:15 to 70:30) to give the titled compound (685 mg) as a colorless amorphous mass.
MS(ESI/APCI Dual): 601(M+H)
¹H NMR (300 MHz, CHLOROFORM-d) δ ppm 0.94 (d, J=6.7 Hz, 3 H), 1.44 (s, 9 H), 1.62 - 1.88 (m, 8 H), 1.95 - 2.12 (m, 1 H), 2.34 (s, 3 H), 2.38 - 2.46 (m, 1 H), 2.48 - 2.57 (m, 3 H), 2.58 - 2.71 (m, 5 H), 2.80 - 2.91 (m, 1 H), 3.09 - 3.27 (m, 3 H), 5.27 - 5.37 (m, 1 H), 6.07 - 6.14 (m, 1 H), 6.73 - 6.81 (m, 1 H), 6.84 - 6.98 (m, 5 H), 7.06 - 7.11 (m, 1 H), 7.26 - 7.31 (m, 1 H), 7.94 (s, 1 H)

### (4) Synthesis of (2S)-5-[(tert-butoxycarbonyl)amino]-2-[(7-methyl-4,5-dihydroimidazo[1,5-a]quinolin-3-yl)methyl]pentanoic acid

To a solution of (1R,2S)-1-phenyl-2-(pyrrolidin-1-yl)propyl (2S)-5-[(tert-butoxycarbonyl)amino]-2-[(7-methyl-4,5-dihydroimidazo[1,5-a]quinolin-3-yl)methyl]pentanoate (500 mg) in methanol (10 ml), 20% palladium hydroxide (44% water content, 250 mg) and the mixture was stirred under hydrogen purge for 3.5 hours at 60 °C. The reaction mixture was filtered through Celite and the solvent was removed in vacuo; the resulting residue was purified by silica gel column chromatography (eluent: chloroform/methanol = 9:1), then by HPLC (CAPCELLPAK MGII; eluent: 0.025% acetic acid/methanol = 35:65); the purified residue was reduced to powder from n-hexane/ethyl acetate, to give the titled compound (105 mg) as a colorless powder.
MS(ESI/APCI Dual): 414(M+H)
¹H NMR (300 MHz, CHLOROFORM-d) δppm 1.41 (s, 9 H), 1.43 - 1.65 (m, 3 H), 1.69 - 1.88 (m, 1 H), 2.36 (s, 3 H), 2.72 - 2.93 (m, 7 H), 3.05 - 3.17 (m, 2 H), 4.57 - 4.70 (m, 1 H), 7.08 - 7.15 (m, 2 H), 7.27 - 7.32 (m, 1 H), 8.02 (s, 1 H)

### (5) Synthesis of (2S)-5-amino-2-[(7-methyl-4,5-dihydroimidazo[1,5-a]quinolin-3-yl)methyl]pentanoic acid dihydrochloride

To (2S)-5-[(tert-butoxycarbonyl)aminol-2-[(7-methyl-4,5-dihydroimidazo[1,5-a]quinolin-3-yl)methyllpentanoic acid (105 mg), an aqueous solution of 6 M hydrochloric acid (4 ml) was added and the mixture was stirred for 2.5 hours at room temperature. The solvent was removed in vacuo to give the titled compound (compound 29, 95 mg) as a colorless amorphous mass.
MS(ESI/APCI Dual): 314 (M+H)
¹H NMR (600 MHz, DEUTERIUM OXIDE) δ ppm 1.63 - 1.79 (m, 4H), 2.36 (s, 3 H), 2.71 - 2.78 (m, 1 H), 2.91 - 2.98 (m, 5 H), 2.99 - 3.06 (m, 3 H), 7.27 (d, J=8.3 Hz, 1 H), 7.30 (s, 1 H), 7.53 (d, J=8.3 Hz, 1 H), 9.13 (s, 1 H)

### Example 30

### Synthesis of (S)-5-amino-2-((7-propyl-4,5-dihydroimidazo[1,5-a]quinolin-3-yl)methyl)pentanoic acid dihydrochloride

### (1) Synthesis of (Z)-tert-butyl 2-oxo-3-((7-propen-1-yl)-4,5-dihydroimidazo[1,5-a]quinolin-3-yl)methyl)piperidine-1-carboxylate

To a solution of tert-butyl 2-oxo-3-((7-trifluoromethylsulfonyloxy-4,5-dihydroimidazo[1,5-a]quinolin-3-yl)methyl)piperidine-1-carboxylate (5.13 g) in tetrahydrofuran (250 ml), (1Z)-1-propen-1-yl-boronic acid (3.33 g), cesium carbonate (12.6 g) and 1,1-bis(diphenylphosphino)ferrocene-palladium(II) dichloride dichloromethane complex (2.38 g) were added and the mixture was heated under reflux for 7 hours. The reaction system was filtered through Celite and concentrated in vacuo; the resulting residue was purified by NH silica gel column chromatography (eluent: n-hexane/ethyl acetate = 1:1 to 0:1). The purified residue was reduced to powder from n-hexane/diethyl ether, to give the titled compound (1.68 g) as a colorless powder.
MS(ESI/APCI Dual): 422(M+H)
¹H NMR (300 MHz, CHLOROFORM-d) δ ppm 1.52 (s, 9 H), 1.55 - 1.68 (m, 2 H), 1.73 - 1.96 (m, 4 H), 1.98 - 2.13 (m, 1 H), 2.60 - 2.73 (m, 1 H), 2.75 - 3.00 (m, 5 H), 3.13 - 3.27 (m, 1 H), 3.48 - 3.64 (m, 1 H), 3.73 - 3.87 (m, 1 H), 5.72 - 5.89 (m, 1 H), 6.35 - 6.46 (m, 1 H), 7.20 - 7.25 (m, 2 H), 7.33 - 7.40 (m, 1 H), 7.93 (s, 1 H)

### (1) Synthesis of tert-butyl 2-oxo-3-[(7-propyl-4,5-dihydroimidazo[1,5-a]quinolin-3-yl)methyl]piperidine-1-carboxylate

To a solution of tert-butyl 2-oxo-3-({7-[(1Z)-1-propen-1-yl]-4,5-dihydroimidazo[1,5-a]quinolin-3-yl)methyl)piperidine-1-carboxylate (1.68 g) in methanol (30 ml), 10% palladium-activated carbon (500 mg) was added and the mixture was stirred under hydrogen purge for an hour at room temperature. The reaction mixture was filtered through Celite and the solvent was removed in vacuo. The resulting residue was purified by silica gel column chromatography (eluent: chloroform/methanol = 49:1 to 9:1) and the purified residue was reduced to powder from n-hexane/diethyl ether, to give the titled compound (1.20 g) as a colorless powder.
MS(ESI/APCI Dual): 424(M+H)
¹H NMR (300 MHz, CHLOROFORM-d) δ ppm 0.95 (t, J=7.3 Hz, 3 H), 1.52 (s, 9 H), 1.56 - 1.69 (m, 3 H), 1.70 - 1.91 (m, 2 H), 1.97 - 2.11 (m, 1 H), 2.53 - 2.61 (m, 2 H), 2.62 - 2.71 (m, 1 H), 2.80 - 2.96 (m, 5 H), 3.08 - 3.25 (m, 1 H), 3.51 - 3.62 (m, 1 H), 3.74 - 3.86 (m, 1 H), 7.05 - 7.12 (m, 2 H), 7.28 - 7.33 (m, 1 H), 7.91 (s, 1 H)

### (3) Synthesis of 5-[(tert-butoxycarbonyl)amino]-2-[(7-propyl-4,5-dihydroimidazo[1,5-a]quinolin-3-yl)methyl]pentanoic acid

Using tert-butyl 2-oxo-3-[(7-propyl-4,5-dihydroimidazo[1,5-a]quinolin-3-yl)methyl]piperidine-1-carboxylate (1.20 g), reaction and purification were performed by the same procedures as in Example 14(7) to give the titled compound (1.35 g) as a colorless amorphous mass.
MS(ESI/APCI Dual): 442(M+H)

¹H NMR (300 MHz, CHLOROFORM-d) δ ppm 0.91 - 0.99 (m, 3 H), 1.41 (s, 9 H), 1.45 - 1.84 (m, 6 H), 2.52 - 2.63 (m, 2 H), 2.77 - 2.94 (m, 7 H), 3.05 - 3.17 (m, 2 H), 4.60 - 4.71 (m, 1 H), 7.09 - 7.16 (m, 2 H), 7.35 (d, J=8.7 Hz, 1 H), 8.15 (s, 1 H)

### (4) Synthesis of (1R,2S)-1-phenyl-2-(pyrrolidin-1-yl)propyl

### (2S)-5-[(tert-butoxycarbonyl)amino]-2-[(7-propyl-4,5-dihydroimidazo[1,5-a]quinolin-3-yl)methyl]pentanoate

Using 5-[(tert-butoxycarbonyl)amino]-2-[(7-propyl-4,5-dihydroimidazo[1,5-a]quinolin-3-yl)methyl]pentanoic acid (1.35 g), reaction and purification were performed by the same procedures as in Example 2(2) to give the titled compound (632 mg) as a colorless amorphous mass.
MS(ESI/APCI Dual): 629(M+H)
¹H NMR (300 MHz, CHLOROFORM-d) δ ppm 0.90 - 1.02 (m, 6 H), 1.44 (s, 9 H), 1.61 - 1.80 (m, 10 H), 1.94 - 2.10 (m, 1 H), 2.35 - 2.46 (m, 1 H), 2.49 - 2.69 (m, 10 H), 2.79 - 2.91 (m, 1 H), 3.12 - 3.24 (m, 3 H), 5.24 - 5.35 (m, 1 H), 6.06 - 6.13 (m, 1 H), 6.69 - 6.78 (m, 1 H), 6.82 - 6.96 (m, 5 H), 7.05 - 7.12 (m, 1 H), 7.30 (d, J=8.4 Hz, 1 H), 7.95 (s, 1 H)

### (5) Synthesis of (2S)-5-[(tert-butoxycarbonyl)amino]-2-[(7-propyl-4,5-dihydroimidazo[1,5-a]quinolin-3-yl)methyl]pentanoic acid

To a solution of (1R,2S)-1-phenyl-2-(pyrrolidin-1-yl)propyl (2S)-5-(tert-butoxycarbonyl)amino]-2-[(7-propyl-4,5-dihydroimidazo[1,5-a]quinolin-3-yl)methyl]pentanoate (632 mg) in methanol (10 ml), 10% palladium-activated carbon (300 mg) was added and the mixture was stirred under hydrogen purge for 5 hours at 60 °C. The reaction mixture was filtered through Celite and the solvent was removed in vacuo. The resulting residue was purified by silica gel column chromatography (eluent: chloroform/methanol = 19:1 to 9:1) and subsequent recrystallization from ethyl acetate gave the titled compound (245 mg) as a colorless powder.
MS(ESI/APCI Dual): 442(M+H)
¹H NMR (300 MHz, CHLOROFORM-d) δ ppm 0.95 (t, J=7.3 Hz, 3 H), 1.41 (s, 9 H), 1.44 - 1.72 (m, 5 H), 1.72 - 1.86 (m, 1 H), 2.54 - 2.63 (m, 2 H), 2.73 - 2.94 (m, 7 H), 3.06 - 3.16 (m, 2 H), 4.58 - 4.68 (m, 1 H), 7.08 - 7.15 (m, 2 H), 7.29 - 7.35 (m, 1 H), 8.02 (s, 1 H)

### (6) Synthesis of (2S)-5-amino-2-[(7-propyl-4,5-dihydroimidazo[1,5-a]quinolin-3-yl)methyl]pentanoic acid dihydrochloride

To a solution of (2S)-5-[(tert-butoxycarbonyl)amino]-2-[(7-propyl-4,5-dihydroimidazo[1,5-a]quinolin-3-yl)methyl]pentanoic acid (120 mg) in ethyl acetate (8 ml), a solution of 4 M hydrochloric acid in ethyl acetate (4 ml) was added and the mixture was stirred for an hour at room temperature. The solvent was removed in vacuo and after adding ethyl acetate, decantation was performed. Water was added to the resulting residue and the solvent was removed in vacuo to give the titled compound (compound 30, 115 mg) as a colorless amorphous mass.
MS(ESI/APCI Dual): 342 (M+H)
¹H NMR (600 MHz, DEUTERIUM OXIDE) δ ppm 0.90 (t, J=7.3 Hz, 3 H), 1.57 - 1.73 (m, 6 H), 2.56 - 2.62 (m, 1 H), 2.63 (t, J=7.6 Hz, 2 H), 2.81 - 2.88 (m, 1 H), 2.91 - 3.05 (m, 7 H), 7.30 (d, J=8.3 Hz, 1 H), 7.33 (s, 1 H), 7.58 (d, J=8.3 Hz, 1 H), 9.11 (s, 1 H)

### Example 31

### Synthesis of methyl 5-amino-2-(4,5-dihydroimidazo[1,5-a]quinolin-3-ylmethyl)pentanoate dihydrochloride

To a solution of methyl 5-[(tert-butoxycarbonyl)amino]-2-(4,5-dihydroimidazo[1,5-a]quinolin-3-ylmethyl)pentanoate (348 mg) in ethyl acetate (5 ml), a solution of 4 M hydrochloric acid in ethyl acetate (5 ml) was added and the mixture was stirred for 18 hours at room temperature. The solvent was removed in vacuo to give the titled compound (compound 31, 327 mg) as a colorless amorphous mass.
MS(ESI/APCI Dual): 314 (M+H)
¹H NMR (300 MHz, CHLOROFORM-d) δ ppm 1.77 - 2.09 (m, 4 H), 2.91 - 3.24 (m, 9 H), 3.64 (s, 3 H), 7.28 - 7.34 (m, 2 H), 7.37 - 7.47 (m, 1 H), 8.12 (d, J=7.8 Hz, 1 H), 8.52 (br. s., 2 H), 10.36 (s, 1 H)

### Example 32

### Synthesis of ethyl (2S)-5-amino-2-(4,5-dihydroimidazo[1,5-a]quinolin-3-ylmethyl)pentanoate dihydrochloride

### (1) Synthesis of ethyl (2S)-5-E(tert-butoxycarbonyl)amino]-2-(4,5-dihydroimidazo[1,5-a]quinolin-3-ylmethyl)pentanoate

To a solution of (2S)-5-[(tert-butoxycarbonyl)amino]-2-(4,5-dihydroimidazo[1,5-a]quinolin-3-ylmethyl)pentanoic acid (200 mg) in chloroform (5.0 ml), ethanol (47 µl), N-[3-(dimethylamino}propyl]-N'-ethylcarbodiimide hydrochloride (144 mg) and 4-dimethylaminopyridine (6 mg) were added and the mixture was stirred for 20 hours at room temperature. To the reaction mixture, a saturated aqueous solution of sodium hydrogencarbonate was added and extraction was conducted with ethyl acetate. After washing the organic layer with brine, it was dried over anhydrous sodium sulfate. The solvent was concentrated in vacuo and the residue was purified by silica gel column chromatography (eluent: n-hexane/ethyl acetate = 9:1 through 1:1 to 0:1) to give the titled compound (230 mg) as a colorless oil.
MS(ESI/APCI Dual): 428 (M+H)
¹H NMR (300 MHz, CHLOROFORM-d) δ ppm 1.19 (t, J=7.1 Hz, 3 H), 1.42 (s, 9 H), 1.46 - 1.76 (m, 4 H), 2.64 - 2.76 (m, 1 H), 2.79 - 2.95 (m, 6 H), 3.04 - 3.17 (m, 2 H), 4.02 - 4.14 (m, 2 H), 4.69 - 4.82 (m, 1 H), 7.11 - 7.20 (m, 1 H), 7.25 - 7.33 (m, 2 H), 7.37 - 7.43 (m, 1 H), 7.94 (s, 1 H)

### (2) Synthesis of ethyl (2S)-5-amino-2-(4,5-dihydroimidazo[1,5-a]quinolin-3-ylmethyl)pentanoate dihydrochloride

To a solution of ethyl (2S)-5-[(tert-butoxycarbonyl)amino)-2-(4,5-dihydroimidazo[1,5-a]quinolin-3-ylmethyl)pentanoate (207 mg) in ethyl acetate (5 ml), a solution of 4 M hydrochloric acid in ethyl acetate (5 ml) was added and the mixture was stirred for 2 hours at room temperature. The solvent was removed in vacuo and an aqueous solution of 6 M hydrochloric acid was added to the resulting residue; the solvent was removed in vacuo to give the titled compound (compound 32, 148 mg) as a yellow amorphous mass.
MS(ESI/APCI Dual): 328 (M+H)
¹H NMR (300 MHz, DMSO-d₆) δ ppm 1.12 (t, J=7.1 Hz, 3 H), 1.52 - 1.66 (m, 4 H), 2.70 - 3.03 (m, 9 H), 4.04 (q, J=7.1 Hz, 2 H), 7.36 - 7.54 (m, 3 H), 7.88 - 7.94 (m , 1 H), 7.95 - 8.07 (m, 2 H), 9.84 (s, 1 H)

### Example 33

### Synthesis of isopropyl (2S)-5-amino-2-(4,5-dihydroimidazo[1,5-a]quinolin-3-ylmethyl)pentanoate dihydrochloride

### (1) Synthesis of isopropyl (2S)-5-[(tert-butoxycarbonyl)amino]-2-(4,5-dihydroimidazo[1,5-a]quinolin-3-ylmethyl)pentanoate

To a solution of (2S)-5-[(tert-butoxycarbonyl)amino]-2-(4,5-dihydroimidazo[1,5-a]quinolin-3-ylmethyl)pentanoic acid (200 mg) in chloroform (5.0 ml), isopropanol (62 µ). N-[3-(dimethylamino)propyl]-N'-ethylcarbadiimide hydrochloride (144 mg) and 4-dimethylaminopyridine (6 mg) were added and the mixture was stirred for 20 hours at room temperature. To the reaction mixture, a saturated aqueous solution of sodium hydrogencarbonate was added and extraction was conducted with ethyl acetate. After washing the organic layer with brine, it was dried over anhydrous sodium sulfate. The solvent was concentrated in vacuo and the residue was purified by silica gel column chromatography (eluent: n-hexane/ethyl acetate = 9:1 through 1:1 to 0:1) to give the titled compound (169 mg) as a pale yellow oil.
MS(ESI/APCI Dual): 442 (M+H)
¹H NMR (300 MHz, CHLOROFORM-d) δ ppm 1.12 (d, J=6.2 Hz, 3 H), 1.20 (d, J=6.2 Hz, 3 H), 1.42 (s, 9 H), 1.45 - 1.74 (m, 4 H), 2.63 - 2.73 (m, 1 H), 2.77 - 2.93 (m, 6 H), 3.04 - 3.17 (m, 2 H), 4.70 - 4.81 (m, 1 H), 4.85 - 5.01 (m, 1 H), 7.12 - 7.20 (m, 1 H), 7.25 - 7.33 (m, 2 H), 7.37 - 7.43 (m, 1 H), 7.94 (s, 1 H)

### (2) Synthesis of isopropyl (2S)-5-amino-2-(4,5-dihydroimidazo[1,5-a]quinolin-3-ylmethyl)pentanoate dihydrochloride

To a solution of isopropyl (2S)-5-[(tert-butoxycarbonyl)amino]-2-(4,5-dihydroimidazo[1,5-a]quinolin-3-ylmethyl)pentanoate (169 mg) in ethyl acetate (5 ml), a solution of 4 M hydrochloric acid in ethyl acetate (5 ml) was added and the mixture was stirred for 2 hours at room temperature. The solvent was removed in vacuo and an aqueous solution of 6 M hydrochloric acid was added to the resulting residue; the solvent was removed in vacuo to give the titled compound (compound 33, 119 mg) as a yellow amorphous mass. MS(ESI/APCI Dual): 342 (M+H)
¹H NMR (300 MHz, DMSO-d₆) δ ppm 1.05 (d, J=6.2 Hz, 3 H), 1.16 (d, J=6.2 Hz, 3 H), 1.45 - 1.69 (m, 4 H), 2.68 - 3.06 (m, 9 H), 4.84 (quin, J=6.2 Hz, 1 H), 7.36 - 7.54 (m, 3 H), 7.88 - 7.96 (m, 1 H), 7.98 - 8.13 (m, 2 H), 9.91 (s, 1 H)

### Example 34

### Synthesis of heptyl (2S)-5-amino-2-(4,5-dihydroimidazo[1,5-a]quinolin-3-ylmethyl)pentanoate dihydrochloride

### (1) Synthesis of heptyl (2S)-5-[(tert-butoxycarbonyl)amino]-2-(4,5-dihydroimidazo[1,5-a]quinolin-3-ylmethyl)pentanoate

To a solution of (2*S*)-5-[(tert-butoxycarbonyl)amino]-2-(4,5-dihydroimidazo[1,5-a]quinolin-3-ylmethyl)pentanoic acid (200 mg) in chloroform (5.0 ml), n-heptanol (93 mg), N-[3-(dimethylamino)propyl]-N'-ethylcarbodiimide hydrochloride (144 mg) and 4-dimethylaminopyridine (6 mg) were added and the mixture was stirred for 20 hours at room temperature. To the reaction mixture, a saturated aqueous solution of sodium hydrogencarbonate was added and extraction was conducted with ethyl acetate. After washing the organic layer with brine, it was dried over anhydrous sodium sulfate. The solvent was concentrated in vacuo and the residue was purified by silica gel column chromatography (eluent: n-hexane/ethyl acetate = 9:1 through 1:1 to 0:1) to give the titled compound (221 mg) as a pale yellow oil.
MS(ESI/APCI Dual): 498(M+H)
¹H NMR (300 MHz, CHLOROFORM-d) δ ppm 0.80 - 0.88 (m, 3 H), 1.10 - 1.32 (m, 8 H), 1.42 (s, 9 H), 1.46 - 1.73 (m, 6 H), 2.63 - 2.75 (m, 1 H), 2.79 - 2.94 (m, 6 H), 3.04 - 3.17 (m, 2 H), 3.95 - 4.05 (m, 2 H), 4.68 - 4.80 (m, 1 H), 7.11 - 7.19 (m, 1 H), 7.25 - 7.33 (m, 2 H), 7.37 - 7.42 (m, 1 H), 7.94 (s, 1 H)

### (2) Synthesis of heptyl (2S)-5-amino-2-(4,5-dihydroimidazo[1,5-a]quinolin-3-ylmethyl)pentanoate dihydrochloride

To a solution of heptyl (2S)-5-[(tert-butoxycarbonyl)amino]-2-(4,5-dihydroimidazo[1,5-a]quinolin-3-ylmethyl)pentanoate (221 mg) in ethyl acetate (5 ml), a solution of 4 M hydrochloric acid in ethyl acetate (5 ml) was added and the mixture was stirred for 2 hours at room temperature. The solvent was removed in vacuo and an aqueous solution of 6 M hydrochloric acid was added to the resulting residue; the solvent was removed in vacuo to give the titled compound (compound 34, 180 mg) as a yellow amorphous mass.
MS(ESI/APCI Dual): 398 (M+H)
¹H NMR (300 MHz, DMSO-d₆) δ ppm 0.75 - 0.83 (m, 3 H), 1.05 - 1.26 (m, 8 H), 1.36 - 1.50 (m, 2 H), 1.53 - 1.67 (m, 4 H), 2.70 - 3.03 (m, 9 H), 3.87 - 4.07 (m, 2 H), 7.35 - 7.54 (m, 3 H), 7.87 - 7.94 (m, 1 H), 7.95 - 8.09 (m, 2 H), 9.89 (s, 1 H)

### Example 35

### Synthesis of 1-{[(cyclohexyloxy)carbonyl]oxy}ethyl (2S)-_ 5-amino-2-(4,5-dihydroimidazo[1,5-a]quinolin-3-ylmethyl)pentanoate dihydrochloride

### (1) Synthesis of 1-{[(cyclohexyloxy)carbonyl]oxy)ethyl (2S)-5-[(tert-butoxycarbonyl)amino]-2-(4,5-dihydroimidazo[1,5-a]quinolin-3-ylmethyl)pentanoate

To a solution of (2S)-5-[(tert-butoxycarbonyl)amino]-2-(4,5-dihydroimidazo[1,5-a]quinolin-3-ylmethyl)pentanoic acid (400 mg) in N,N-dimethylformamide (3.3 ml), cyclohexyl 1-iodoethyl carbonate (597 mg) and cesium carbonate (489 mg) were added and the mixture was stirred for an hour at the same temperature. Afte adding water to the reaction mixture, extraction was conducted with ethyl acetate. The organic layer was dried over anhydrous sodium sulfate and the solvent was concentrated in vacuo. The resulting residue was purified by silica gel column chromatography (eluent: n-hexane/ethyl acetate = 1:4) to give the titled compound (366 mg) as a brown oil.
MS(ESI/APCI Dual): 570 (M+H)
¹H NMR (300 MHz, CHLOROFORM-d) δppm 1.15 - 1.98 (m, 14 H), 1.42 (s, 9 H), 1.48 (d, J=6.0 Hz, 3 H), 2.61 - 2.77 (m, 1 H), 2.77 - 2.99 (m, 5 H), 3.02 - 3.18 (m, 2 H), 4.40 - 4.68 (m, 1 H), 4.75 - 4.88 (m, 1 H), 6.66 - 6.78 (m, 1 H), 7.10 - 7.22 (m, 1 H), 7.24 - 7.35 (m, 2 H), 7.39 (d, J=8.4 Hz, 1 H), 7.926, 7.933 (s, 1 H)

### (2) Synthesis of 1-{[(cyclohexyloxy)carbonyl]oxy}ethyl (2S)-5-amino-2-(4,5-dihydroimidazo[1,5-a]quinolin-3-ylmethyl)pentanoate dihydrochloride

To a solution of 1-{[(cyclohexyloxy)carbonyl)oxy}ethyl(2S)-5-[(tert-butoxycarbonyl)amino]-2-(4,5-dihydroimidazo[1,5-a]quinolin-3-ylmethyl)pentanoate (183 mg) in ethyl acetate (3.2 ml), a solution of 4 M hydrochloric acid in ethyl acetate (3.2 ml) was added and the mixture was stirred for an hour at room temperature. The solvent was removed in vacuo and after adding water to the resulting residue, the solvent was removed in vacuo to give the titled compound (compound 35, 153 mg) as a pale brown amorphous mass.
MS(ESI/APCI Dual): 470 (M+H)
¹H NMR (600 MHz, METHANOL-d₃) δppm 1.06 - 1.89 (m, 17 H), 2.88 - 3.15 (m, 9 H), 4.08 - 4.15, 4.45 - 4.52 (m, 1 H), 6.51 - 6.55, 6.63 - 6.67 (m, 1 H), 7.41 - 7.53 (m, 3 H), 7.81 (d, J=7.3 Hz, 1 H), 9.61 (s, 1 H)
Optical purity: 99.84%ee
temp.: 25 °C
Mobile phase: n-Hexane:IPA:TFA:DEA = 85:15:0.5:0.5
r.t.: 12.38, 24.89 min

### Example 36

### Synthesis of (5-methyl-2-oxo-1,3-dioxol-4-yl)methyl (2S)-5-amino-2-(4,5-dihydroimidazo[1,5-a]quinolin-3-ylmethyl)pentanoate dihydrochloride

### (1) Synthesis of (5-methyl-2-oxo-1,3-dioxol-4-yl)methyl (2S)-5-[(tert-butoxycarbonyl)amino]-2-(4,5-dihydroimidazo[1,5-a]quinolin-3-ylmethyl)pentanoate

To a solution of (2S)-5-[(tert-butoxycarbonyl)amino]-2-(4,5-dihydroimidazo[1,5-a]quinolin-3-ylmethyl)pentanoic acid (500 mg) in N,N-dimethylformamide (4.2 ml), 4-(chloromethyl)-5-methyl-1,3-dioxol-2-one (279 mg) and cesium carbonate (613 mg) were added under cooling with ice and the mixture was stirred for an hour at the same temperature. Afte adding water to the reaction mixture, extraction was conducted with ethyl acetate. The organic layer was dried over anhydrous sodium sulfate and the solvent was removed in vacuo. The resulting residue was purified by silica gel column chromatography (eluent: n-hexane/ethyl acetate = 1:4) to give the titled compound (130 mg) as a colorless oil.
MS(ESI/APCI Dual): 512 (M+H)
¹H NMR (300 MHz, CHLOROFORM-d) δ ppm 1.42 (s, 9 H), 1.45 - 1.81 (m, 4 H), 2.14 (s, 3 H), 2.66 - 2.94 (m, 7 H), 3.03 - 3.21 (m, 2 H), 4.80 (s, 2 H), 7.12 - 7.22 (m, 1 H), 7.24 - 7.36 (m, 2 H), 7.37 - 7.46 (m, 1 H), 7.92 (s, 1 H)

### (2) Synthesis of (5-methyl-2-oxo-1,3-dioxol-4-yl)methyl (2S)-5-amino-2-(4,5-dihydroimidazo[1,5-a]quinolin-3-ylmethyl)pentanoate dihydrochloride

To a solution of (5-methyl-2-oxo-1,3-dioxol-4-yl)methyl (2S)-5-[(tert-butoxycarbonyl)amino]-2-(4,5-dihydroimidazo[1,5-a]quinolin-3-ylmethyl)pentanoate (130 mg) in ethyl acetate (2.5 ml), a solution of 4 M hydrochloric acid in ethyl acetate (2.5 ml) was added and the mixture was stirred for an hour at room temperature. The solvent was removed in vacuo and after adding water to the resulting residue, the solvent was removed in vacuo to give the titled compound (compound 36, 110 mg) as a yellow amorphous mass.
MS(ESI/APCI Dual): 412 (M+H)
¹H NMR (600 MHz, DEUTERIUM OXIDE) δppm 1.64 - 1.90 (m, 4 H), 2.06 (s, 3 H), 2.78 - 3.15 (m, 9 H), 4.82 (d, J=14.2 Hz, 1 H), 5.03 (d, J=14.2 Hz, 1 H), 7.36 - 7.54 (m, 3 H), 7.67 (d, J=8.3 Hz, 1 H), 8.99 (s, 1 H)
Optical purity: 97.65%ee
temp.: 25 °C
Mobile phase: n-Hexane:IPA:TFA:DEA = 80:20:0.5:0.5
r.t.: 28.29 min

### Example 37

### Synthesis of (2S)-2-(4,5-dihydroimidazo[1,5-a]quinolin-3-ylmethyl)-5-[(ethoxycarbonyl)amino]pentanoic acid

To a solution of (2S)-5-amino-2-(4,5-dihydroimidazo[1,5-a]quinolin-3-ylmethyl)pentanoic acid (200 mg) in N,N-dimethylformamide (7 ml), ethyl 4-nitrophenyl carbonate (144 mg) was added and the mixture was stirred for 22 hours at room temperature. After adding toluene to the reaction mixture, the solvent was concentrated in vacuo and the resulting residue was purified by silica gel column chromatography (eluent: chloroform/methanol = 9:1) to give the titled compound (compound 37, 162 mg) as a colorless powder. MS(ESI/APCI Dual): 372 (M+H)
¹H NMR (600 MHz, DMSO-d₆) δ ppm 1.10 (t, J=7.1 Hz, 3 H), 1.31 - 1.51 (m, 4 H), 2.52 - 2.64 (m, 2 H), 2.70 - 2.76 (m, 1 H), 2.78 - 2.81 (m, 2 H), 2.82 - 2.87 (m, 2 H), 2.87 - 2.96 (m, 2 H), 3.92 (q, J=7.1 Hz, 2 H), 7.03 (t, J=5.5 Hz, 1 H), 7.15 - 7.20 (m, 1 H), 7.30 - 7.34 (m, 1 H), 7.35 - 7.38 (m, 1 H), 7.68 - 7.72 (m, 1 H), 8.26 (s, 1 H)

### Example 38

### Synthesis of (2S)-5-({[1-_ (acetyloxy)ethoxy]carbonyl}amino)-2-(4,5-dihydroimidazo[1,5-a]quinolin-3-ylmethyl)pentanoic acid

To a solution of (2S)-5-amino-2-(4,5-dihydroimidazo[1,5-a]quinolin-3-ylmethyl)pentanoic acid (256 mg) in N,N-dimethylformamide (8.5 ml), 1-{[(4-nitrophenoxy)carbonyl]oxy}ethyl acetate (230 mg) was added and the mixture was stirred for 15 hours at room temperature. After adding brine to the reaction mixture, extraction was conducted with chloroform. The organic layer was dried over anhydrous sodium sulfate and the solvent was removed in vacuo. The resulting residue was purified by silica gel column chromatography (eluent: chloroform/methanol = 30:1 to 10:1) to give the titled compound (compound 38, 258 mg) as a pale yellow powder.
MS(ESI/APCI Dual): 430(M+H)
¹H NMR (600 MHz, DMSO-d₆) δppm 1.27 - 1.56 (m, 7 H), 1.99 (s, 3 H), 2.52 - 2.64 (m, 2 H), 2.65 - 3.03 (m, 7 H), 6.57 - 6.67 (m, 1 H), 7.15 - 7.20 (m, 1 H), 7.30 - 7.34 (m, 1 H), 7.35 - 7.38 (m, 1 H), 7.39 - 7.45 (m, 1 H), 7.65 - 7.75 (m, 1 H), 8.26 (s, 1 H)

### Example 39

### Synthesis of (2S)-2-(4,5-dihydroimidazo[1,5-a]quinolin-3-ylmethyl)-5-({[1-(propanoyloxy)ethoxy]carbonyl}amino)pentanoic acid

To a solution of (2S)-5-amino-2-(4,5-dihydroimidazo[1,5-a]quinolin-3-ylmethyl)pentanoic acid (150 mg) in N,N-dimethylformamide (5.0 ml), 1-{[(4-nitrophenoxy)carbonyl]oxy}ethyl propanoate (142 mg) was added and the mixture was stirred for 15 hours at room temperature. After adding brine to the reaction mixture, extraction was conducted with chloroform. The organic layer was dried over anhydrous sodium sulfate and the solvent was removed in vacuo. The resulting residue was purified by silica gel column chromatography (eluent: chloroform/methanol = 30:1 to 10:1) to give the titled compound (compound 39, 100 mg) as a pale yellow powder.
MS(ESI/APCI Dual): 444(M+H)
¹H NMR (600 MHz, DMSO-d₆) δ ppm 0.99 (t, J=7.3 Hz, 3 H), 1.28 - 1.57 (m, 7 H), 2.28 (q, J=7.3 Hz, 2 H), 2.52 - 2.65 (m, 2 H), 2.67 - 3.03 (m, 7 H), 6.61 - 6.67 (m, 1 H), 7.15 - 7.20 (m, 1 H), 7.30 - 7.35 (m, 1 H), 7.35 - 7.38 (m, 1 H), 7.39 - 7.44 (m, 1 H), 7.67 - 7.72 (m, 1 H), 8.26 (s, 1 H)

### Example 40

### Synthesis of (2S)-5-({[1-(butanoyloxy)ethoxy]carbonyl}amino)-2-(4,5-dihydroimidazo[1,5-a]quinolin-3-ylmethyl)pentanoic acid

To a solution of (2S)-5-amino-2-(4,5-dihydroimidazo[1,5-a]quinolin-3-ylmethyl)pentanoic acid (150 mg) in N,N-dimethylformamide (5.0 ml), 1-{[(4-nitrophenoxy)carbonyl]oxy}ethyl butanoate (149 mg) was added and the mixture was stirred for 15 hours at room temperature. After adding brine to the reaction mixture, extraction was conducted with chloroform. The organic layer was dried over anhydrous sodium sulfate and the solvent was removed in vacuo. The resulting residue was purified by silica gel column chromatography (eluent: chloroform/methanol = 30:1 to 10:1) to give the titled compound (compound 40, 101 mg) as a pale yellow powder.
MS(ESI/APCI Dual): 458 (M+H)
¹H NMR (600 MHz, DMSO-d₆) δ ppm 0.86 (t, J=7.3 Hz, 3 H), 1.27 - 1.58 (m, 9 H), 2.24 (t, J=7.1 Hz, 2 H), 2.53 - 2.64 (m, 2 H), 2.64 - 3.02 (m, 7 H), 6.58 - 6.72 (m, 1 H), 7.12 - 7.22 (m, 1 H), 7.27 - 7.39 (m, 2 H), 7.39 - 7.46 (m, 1 H), 7.65 - 7.73 (m, 1 H), 8.27 (s, 1 H)

### Example 41

### Synthesis of (2S)-2-(4,5-dihydroimidazo[1,5-a]quinolin-3-ylmethyl)-5-[({1-[(2-methylpropanoyl)oxy]ethoxy}carbonyl)amino]pentanoic acid

To a solution of (2S)-5-amino-2-(4,5-dihydroimidazo[1,5-a]quinolin-3-ylmethyl)pentanoic acid (212 mg) in N,N-dimethylformamide (7.1 ml), 1-{[(4-nitrophenoxy)carbonyl]oxy}ethyl 2-methylpropanoate (211 mg) was added and the mixture was stirred for 15 hours at room temperature. After adding brine to the reaction mixture, extraction was conducted with chloroform. The organic layer was dried over anhydrous sodium sulfate and the solvent was removed in vacuo. The resulting residue was purified by silica gel column chromatography (eluent: chloroform/methanol = 30:1) to give the titled compound (compound 41, 40 mg) as a colorless powder.
MS(ESI/APCI Dual): 458 (M+H)
¹H NMR (600 MHz, DMSO-d₆) δppm 0.97 - 1.12 (m, 6 H), 1.28 - 1.56 (m, 7 H), 2.41 - 2.64 (m, 3 H), 2.67 - 3.01 (m, 7 H), 6.57 - 6.68 (m, 1 H), 7.13 - 7.22 (m, 1 H), 7.28 - 7.39 (m, 2 H), 7.40 - 7.46 (m, 1 H), 7.65 - 7.74 (m, 1 H), 8.26 (s, 1 H)

### Example 42

### Synthesis of (2S)-5-[({1-[(cyclohexylcarbonyl)oxy]ethoxy}carbonyl}amino]-2-(4,5-dihydroimidazo[1,5-a]quinolin-3-ylmethyl)pentanoic acid

To a solution of (2S)-5-amino-2-(4,5-dihydroimidazo[1,5-a]quinolin-3-ylmethyl)pentanoic acid (150 mg) in N,N-dimethylformamide (5.0 ml), 1-{[(4-nitrophenoxy)carbonyl]oxy}ethyl cyclohexanecarboxylate (169 mg) was added and the mixture was stirred for 15 hours at room temperature. After adding brine to the reaction mixture, extraction was conducted with chloroform. The organic layer was dried over anhydrous sodium sulfate and the solvent was removed in vacuo. The resulting residue was purified by silica gel column chromatography (eluent: chloroform/methanol = 30:1 to 10:1) to give the titled compound (compound 42, 209 mg) as a pale yellow powder.
MS(ESI/APCI Dual): 498(M+H)
¹H NMR (600 MHz, DMSO-d₆) δ ppm 1.11 - 1.59 (m, 13 H), 1.59 - 1.70 (m, 2 H), 1.70 - 1.83 (m, 2 H), 2.20 - 2.34 (m, 1 H), 2.52 - 2.64 (m, 2 H), 2.66 - 3.02 (m, 7 H), 6.60 - 6.67 (m, 1 H), 7.15 - 7.21 (m, 1 H), 7.30 - 7.39 (m, 2 H), 7.39 - 7.45 (m, 1 H), 7.67 - 7.73 (m, 1 H), 8.26 (s, 1 H)

### Example 43

### Synthesis of (2S)-2-(4,5-dihydroimidazo[1,5-a]quinolin-3-ylmethyl)-5-[({1-[(phenylcarbonyl)oxy]ethoxy}carbonyl)amino]pentanoic acid

To a solution of (2S)-5-amino-2-(4,5-dihydroimidazo[1,5-a]quinolin-3-ylmethyl)pentanoic acid (150 mg) in N,N-dimethylformamide (5.0 ml), 1-{[(4-nitrophenoxy)carbonyl]oxy}ethyl benzoate (166 mg) was added and the mixture was stirred for 15 hours at room temperature. After adding brine to the reaction mixture, extraction was conducted with chloroform. The organic layer was dried over anhydrous sodium sulfate and the solvent was removed in vacuo. The resulting residue was purified by silica gel column chromatography (eluent: chloroform/methanol = 30:1 to 10:1) to give the titled compound (compound 43, 201 mg) as a colorless powder.
MS(ESI/APCI Dual): 492(M+H)
¹H NMR (600 MHz, DMSO-d₆) δppm 1.30 - 1.61 (m, 7 H), 2.51 - 2.63 (m, 2 H), 2.65 - 2.88 (m, 5 H), 2.88 - 3.04 (m, 2 H), 6.83 - 6.93 (m, 1H), 7.12 - 7.21 (m, 1 H), 7.26 - 7.40 (m, 2 H), 7.46 - 7.58 (m, 3 H), 7.62 - 7.73 (m, 2 H), 7.86 - 7.98 (m, 2 H), 8.26 (s, 1 H)

### Example 44

### Synthesis of (2S)-5-({[1-(acetyloxy)-2-methylpropoxy]carbonyl}amino)-2-(4,5-dihydroimidazo[1,5-a]quinolin-3-ylmethyl)pentanoic acid

To a solution of (2S)-5-amino-2-(4,5-dihydroimidazo[1,5-a]quinolin-3-ylmethyl)pentanoic acid (156 mg) in N,N-dimethylformamide (5 ml), 2-methyl-1-{[(4-nitrophenoxy)carbonyl]oxy}propyl acetate (154 mg) was added and the mixture was stirred overnight at room temperature. After adding water to the reaction mixture, extraction was conducted with ethyl acetate. The organic layer was dried over anhydrous sodium sulfate and the solvent was removed in vacuo. The resulting residue was purified by silica gel column chromatography (eluent: chloroform/methanol = 100:0 to 90:10) to give the titled compound (compound 48, 181 mg) as a colorless amorphous mass.
MS(ESI/APCI Dual): 458 (M+H)
¹H NMR (600 MHz, DMSO-d₆) δ ppm 0.82 - 0.89 (m, 6 H), 1.32 - 1.51 (m, 4 H), 1.85 - 1.93 (m, 1 H), 2.00, 2.01 (s, 3 H), 2.52 - 2.60 (m, 2 H), 2.69 - 2.75 (m, 1 H), 2.77 - 2.82 (m, 2 H), 2.82 - 2.87 (m, 2 H), 2.89 - 2.99 (m, 2 H), 6.39 - 6.42 (m, 1 H), 7.15 - 7.19 (m, 1 H), 7.30 - 7.34 (m, 1 H), 7.35 - 7.39 (m, 2 H), 7.67 - 7.72 (m, 1 H), 8.26 (s, 1 H)

### Example 45

### Synthesis of (2S)-2-(4,5-dihydroimidazo[1,5-a]quinolin-3-ylmethyl)-5-({[2-methyl-1-(propanoyloxy)propoxy]carbonyl}amino)pentanoic acid

### (1) Synthesis of 1-iodo-2-methylpropyl 4-nitrophenyl carbonate

To a solution of 1-chloro-2-methylpropyl 4-nitrophenyl carbonate (4.86 g) in toluene (100 ml), sodium iodide (10.7 g) and calcium chloride (7.90 g) were added and the mixture was heated under reflux for 9 hours. After adding water to the reaction mixture, extraction was conducted with ethyl acetate. The organic layer was dried over anhydrous sodium sulfate and the solvent was removed in vacuo to give the titled compound (6.26 g) as an unpurified brown oil.
¹H NMR (300 MHz, CHLOROFORM-d) δ ppm 1.05 - 1.15 (m, 6 H), 1.77 - 1.92 (m, 1 H), 6.75 (d, J=4.0 Hz, 1 H), 7.39 - 7.47 (m, 2 H), 8.28 - 8.34 (m, 2 H)

### (2) Synthesis of 2-methyl-1-{[(4-nitrophenoxy)carbonyl]oxy}propyl propanoate

To a solution of 1-iodo-2-methylpropyl 4-nitrophenyl carbonate (1.20 g) in toluene (10 ml), silver propionate (1.06 g) was added and the mixture was stirred for 2 hours at 90 °C. The reaction mixture was filtered through Celite and the filtrate was concentrated in vacuo. To the resulting residue, ethyl acetate was added and the mixture was washed with water and brine. After drying the organic layer over anhydrous sodium sulfate, the solvent was removed in vacuo. The resulting residue was purified by silica gel column chromatography (elulent: n-hexane/chloroform = 9:1 to 1:1) and by another run of silica gel column chromatography (elulent: n-hexane/ethyl acetate = 1:0 to 19:1) to give the titled compound (411 mg) as a pale yellow oil.
MS(ESI/APCI Dual): 334(M+Na)
¹H NMR (300 MHz, CHLOROFORM-d) δ ppm 1.06 (d, J=6.9 Hz, 6 H), 1.18 (t, J=7.5 Hz, 3 H), 2.15 (qd, J=6.9, 5.0 Hz, 1 H), 2.43 (q, J=7.5 Hz, 2 H), 6.60 (d, J=5.0 Hz, 1 H), 7.38 - 7.45 (m, 2 H), 8.25 - 8.32 (m, 2 H)

### (3) Synthesis of (2S)-2-(4,5-dihydroimidazo[1,5-a]quinolin-3-ylmethyl)-5-({[2-methyl-1-(propanoyloxy)propoxy]carbonyl}amino)pentanoic acid

To a solution of (2S)-5-amino-2-(4,5-dihydroimidazo[1,5-a]quinolin-3-ylmethyl)pentanoic acid (200 mg) in N,N-dimethylformamide (6 ml), 2-methyl-1-{[(4-nitrophenoxy)carbonyl]oxy}propyl propanoate (249 mg) was added and the mixture was stirred for 2 days at room temperature. After adding water to the reaction mixture, extraction was conducted with ethyl acetate. The organic layer was dried over anhydrous sodium sulfate and the solvent was removed in vacuo. The resulting residue was purified by silica gel column chromatography (eluent: chloroform/methanol = 97:3 to 90:10) to give the titled compound (compound 45, 198 mg) as a colorless amorphous mass.
MS(ESI/APCI Dual): 472(M+H)
¹H NMR (600 MHz, DMSO-d₆) δ ppm 0.82 - 0.90 (m, 6 H), 1.00 (t, J=7.6 Hz, 3 H), 1.33 - 1.51 (m, 4 H), 1.85 - 1.93 (m, 1 H), 2.24 - 2.35 (m, 2 H), 2.51 - 2.60 (m, 2 H), 2.70 - 2.75 (m, 1 H), 2.77 - 2.87 (m, 4 H), 2.89 - 2.98 (m, 2 H), 6.41 - 6.44 (m, 1 H), 7.15 - 7.19 (m, 1 H), 7.30 - 7.34 (m, 1 H), 7.35 - 7.38 (m, 2 H), 7.66 - 7.72 (m, 1 H), 8.26 (s, 1 H)

### Example 46

### Synthesis of (2S)-5-({[1-(butanoyloxy)-2-methylpropoxy]carbonyl}amino)-2-(4,5-dihydroimidazo[1,5-a]quinolin-3-ylmethyl)pentanoic acid

### (1) Synthesis of 2-methyl-1-{[(4-nitrophenoxy)carbonyl]oxy}propyl butanoate

To a solution of 1-iodo-2-methylpropyl 4-nitrophenyl carbonate (1.20 g) in toluene (10 ml), silver butanoate (1.14 g) was added and the mixture was stirred for 2 hours at 90 °C. The reaction mixture was filtered through Celite and the filtrate was concentrated in vacuo. To the resulting residue, ethyl acetate was added and the mixture was washed with water and brine. After drying the organic layer over anhydrous sodium sulfate, the solvent was removed in vacuo. The resulting residue was purified by silica gel column chromatography (elulent: n-hexane/chloroform = 9:1 to 1:1) and by another run of silica gel column chromatography (elulent: n-hexane/ethyl acetate = 1:0 to 19:1) to give the titled compound (518 mg) as a pale yellow oil.
MS(ESI/APCI Dual): 348 (M+Na)
¹H NMR (300 MHz, CHLOROFORM-d) δppm 0.98 (t, J=7.4 Hz, 3 H), 1.06 (d, J=6.8 Hz, 6 H), 1.70 (qt, J=7.4, 7.4 Hz, 2 H), 2.15 (qd, J=6.8, 5.0 Hz, 1 H), 2.38 (t, J=7.4 Hz, 2 H), 6.60 (d, J=5.0 Hz, 1 H), 7.38 - 7.45 (m, 2 H), 8.25 - 8.31 (m, 2 H)

### (2) Synthesis of (2S)-5-({[1-(butanoyloxy)-2-methylpropoxy]carbonyl}amino)-2-(4,5-dihydroimidazo[1,5-a]quinolin-3-ylmethyl)pentanoic acid

To a solution of (2S)-5-amino-2-(4,5-dihydroimidazo[1,5-a]quinolin-3-ylmethyl)pentanoic acid (200 mg) in N,N-dimethylformamide (6 ml), 2-methyl-1-{[(4-nitrophenoxy)carbonyl]oxy}propyl butanoate (260 mg) was added and the mixture was stirred for 2 days at room temperature. After adding water to the reaction mixture, extraction was conducted with ethyl acetate. The organic layer was dried over anhydrous sodium sulfate and the solvent was removed in vacuo. The resulting residue was purified by silica gel column chromatography (eluent: chloroform/methanol = 97:3 to 90:10) to give the titled compound (compound 46, 194 mg) as a colorless amorphous mass.
MS(ESI/APCI Dual): 486 (M+H)
¹H NMR (600 MHz, DMSO-d₆) δ ppm 0.82 - 0.90 (m, 9 H), 1.32 - 1.56 (m, 6 H), 1.85 - 1.93 (m, 1H), 2.26 (t, J=6.9 Hz, 2 H), 2.51 - 2.60 (m, 2 H), 2.69 - 2.75 (m, 1 H), 2.77 - 2.87 (m, 4 H), 2.89 - 2.97 (m, 2 H), 6.41 - 6.45 (m, 1H), 7.15 - 7.19 (m, 1H), 7.30 - 7.34 (m, 1 H), 7.35 - 7.39 (m, 2 H), 7.66 - 7.72 (m, 1H), 8.26 (s, 1H)

### Example 47

### Synthesis of (2S)-2-(4,5-dihydroimidazo[1,5-a]quinolin-3-ylmethyl)-5-[({2-methyl-1-[(2-_ methylpropanoyl)oxy]propoxy}carbonyl)amino]pentanoic acid

To a solution of (2S)-5-amino-2-(4,5-dihydroimidazo[1,5-a]quinolin-3-ylmethyl)pentanoic acid (245 mg) in N,N-dimethylformamide (8 ml), 2-methyl-1-{[(4-nitrophenoxy)carbonyl]oxy}propyl 2-methylpropanoate (318 mg) was added and the mixture was stirred overnight at room temperature. After adding water to the reaction mixture, extraction was conducted with ethyl acetate. The organic layer was dried over anhydrous sodium sulfate and the solvent was removed in vacuo. The resulting residue was purified by silica gel column chromatography (eluent: chloroform/methanol = 100:0 to 85:15) to give the titled compound (compound 47, 291 mg) as a colorless powder.
MS(ESI/APCI Dual): 486(M+H)
¹H NMR (600 MHz, DMSO-d₆) δ ppm 0.83 - 0.89 (m, 6 H), 1.03 - 1.09 (m, 6 H), 1.22 - 1.28 (m, 1H), 1.33 - 1.50 (m, 4 H), 1.86 - 1.94 (m, 1 H), 2.51 - 2.58 (m, 2 H), 2.70 - 2.76 (m, 1 H), 2.76 - 2.86 (m, 4 H), 2.89 - 2.96 (m, 2 H), 6.39 - 6.43 (m, 1 H), 7.15 - 7.19 (m, 1 H), 7.29 - 7.40 (m, 3 H), 7.65 - 7.72 (m, 1 H), 8.25 (s, 1 H)

### Example 48

### Synthesis of (2S)-5-[({1-[(cyclohexylcarbonyl)oxy]-2-methylpropoxy}carbonyl)amino]-2-(4,5-dihydroimidazo[1,5-a]quinolin-3-ylmethyl)pentanoic acid

### (1) Synthesis of 2-methyl-1-{[(4-nitrophenoxy)carbonyl]oxy}propyl cyclohexanecarboxylate

To a solution of 1-iodo-2-methylpropyl 4-nitrophenyl carbonate (1.55 g) in toluene (14 ml), silver cyclohexanecarboxylate (2.00 g) was added and the mixture was stirred for an hour at 90 °C. The reaction mixture was filtered through Celite and the filtrate was concentrated in vacuo. To the resulting residue, ethyl acetate was added and the mixture was washed with water and brine. After drying the organic layer over anhydrous sodium sulfate, the solvent was removed in vacuo. The resulting residue was purified by silica gel column chromatography (elulent: n-hexane/chloroform = 9:1 to 1:1) and by another run of silica gel column chromatography (elulent: n-hexane/ethyl acetate = 1:0 to 19:1) to give the titled compound (1.08 g) as a pale yellow oil.
MS(ESI/APCI Dual): 388(M+Na)
¹H NMR (300 MHz, CHLOROFORM-d) δ ppm 1.05 (d, J=6.8 Hz, 6 H), 1.20 - 1.38 (m, 3 H), 1.40 - 1.55 (m, 2 H), 1.59 - 1.70 (m, 1 H), 1.72 - 1.82 (m, 2 H), 1.89 - 2.01 (m, 2 H), 2.08 - 2.22 (m, 1H), 2.33 - 2.45 (m, 1H), 6.59 (d, J=5.0 Hz, 1H), 7.38 - 7.44 (m, 2 H), 8.24 - 8.32 (m, 2 H)

### (2) Synthesis of (2S)-5-[({1-[(cyclohexylcarbonyl)oxy]-2-methylpropoxy}carbonyl)amino]-2-(4,5-dihydroimidazo[1,5-a]quinolin-3-ylmethyl)pentanoic acid

To a solution of (2S)-5-amino-2-(4,5-dihydroimidazo[1,5-a]quinolin-3-ylmethyl)pentanoic acid (200 mg) in N,N-dimethylformamide (6 ml), 2-methyl-1-{[(4-nitrophenoxy)carbonyl]oxy}propyl cycloyhexanecarboxylate (292 mg) was added and the mixture was stirred for 2 days at room temperature. After adding water to the reaction mixture, extraction was conducted with ethyl acetate. The organic layer was dried over anhydrous sodium sulfate and the solvent was removed in vacuo. The resulting residue was purified by silica gel column chromatography (eluent: chloroform/methanol = 97:3 to 85:15) to give the titled compound (compound 48, 202 mg) as a colorless amorphous mass.
MS(ESI/APCI Dual): 526(M+H)
¹H NMR (600 MHz, DMSO-d₆) δ ppm 0.81 - 0.90 (m, 6 H), 1.12 - 1.49 (m, 10 H), 1.51 - 1.57 (m, 1H), 1.60 - 1.67 (m, 2 H), 1.71 - 1.82 (m, 2 H), 1.86 - 1.94 (m, 1H), 2.25 - 2.33 (m, 1 H), 2.53 - 2.59 (m, 1 H), 2.70 - 2.75 (m, 1 H), 2.77 - 2.81 (m, 2 H), 2.82 - 2.87 (m, 2 H), 2.90 - 2.95 (m, 2 H), 6.40 - 6.43 (m, 1H), 7.15 - 7.20 (m, 1H), 7.30 - 7.34 (m, 1H), 7.35 - 7.40 (m, 2 H), 7.66 - 7.73 (m, 1 H), 8.26 (s, 1 H)

### Example 49

### Synthesis of (2S)-2-(4,5-dihydroimidazo[1,5-a]quinolin-3-ylmethyl)-5-[({2-methyl-1-[(phenylcarbonyl)oxy]propoxy}carbonyl)amino]pentanoic acid

To a solution of (2S)-5-amino-2-(4,5-dihydroimidazo[1,5-a]quinolin-3-ylmethyl)pentanoic acid (114 mg) in N,N-dimethylformamide (10 ml), 2-methyl-1-{[(4-nitrophenoxy)carbonyl]oxy}propyl benzoate (136 mg) was added and the mixture was stirred overnight at room temperature. After adding water to the reaction mixture, extraction was conducted with ethyl acetate. The organic layer was dried over anhydrous sodium sulfate and the solvent was removed in vacuo. The resulting residue was purified by silica gel column chromatography (eluent: chloroform/methanol = 97:3 to 90:10) to give the titled compound (compound 49, 172 mg) as a pale pink amorphous mass.
MS(ESI/APCI Dual): 520 (M+H)
¹H NMR (600 MHz, DMSO-d₆) δ ppm 0.92 - 0.99 (m, 6 H), 1.34 - 1.49 (m, 4 H), 2.03 - 2.10 (m, 1 H), 2.52 - 2.60 (m, 2 H), 2.68 - 2.86 (m, 5 H), 2.89 - 2.98 (m, 2 H), 6.65 - 6.69 (m, 1 H), 7.14 - 7.20 (m, 1H), 7.29 - 7.37 (m, 2 H), 7.43 - 7.47 (m, 1 H), 7.52 - 7.57 (m, 2 H), 7.64 - 7.71 (m, 2 H), 7.91 - 7.97 (m, 2 H), 8.25 (s, 1H)

### Example 50

### Synthesis of (2S)-2-(4,5-dihydroimidazo[1,5-a]quinolin-3-ylmethyl)-5-({[(5-methyl-2-oxo-1,3-dioxol-4-yl)methoxy] carbonyl)amino)pentanoic acid

To a solution of (2S)-5-amino-2-(4,5-dihydroimidazo[1,5-a]quinolin-3-ylmethyl)pentanoic acid (500 mg) in N,N-dimethylformamide (16.7 ml), (5-methyl-2-oxo-1,3-dioxol-4-yl)methyl 4-nitrophenyl carbonate (493 mg) was added and the mixture was stirred for 16 hours at room temperature. After adding brine to the reaction mixture, extraction was conducted with chloroform. The organic layer was dried over anhydrous sodium sulfate and the solvent was removed in vacuo. The resulting residue was purified by silica gel column chromatography (eluent: chloroform/methanol = 20:1) to give the titled compound (compound 50, 467 mg) as a colorless amorphous mass.
MS(ESI/APCI Dual): 456 (M+H)
¹H NMR (600 MHz, DMSO-d₆) δppm 1.30 - 1.58 (m, 4 H), 2.13 (s, 3 H), 2.52 - 2.64 (m, 2 H), 2.66 - 2.76 (m, 1 H), 2.76 - 2.89 (m, 4 H), 2.90 - 2.98 (m, 2 H), 4.82 (s, 2 H), 7.14 - 7.21 (m, 1 H), 7.27 - 7.42 (m, 3 H), 7.70 (d, J=7.8 Hz, 1 H), 8.26 (s, 1 H)
Optical purity: 99.87%ee
temp.: 10 °C
Mobile phase: n-Hexane:IPA:TFA:DEA = 80:20:0.5:0.5
r.t.: 34.06 min

### Example 51

### Synthesis of (2S)-2-(4,5-dihydroimidazo[1,5-a]quinolin-3-ylmethyl)-5-({[(2-oxo-5-phenyl-1,3-dioxol-4-yl)methoxy] carbonyl}amino)pentanoic acid

To a solution of (2S)-5-amino-2-(4,5-dihydroimidazo[1,5-a]quinolin-3-ylmethyl)pentanoic acid (150 mg) in N,N-dimethylformamide (5.0 ml), 4-nitrophenyl (2-oxo-5-phenyl-1,3-dioxol-4-yl)methyl carbonate (179 mg) was added and the mixture was stirred for 16 hours at room temperature. After adding brine to the reaction mixture, extraction was conducted with chloroform. The organic layer was dried over anhydrous sodium sulfate and the solvent was removed in vacuo. The resulting residue was purified by silica gel column chromatography (eluent: chloroform/methanol = 20:1) to give the titled compound (compound 51, 125 mg) as a colorless amorphous mass.
MS(ESI/APCI Dual): 518(M+H)
¹H NMR (600 MHz, DMSO-D₆) ppm 1.30 - 1.58 (m, 4 H), 2.52 - 2.65 (m, 2 H), 2.67 - 2.91 (m, 5 H), 2.91 - 3.05 (m, 2 H), 5.08 (s, 2 H), 7.13 - 7.20 (m, 1 H), 7.27 - 7.40 (m, 2 H), 7.45 - 7.61 (m, 4 H), 7.61 - 7.74 (m, 3 H), 8.26 (s, 1 H)

### Example 52

### Synthesis of (2S)-2-(4,5-dihydroimidazo[1,5-a]quinolin-3-ylmethyl)-5-[({[2-oxo-5-(propan-2-yl)-1,3-dioxol-4-yl]methoxy}carbonyl)amino]pentanoic acid

### (1) Synthesis of 4-nitrophenyl [2-oxo-5-(propan-2-yl)-1,3-dioxol-4-yl]methyl carbonate

To a solution of 4-(hydroxymethyl)-5-isopropyl-1,3-dioxol-2-one (450 mg) in chloroform (9.5 ml), pyridine (248 mg) and 4-nitrophenyl chlorocarbonate (631 mg) were added under cooling with ice and the mixture was stirred for 3 hours at room temperature. To the reaction mixture, an aqueous solutioln of 1 M hydrochloric acid was added and after extraction with chloroform, the organic layer was washed with brine. After drying the organic layer over anhydrous sodium sulfate, the solvent was removed in vacuo. The resulting residue was purified by silica gel column chromatography (eluent: n-hexane/ethyl acetate = 4:1) to give the titled compound (416 mg) as a colorless oil.
¹H NMR (300 MHz, CHLOROFORM-d) δ ppm 1.27 (d, J=7.0 Hz, 6 H), 2.90 - 3.05 (m, 1 H), 5.06 (s, 2 H), 7.35 - 7.45 (m, 2 H), 8.26 - 8.36 (m, 2 H)

### (2) Synthesis of (2S)-2-(4,5-dihydroimidazo[1,5-a]quinolin-3-ylmethyl)-5-[({[2-oxo-5-(propan-2-yl)-1,3-dioxol-4-yl]methoxy}carbonyl)amino]pentanoic acid

To a solution of (2S)-5-amino-2-(4,5-dihydroimidazo[1,5-a]quinolin-3-ylmethyl)pentanoic acid (150 mg) in N,N-dimethylformamide (5.0 ml), 4-nitrophenyl [2-oxo-5-(propan-2-yl)-1,3-dioxol-4-yl]methyl carbonate (162 mg) was added and the mixture was stirred for 16 hours at room temperature. After adding brine to the reaction mixture, extraction was conducted with chloroform. The organic layer was dried over anhydrous sodium sulfate and the solvent was removed in vacuo. The resulting residue was purified by silica gel column chromatography (eluent: chloroform/methanol = 20:1) to give the titled compound (compound 52, 90 mg) as a colorless amorphous mass.
MS(ESI/APCI Dual): 484(M+H)
¹H NMR (600 MHz, DMSO- d₆) δ ppm 1.14 (d, J=6.9 Hz, 6 H), 1.31 - 1.53 (m, 4 H), 2.52 - 2.61 (m, 2 H), 2.68 - 2.76 (m, 1 H), 2.76 - 2.82 (m, 2 H), 2.82 - 2.88 (m, 2 H), 2.89 - 2.98 (m, 2 H), 2.99 - 3.08 (m, 1 H), 4.83 (s, 2 H), 7.13 - 7.21 (m, 1 H), 7.28 - 7.41 (m, 3 H), 7.69 (d, J=7.8 Hz, 1 H), 8.26 (s, 1 H)

### Example 53

### Synthesis of (2S)-2-(4,5-dihydroimidazo[1,5-a]quinolin-3-ylmethyl)-5-({[(2-oxa-5-propyl-1,3-dioxol-4-yl)methoxy]carbonyl}amino)pentanoic acid

### (1) Synthesis of 4-nitrophenyl (2-oxo-5-propyl-1,3-dioxol-4-yl)methyl carbonate

To a solution of 4-(hydroxymethyl)-5-propyl-1,3-dioxol-2-one (380 mg) in chloroform (8.0 ml), pyridine (209 mg) and 4-nitrophenyl chlorocarbonate (532 mg) were added under cooling with ice and the mixture was stirred for 3 hours at room temperature. To the reaction mixture, an aqueous solutioln of 1 M hydrochloric acid was added and after extraction with chloroform, the organic layer was washed with brine. After drying the organic layer over anhydrous sodium sulfate, the solvent was removed in vacuo. The resulting residue was purified by silica gel column chromatography (eluent: n-hexane/ethyl acetate = 4:1) to give the titled compound (750 mg) as a colorless oil.
¹H NMR (300 MHz, CHLOROFORM-d) δ ppm 0.99 (t, J=7.4 Hz, 3 H), 1.60 - 1.75 (m, 2 H), 2.52 (t, J=7.4 Hz, 2 H), 5.05 (s, 2 H), 7.36 - 7.45 (m, 2 H), 8.27 - 8.35 (m, 2 H)

### (2) Synthesis of (2S)-2-(4,5-dihydroimidazo[1,5-a]quinolin-3-ylmethyl)-5-({[(2-oxo-5-propyl-1,3-dioxol-4-yl)methoxy]carbonyl}amino)pentanoic acid

To a solution of (2S)-5-amino-2-(4,5-dihydroimidazo[1,5-a]quinolin-3-ylmethyl)pentanoic acid (150 mg) in N,N-dimethylformamide (5.0 ml), 4-nitrophenyl (2-oxo-5-propyl-1,3-dioxol-4-yl)methyl carbonate (162 mg) was added and the mixture was stirred for 15 hours at room temperature. To the reaction mixture, water and diethyl ether were added and the precipitating powder was recovered by filtration. The powder was then washed with water and diethyl ether to give the titled compound (compound 53, 151 mg) as a colorless powder. MS(ESI/APCI Dual): 484 (M+H)
¹H NMR (600 MHz, DMSO-d₆) δ ppm 0.88 (t, J=7.6 Hz, 3 H), 1.32 - 1.56 (m, 6 H), 2.46 - 2.60 (m, 4 H), 2.68 - 2.76 (m, 1 H), 2.77 - 2.82 (m, 2 H), 2.82 - 2.88 (m, 2 H), 2.90 - 2.98 (m, 2 H), 4.83 (s, 2 H), 7.14 - 7.21 (m, 1 H), 7.29 - 7.40 (m, 3 H), 7.67 - 7.72 (m, 1 H), 8.26 (s, 1 H)

### Example 54

### Synthesis of (2S)-5-({[(5-tert-butyl-2-oxo-1,3-dioxol-4-yl)methoxy]carbonyl}amino)-2-(4,5-dihydroimidazo[1,5-a]quinolin-3-ylmethyl)pentanoic acid

### (1) Synthesis of (5-tert-butyl-2-oxo-1,3-dioxol-4-yl)methyl 4-nitrophenyl carbonate

To a solution of 4-tert-butyl-5-(hydroxymethyl)- 1,3-dioxol-2-one (400 mg) in chloroform (5 ml), pyridine (210 µL) and 4-nitrophenyl chlorocarbonate (516 mg) were added under cooling with ice and the mixture was stirred for 19 hours at room temperature. To the reaction mixture, an aqueous solutioln of 1 M hydrochloric acid was added and after two extractions with ethyl acetate, the organic layer was dried over anhydrous magnesium sulfate and the solvent was then removed in vacuo. The resulting residue was purified by silica gel column chromatography (eluent: n-hexane/ethyl acetate = 80:20) and subsequently by another run of silica gel column chromatography (eluent: n-hexane/ethyl acetate = 85:15) to give the titled compound (490 mg) as a colorless powder. MS(ESI/APCI Dual): 360 (M+Na)
¹H NMR (300 MHz, CHLOROFORM-d) δ ppm 1.35 (s, 9 H), 5.17 (s, 2 H), 7.38 - 7.45 (m, 2 H), 8.28 - 8.38 (m, 2 H)

### (2) Synthesis of (2S)-5-({[(5-tert-butyl-2-oxo-1,3-dioxol-4-yl)methoxy]carbonyl)amino)-2-(4,5-dihydroimidazo[1,5-a]quinolin-3-ylmethyl)pentanoic acid

To a solution of (2S)-5-amino-2-(4,5-dihydroimidazo[1,5-a]quinolin-3-ylmethyl)pentanoic acid (153 mg) in N,N-dimethylformamide (5 ml), (5-tert-butyl-2-oxo-1,3-dioxol-4-yl)methyl 4-nitrophenyl carbonate (210 mg) was added and the mixture was stirred for 46 hours at room temperature. After adding toluene to the reaction mixture, the solvent was removed in vacuo. The resulting residue was purified by silica gel column chromatography (eluent: chloroform/methanol = 95:5 to 90:10), subsequently by another run of silica gel column chromatography (eluent: chloroform/methanol = 93:7 to 90:10) to give the titled compound (compound 54, 160 mg) as a colorless amorphous.
MS(ESI/APCI Dual): 498 (M+H)
¹H NMR (600 MHz, CHLOROFORM-d) δ ppm 1.30 (s, 9 H), 1.49 - 1.57 (m, 1 H), 1.58 - 1.68 (m, 2 H), 1.73 - 1.83 (m, 1 H), 2.72 - 2.83 (m, 2 H), 2.85 - 2.96 (m, 5 H), 3.15 - 3.24 (m, 2 H), 4.90 (s, 2 H), 5.22 - 5.28 (m, 1 H), 7.18 - 7.22 (m, 1 H), 7.28 - 7.34 (m, 2 H), 7.39 - 7.44 (m, 1 H), 8.11 (s, 1 H)

### Example 55

### Synthesis of (2S)-2-(4,5-dihydroimidazo[1,5-a]quinolin-3-ylmethyl)-5-[({1-[(3-methylbutanoyl)oxy]ethoxy}carbonyl)amino]pentanoic acid

### (1) Synthesis of 1-{[(4-nitrophenoxy)carbonyl]oxy)ethyl 3-methylbutanoate

To a solution of 1-iodoethyl 4-nitrophenyl carbonate (1.88 g) in toluene (18.6 ml), silver isobutanoate (2.33 g) was added and the mixture was stirred for 3 hours at 70 °C. The reaction mixture was filtered and water was added to the filtrate; after three extractions with ethyl acetate, the organic layer was washed with brine. After drying the organic layer over anhydrous sodium sulfate, the solvent was removed in vacuo. The resulting residue was purified by silica gel column chromatography (elulent: n-hexane/chloroform = 1:1 to n-hexane/ethyl acetate = 4:1) to give the titled compound (640 mg) as a pale yellow oil.
¹H NMR (300 MHz, CHLOROFORM-d) δ ppm 0.98 (d, J=6.7 Hz, 6 H), 1.62 (d, J=5.4 Hz, 3 H), 2.08 - 2.21 (m, 1H), 2.24 - 2.29 (m, 2 H), 6.86 (q, J=5.4 Hz, 1 H), 7.36 - 7.47 (m, 2 H), 8.24 - 8.35 (m, 2 H)

### (2) Synthesis of (2S)-2-(4,5-dihydroimidazo[1,5-a]quinolin-3-ylmethyl)-5-[({1-[(3-methylbutanoyl)oxy]ethoxy}carbonyl)amino]pentanoic acid

To a solution of (2S)-5-amino-2-(4,5-dihydroimidazo[1,5-a]quinolin-3-ylmethyl)pentanoic acid (150 mg) in N,N-dimethylformamide (5.0 ml), 1-{[(4-nitrophenoxy)carbonyl]oxy}ethyl 3-methylbutanoate (156 mg) was added and the mixture was stirred for 15 hours at room temperature. Water was added to the reaction mixture and after three extractions with ethyl acetate, the organic layer was washed with brine. After drying the organic layer over anhydrous sodium sulfate, the solvent was removed in vacuo. The resulting residue was purified by silica gel column chromatography (elulent: chloroform/methanol = 100:0 to 97:3) to give the titled compound (compound 55, 200 mg) as a colorless amorphous mass.
MS(ESI/APCI Dual): 472 (M+H)
¹H NMR (600 MHz, DMSO-d₆) δ ppm 0.89 (d, J=6.9 Hz, 6 H), 1.28 - 1.55 (m, 7 H), 1.89 - 2.00 (m, 1 H), 2.08 - 2.20 (m, 2 H), 2.52 - 2.63 (m, 2 H), 2.67 - 2.88 (m, 5 H), 2.88 - 3.01 (m, 2 H), 6.60 - 6.71 (m, 1 H), 7.12 - 7.22 (m, 1 H), 7.28 - 7.40 (m, 2 H), 7.40 - 7.48 (m, 1 H), 7.70 (d, J=7.8 Hz, 1 H), 8.27 (s, 1 H)

The structural formulas of compounds 1-55 prepared in the Examples of the present invention are shown in the following Tables 1-1, 1-2, and 1-3.

### [Table 1-1]

**Table 1-1**

| Ex. No. | structure | salt | Ex. No. | structure | salt |
|---|---|---|---|---|---|
| 1 | 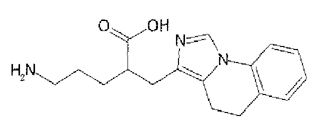 | - | 11 | 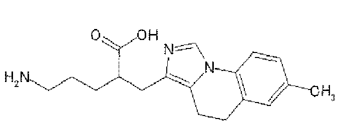 | 2HCl |
| 2 | 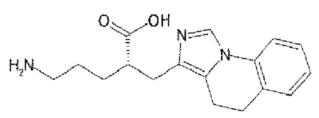 | - | 12 | 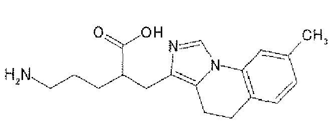 | - |
| 3 | 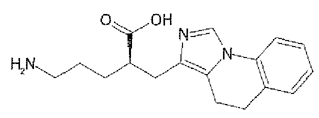 | - | 13 | 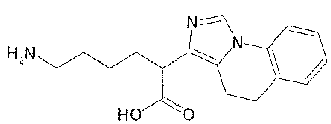 | - |
| 4 | 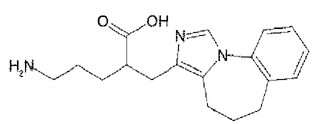 | - | 14 | 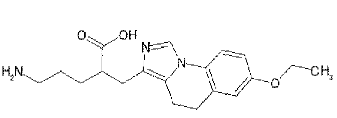 | - |
| 5 | 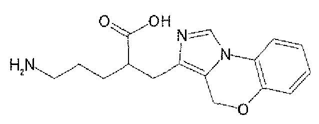 | - | 15 | 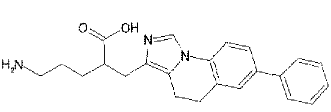 | 2HCl |
| 6 | 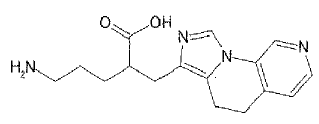 | - | 16 | 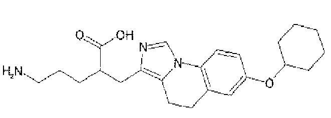 | - |
| 7 | 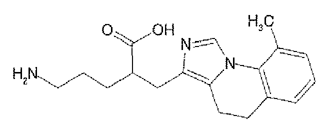 | 2HCl | 17 | 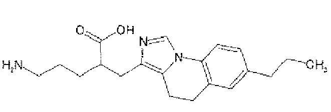 | 2HCl |
| 8 | 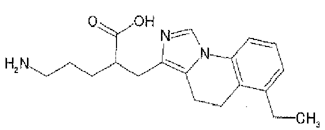 | - | 18 | 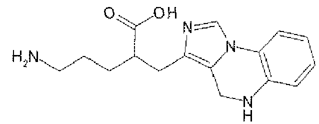 | - |
| 9 | 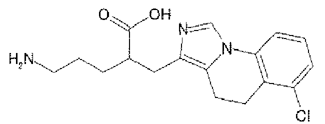 | - | 19 | 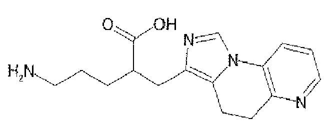 | - |
| 10 | 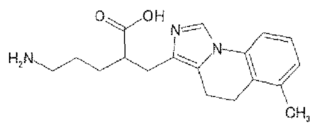 | - | 20 | 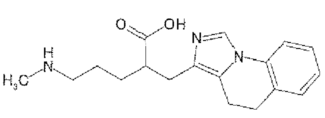 | - |

### [Table 1-2]

**Table 1-2**

| Ex. No. | structure | salt | Ex. No. | structure | salt |
|---|---|---|---|---|---|
| 21 | 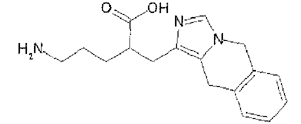 | - | 31 | 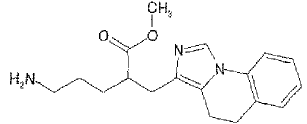 | 2HCl |
| 22 | 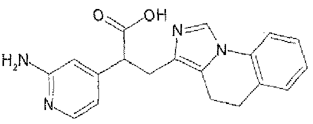 | 2HCl | 32 | 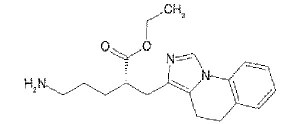 | 2HCl |
| 23 | 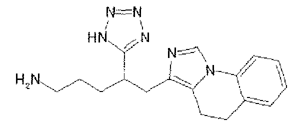 | - | 33 | 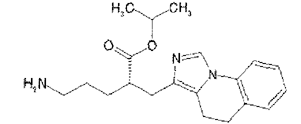 | 2HCl |
| 24 | 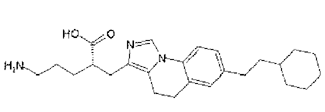 | 2HCl | 34 | 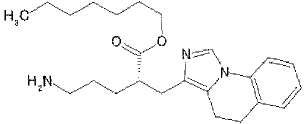 | 2HCl |
| 25 | 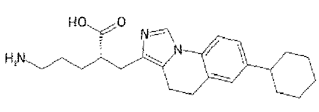 | 2HCl | 35 | 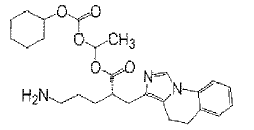 | 2HCl |
| 26 | 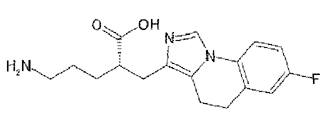 | 2HCl | 36 | 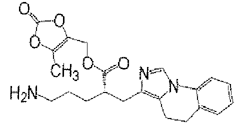 | 2HCl |
| 27 | 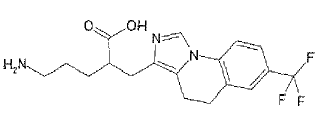 | 2HCl | 37 | 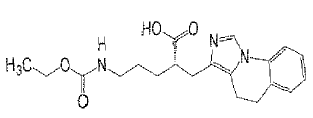 | - |
| 28 | 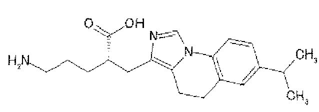 | 2HCl | 38 | 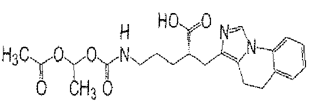 | - |
| 29 | 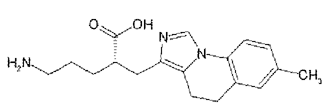 | 2HCl | 39 | 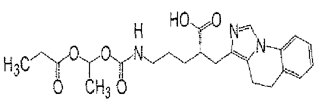 | - |
| 30 | 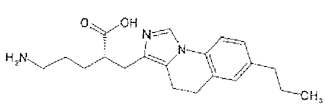 | 2HCl | 40 | 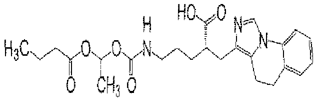 | - |

### [Table 1-3]

**Table 1-3**

| Ex. No. | structure | salt | Ex. No. | structure | salt |
|---|---|---|---|---|---|
| 41 | 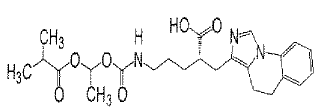 | - | 51 | 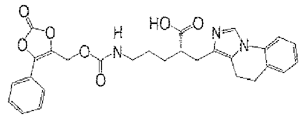 | |
| 42 | 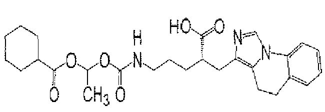 | - | 52 | 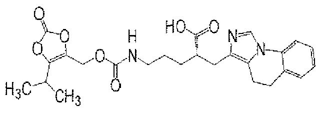 | |
| 43 | 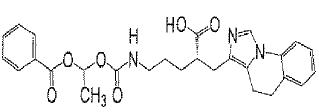 | - | 53 | 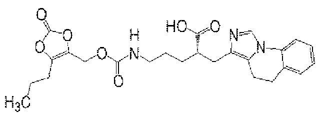 | |
| 44 | 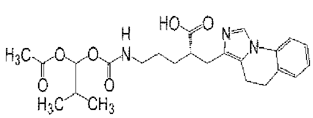 | - | 54 | 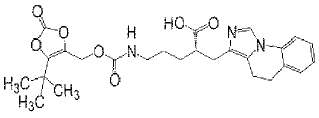 | |
| 45 | 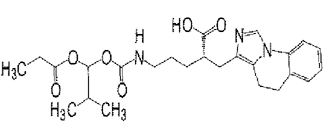 | - | 55 | 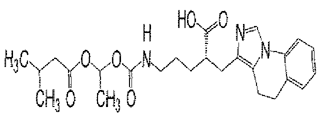 | |
| 46 | 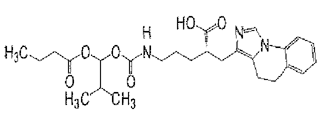 | - | | | |
| 47 | 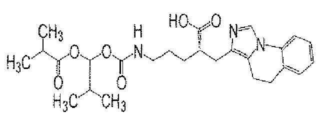 | - | | | |
| 48 | 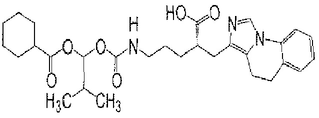 | - | | | |
| 49 | 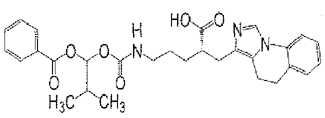 | - | | | |
| 50 | 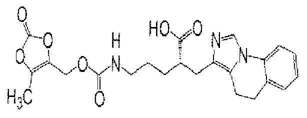 | - | | | |

### Test 1 [TAPIa Inhibition Test]

Compounds of the present invention were measured for their TAFIa inhibitory activity as follows based on the method described in Thromb. Haemost. 371 (1988).

### (a) Preparation of TAPIa solution

To 450 µl of TAFI (the product of Enzyme Research Laboratories whose concentration was adjusted to 18 µg/ml with buffer A: 100 mM Tris-HCl buffer , pH 7.4), 45 µl of a thrombomodulin solution (a rabbit lung derived thrombomodulin produced by American Diagnostia whose concentration was adjusted to 1 µg/ml with buffer B: 50 mM Tris-HCl, pH 7.5, containing 0.15 M NaCl) and 45 µl of a thrombin solution (a freeze-dried human plasma derived thrombin produced by Sigma and dissolved in water to have a concentration of 30 µ/ml) were added and the mixture was left to stand for 25 minutes at room temperature.

### (b) Method of measuring TAFIa inhibitory activity

To wells on a 96-well microplate, the above-prepared TAFIa solution, a test compound, and a substrate solution (Hip-Arg produced by Sigma and dissolved in buffer C of 100 mM Tris-HCl buffer, pH 8.3, to have a concentration of 3.6 mM) were added in respective amounts of 20 µl/well, 10 µl/well, and 70 µl/well. The individual components were mixed well and the reaction was carried out for 40 minutes at room temperature.
Subsequently, a color former (1% cyanuric chloride in 1,4-dioxane) was added in an amount of 50 µl to each well and the plate was left to stand for 3 minutes at room temperature; then, the absorbance at 405 nm was measured with a microplate reader (Spectramax M2 of Molecular Devices). With the absorbance in the absence of a test compound minus the absorbance in the absence of an enzyme being taken as 100%, the concentration of the compound that inhibited 50% of the reaction (IC₅₀) was calculated from the absorbance in the presence of the test compound minus the absorbance in the absence of the enzyme.
For several compounds of the present invention, the above test was conducted and on the basis of the results of measurements, TAFIa inhibitory activity was calculated to give the results shown in Table 2.

### [Table 2]

**Table 2**

| Ex. No. | IC₅₀ (nM) |
|---|---|
| 1 | 95 |
| 2 | 63 |
| 3 | 860 |
| 4 | 3300 |
| 5 | 1100 |
| 6 | 1600 |
| 7 | 6400 |
| 8 | 270 |
| 9 | 190 |
| 10 | 150 |
| 11 | 61 |
| 12 | 1800 |
| 13 | 3300 |
| 14 | 110 |
| 15 | 140 |
| 16 | 180 |
| 17 | 67 |
| 18 | 630 |
| 19 | 580 |
| 20 | 2300 |
| 21 | 1400 |
| 22 | 5100 |
| 23 | 4400 |
| 24 | 43 |
| 25 | 28 |
| 26 | 72 |
| 27 | 410 |
| 28 | 33 |
| 29 | 25 |
| 30 | 28 |

### Test 2 [Measurement of in vivo exposure based on plasma level in rats]

To determine the in vivo exposure, compounds 38, 41 amd 50 as exemplary prodrug compounds of the present invention and compound 2 as their parent compound were orally administered to rats and the plasma level of compound 2 was measured as described below for comparative purposes.
Seven-week old rats (200-260 g; male; lineage; Crl:CD (SD)) purchased from Charles River Laboratories Japan Inc. were acclimatized for at least two days before they were administered with compounds of the present invention. The compounds of the present invention were dissolved in various administration solvents at a concentration equivalent to 2 mg/mL as calculated for the parent compound 2 and they were then administered orally in an amount equivalent to 10 mg/kg of that parent compound. Half an hour and two hours later, blood was taken from the tail vein of each rat through a blood collecting tube (EDTA loaded) and immediately centrifuged (10,000 x g at 4 °C for 3 minutes) to recover plasma samples, which were stored frozen at -70 °C and below. After thawing the plasma samples under cooling on ice, a solution of internal standard substance in a 9:1 mixture of acetonitrile and methanol was added, followed by deproteinization and centrifugation (3600 x g at 4 °C for 10 minutes). The concentration of the parent compound 2 in the supernatant was measured by LC/MS/MS.
As the following Table 3 summarizes, the administration of the prodrug compounds of the present invention showed higher plasma levels of the parent compound 2, indicating higher in vivo exposures of that parent compound. Therefore, it is believed that by administering the prodrug compounds of the present invention, the physiological action of the parent compound will be exhibited more effectively than when theparent compound is administered on its own.

### [Table 3]

**Table 3**

| Comparison of Plasma Levels of Compound 2 (Parent Compound) for Various Compounds of the Present Invention | | |
|---|---|---|
| Compound | Plasma Level of Compound 2 (Parent Compound) | |
| (10 mg/kg p.o.) | (ng/mL) | |
| | 0.5 hrs later | 2 hrs later |
| Compound 2 | 173 | 118 |
| (parent compound) | | |
| Compound 38 | 2020 | 377 |
| Compound 41 | 1390 | 439 |
| Compound 50 | 1310 | 328 |

Administration solvent for compound 2: physiological saline Administration solvent for compounds 38, 41 and 50: 0.01 M HCl in water
Internal standard substance for compound 2: midazolam (Wako Pure Chemical Industries, Ltd.)
Internal standard substance for compounds 38, 41 and 50: compound 24 of the present invention

### INDUSTRIAL APPLICABILITY

The present invention provides pharmaceuticals that have sufficiently high TAPIa inhibitory activity to be effective for preventing or treating clot-derived diseases and the like, and it is therefore expected to relieve the burden on patients and contribute to the progress of the pharmaceutical industry.

## Claims

1. A compound represented by the following formula (I), or a pharmaceutically acceptable salt thereof: 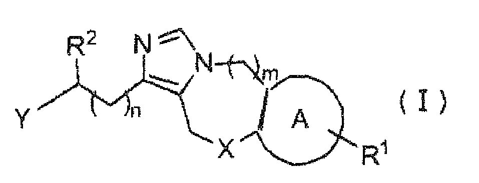
wherein A is a benzene ring or a pyridine ring;
X is the formula -(CH₂)-, the formula -(CH₂)₂-, an oxygen atom, a nitrogen atom or a single bond;
Y is the formula -(CH₂)₃-NH-R³, the formula -(CH₂)₄-NH-R³ or a 2-aminopyridyl group;
R³ is a hydrogen atom, a C₁₋₆ alkyl group, or the formula -CO₂R⁴;
R⁴ is a C₁₋₆ alkyl group, the formula -CHR⁵OC(O)R⁶, or a substituent having the structure represented by the following formula Ia; 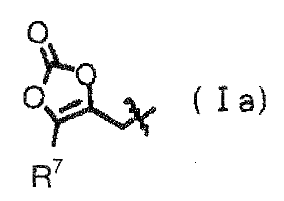
R⁵ is a C₁₋₆ alkyl group;
R⁶ is a C₁₋₆ alkyl group, a C₃₋₈ cycloalkyl group, or a phenyl group;
R⁷ is a C₁₋₆ alkyl group or a phenyl group;
R¹ is a hydrogen atom, a halogen atom, a C₁₋₄ alkyl group substituted by 1-3 halogen atoms, a C₁₋₁₀ alkyl group, a C₁₋₈ alkoxy group, a C₃₋₈ cycloalkyl group, a C₃₋₈ cycloalkoxy group, a C₄₋₁₄ cycloalkylalkyl group, or a phenyl group;
R² is CO₂R⁸, or a tetrazolyl group;
R⁸ is a hydrogen atom, a C₁₋₁₀ alkyl group, or a substituent having the structure represented by the following formula Ib or Ic: 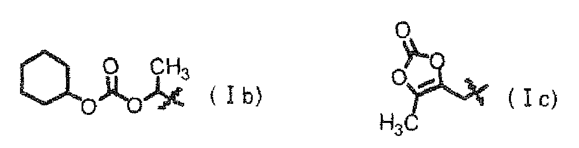
m and n are each an integer of zero or one.

2. The compound of claim 1, which is a compound represented by the following formula (II), or a pharmaceutically acceptable salt thereof: 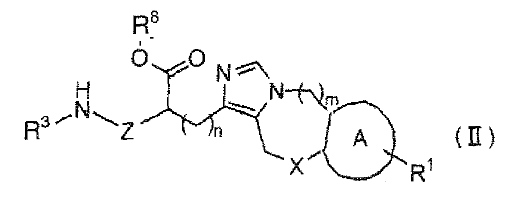
wherein Z is the formula -(CH₂)₃- or the formula -(CH₂)₄-;
A, X, R¹, R³-R⁸, m and n are as defined in claim 1.

3. The compound of claim 2, which is a compound represented by the following formula (III), or a pharmaceutically acceptable salt thereof: 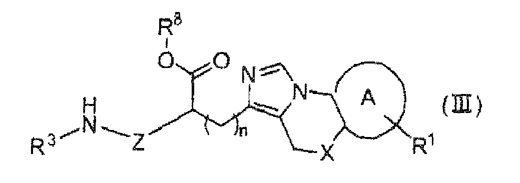
wherein X is the formula -(CH₂)-, the formula -(CH₂)₂-, an oxygen atom, or a nitrogen atom;
A, Z, R¹, R³-R⁸, and n are as defined in claim 2.

4. The compound of claim 3, which is a dihydroimidazoquinoline compound represented by the following formula (IV), or a pharmaceutically acceptable salt thereof: 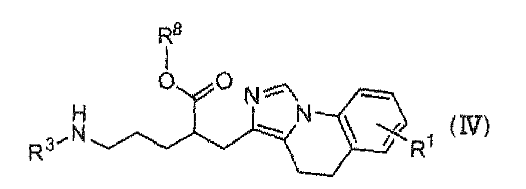 wherein R¹ and R³-R⁸ are as defined in claim 3.

5. The compound of claim 4, which is a dihydroimidazoquinoline compound represented by the following formula (V), or a pharmaceutically acceptable salt thereof: 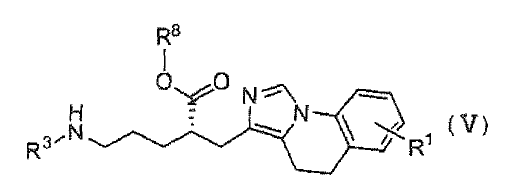 wherein R¹ and R³-R⁸ are as defined in claim 4.

6. The compound of claim 5, which is a dihydroimidazoquinoline compound represented by the following formula (VI), or a pharmaceutically acceptable salt thereof: 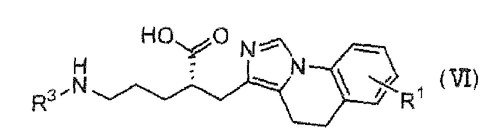 wherein R¹ and R³-R⁷ are as defined in claim 5.

7. The compound of claim 5, which is a dihydroimidazoquinoline compound represented by the following formula (VII), or a pharmaceutically acceptable salt thereof: 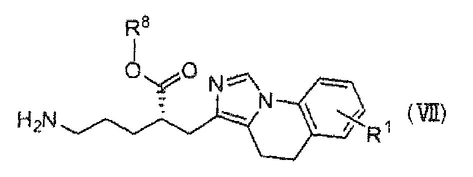 wherein R¹ and R⁸ are as defined in claim 5.

8. The compound of claim 3, which is a compound represented by the following formula (VIII), or a pharmaceutically acceptable salt thereof: 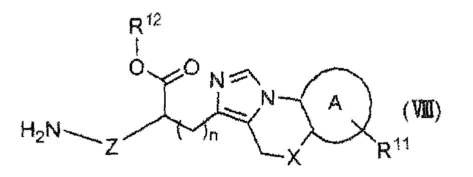
wherein A, X, Z and n are as defined in claim 3;
R¹¹ is a hydrogen atom, a halogen atom, a C₁₋₁₀ alkyl group, a C₁₋₈ alkoxy group, a C₃₋₈ cycloalkoxy group, or a phenyl group; and
R¹² is a hydrogen atom or a C₁₋₆ alkyl group.

9. The compound of claim 8, which is a dihydroimidazoquinoline compound represented by the following formula (IX), or a pharmaceutically acceptable salt thereof: 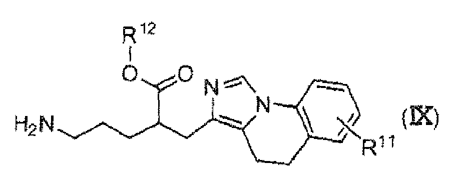 wherein R¹¹ and R¹² are as defined in claim 8.

10. The compound of claim 9, which is a dihydroimidazoquinoline compound represented by the following formula (X), or a pharmaceutically acceptable salt thereof: 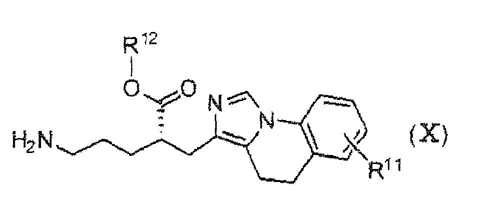 wherein R¹¹ and R¹² are as defined in claim 9.

11. A TAFIa inhibitor comprising the compound or the pharmaceutically acceptable salt thereof defined in any one of claims 1 to 10, as an active ingredient.

12. An agent for preventing or treating a clot-derived disease that comprises the compound or the pharmaceutically acceptable salt thereof defined in any one of claims 1 to 10, as an active ingredient.
